# EUROPÄISCHE PATENTSCHRIFT

(11) **EP 2 793 622 B1**
(45) Veröffentlichungstag und Bekanntmachung des Hinweises auf die Patenterteilung: **27.02.2019**
(21) Anmeldenummer: 12801456.0
(22) Anmeldetag: 03.12.2012
(51) Int. Cl.: A23L 3/3508, A61K 31/215, A23L 3/3517, A01N 37/36, A61Q 5/00, A61Q 5/02, A61K 8/04, C07C 67/303, C07C 69/757, C07C 69/675, C07C 233/58, C09B 69/00, C11D 3/20, C11D 3/32, A23L 33/10

(54) **VERWENDUNG VON CYCLOHEXANOLDERIVATEN ALS ANTIMIKROBIELLE WIRKSTOFFE**
USE OF CYCLOHEXANOL DERIVATIVES AS ANTIMICROBIAL ACTIVE INGREDIENTS
UTILISATION DE DÉRIVÉS DU CYCLOHÉXANOL COMME PRINCIPES ACTIFS ANTIMICROBIENS

(30) Priorität: 21.12.2011 EP 11010039; 31.07.2012 EP 12005577
(43) Veröffentlichungstag der Anmeldung: 29.10.2014
(73) Patentinhaber: Merck Patent GmbH, 64293 Darmstadt (DE)
(72) Erfinder: RUDOLPH, Thomas, 64291 Darmstadt (DE); MUELLER, Tatjana, 64832 Hergershausen (DE)
(86) Internationale Anmeldenummer: PCT/EP2012/004980
(87) Internationale Veröffentlichungsnummer: WO 2013/091775

(56) Entgegenhaltungen:
- EP-A2- 0 694 605
- WO-A1-2011/050871
- DE-B3-102004 062 894
- GB-A- 1 593 469
- JP-B- S4 832 102
- US-A- 3 535 367
- Ahmed G.Hegazi,Faten K.Abd El Hady: "Egyptian Propolis: 1- Antimicrobial Activity and Chemical Composition of Upper Egypt Propoliy - Apimondia", Proceedings of the 37th INternational Apicultural Congress,28 October -1 November 2001,Durban, South Africa, 28. Oktober 2001 (2001-10-28), Seiten 1-8, XP002714338, APIMONDIA 2001 ISBN: 0-620-27768-8 Gefunden im Internet: URL:http:///www.apimondia.com/congresses/2 001/Papers/004.pdf [gefunden am 2013-10-02]
- V.L. GEIN ET AL.: "Synthesis and Antimicrobial Activity of 2-Acetyl-5-Hydroxy-5-Methyl-3-Phenyl-1-Cyc lohexanone and Alkyl-4-Hydroxy-4-Methyl-2-Oxo-6-Phenylcyc lohexane-1-Carboxylates", PHARMACEUTICAL CHEMISTRY JOURNAL, Bd. 44, Nr. 5, Mai 2010 (2010-05), - Mai 2010 (2010-05), Seiten 13-15, XP002714339,
- DATABASE REGISTRY [Online] CHEMICAL ABSTRACTS SERVICE, COLUMBUS, OHIO, US; 17. November 1989 (1989-11-17), "Cylohexanecarboxylic Acid, 3-Hydroxy-, Phenylmethyl Ester", XP002714340, Database accession no. 123762-07-2
- DATABASE REGISTRY [Online] CHEMICAL ABSTRACTS SERVICE, COLUMBUS, OHIO, US; 16. November 1984 (1984-11-16), "Cyclohexanecarboxylic, 4-Hydroxy-, Ethyl Ester", XP002714341, Database accession no. 17159-80-7
- DIDIER BUISSON, ROBERT AZERAD: "Diastereoselective and enantioselective microbial reduction of cyclic alpha-alkyl beta-ketoesters", TETRAHEDRON LETTERS, vol. 27, no. 23, 1986, - 1986, pages 2631-2634, DOI: 10.1016/S0040-4039(00)84603-1

## Beschreibung

Die vorliegende Erfindung betrifft die Verwendung mindestens eines Cyclohexanolderivats der Formel I als antimikrobieller Wirkstoff oder als Anti-Akne-, Antischuppen-, Antitranspirant- oder desodorierender Wirkstoff, Zubereitungen enthaltend diese Verbindungen, sowie spezielle Cyclohexanolderivate und ein Verfahren zu deren Herstellung.

Mikrobielle Kontaminierung stellt ein wesentliches Problem in unserem täglichen Leben dar, zum Beispiel in Zusammenhang mit kosmetischen oder pharmazeutischen Produkten, Lebensmitteln, Oberflächen in Bädern oder Küchen oder Operationsbesteck. Gewöhnlich werden Konservierungsmittel verwendet, um mikrobielle Kontaminierung zu vermeiden.

Antimikrobielle Wirkstoffe sind jedoch nicht nur als Konservierungsmittel notwendig. Auch für viele kosmetische Verwendungen spielen antimikrobielle Wirkstoffe eine wichtige Rolle:
Schuppenbildung ist eine bei der Bevölkerung weit verbreitete Störung der Kopfhaut, die meist von leichtem bis mittlerem Juckreiz begleitet wird. Die Bildung von solchen gewöhnlichen Kopfschuppen ist nicht als Hautkrankheit im allgemeinen Sinne aufzufassen. Schuppen können durch Kopfhautstörungen entstehen, die beispielsweise durch übermäßige Sonnenaussetzung, Umwelteinflüsse aus der Luft oder kosmetische Haarprodukte ausgelöst werden können. Die Kopfschuppen entstehen in diesem Fall durch eine Überproduktion von Hornzellen, ausgelöst durch winzige Entzündungsherde der Kopfhaut, deren Ursache z.B. eine vermehrte Keimbesiedlung mit Pilzen (wie *Malassezia furfur* oder *Malassezia globosa)* oder Bakterien ist. Die unvollständig ausgereiften Hornzellen schilfern als Folge verfrüht in größeren Zellverbänden (Schuppen) ab. Da durch den Hautzellenverlust die äußerste Hautschicht dünner wird, führt Schuppenbildung zu einer erhöhten Empfindlichkeit der Kopfhaut, welche sich in Juckreiz und Rötungen äußert.

Unter Akne versteht man eine Hautstörung, die sich in entzündeten Papeln, Pusteln oder Knoten äußert, hervorgerufen durch eine verstärkte Talkproduktion und Verhornungsstörung der Haut. Die Entzündungen können mit Rötung, Schwellung und Druckschmerzen verbunden sein. Neben genetischer Prädisposition, können Androgene, komedogene Substanzen (z.B. in Kosmetika), Rauchen, Stress oder eine übermäßige Besiedlung der Haut mit Bakterien mögliche Ursachen für Aknebildung sein. Akne kann beispielsweise durch Mikroorganismen wie *Propionibacterium acnes, Propionibacterium granulosum* oder *Staphylococcus epidermidis* ausgelöst werden. *Propionibacterium acnes* ist ein Bakterium, welches gewöhnlicherweise die Haut bevölkert und sich von Sebum ernährt. Akne kann beispielsweise entstehen, wenn die Zahl dieser Bakterien erhöht ist. Die Gegenwart von Bakterien in den Follikeln führt zu Entzündungsreaktionen, welche sich in Form von roten Knoten oder Pusteln äußert. Die Produktion von freien Fettsäuren durch die Bakterien fördert weiterhin die Entzündungsreaktion im Follikel.

Achselschweiß enthält neben Wasser und Salz viele weitere Substanzen (wie Fette, Aminosäuren, Zucker, Milchsäure, Harnstoff etc.). Frisch gebildeter Schweiß ist geruchlos; der typische Schweißgeruch entsteht erst durch die Einwirkung von Hautbakterien auf den Schweiß, welche diesen zersetzen. Beispiele für solche Bakterien sind *Staphylococcus, Corynebacterium* oder *Malassezia.* In Deodorants und Antitranspirantien werden deshalb neben Geruchsstoffen und Antitranspirantien gewöhnlich auch antimikrobielle Stoffe eingesetzt, die die Bakterien kontrollieren sollen, welche an der Geruchsentstehung beteiligt sind.

Bei der Verwendung von antimikrobiellen Stoffen in Zubereitungen ist insbesondere ihre Veträglichkeit, aber auch ihre Formulierbarkeit (d.h. Löslichkeit, Stabiltät etc.) in den entsprechenden Produkten (z.B. Shampoos, Cremes, Deodorants) von großer Bedeutung. Insbesondere in der Kosmetik sind diese Eigenschaften essentiell. So ist es beispielsweise besonders erstrebenswert, dass die Inhaltsstoffe bei Normaldruck zwischen -5°C und 40°C in flüssigem Aggregatzustand vorliegen.

Ziel der vorliegenden Erfindung ist daher die Bereitstellung neuer Inhaltsstoffe mit antimikrobieller Wirkung, die die oben genannten vorteilhaften Eigenschaften aufweisen.

Überraschend wurde nun gefunden, dass bestimmte Cyclohexanolderivate die oben genannten Eigenschaften bei gleichzeitiger hervorragender antimikrobieller Wirkung aufweisen.

Im Stand der Technik werden ähnliche Verbindungen zur Verwendung in der Kosmetik beschrieben:
US 5858958 beschreibt 4-t-Butyl-Cyclohexanol als wirksames Antioxidans. Eine antimikrobielle Wirkung wird nicht beschrieben.

Phosphorsäureester von Cycloalkancarbonsäuren zur Verwendung in Mund- und Zahnpflegemitteln werden in WO 91/09589 offenbart.

WO 03/057184 A2 beschreibt die Verwendung von Benzohydroxamiden zur Hautaufhellung.

Ein weiteres bekanntes Cyclohexanolderivat ist p-Menthane-3,8-diol (PMD) welches als Bestandteil des ätherischen Öles des Lemon-Eucalyptus Insektrepellentwirkung besitzt.

Ein erster Gegenstand der vorliegenden Erfindung ist daher die Verwendung mindestens einer Verbindung der Formel I 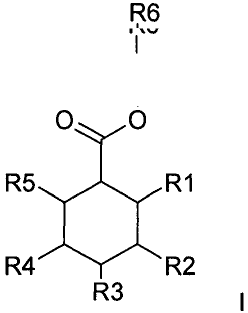 worin R1, R2, R3, R4 und R5 unabhängig voneinander stehen für einen Rest ausgewählt aus
- H, OH, OCOCH₃, O-(CH₂-CH₂-O)ₙ-CH₂-CH₂-OH, mit n=0 bis 20,
- geradkettige oder verzweigte Alkyl- oder Alkoxygruppe mit 1 bis 20 C-Atomen,
wobei mindestens einer der Reste R1, R2, R3, R4 und R5 für OH, OCOCH₃ oder O-(CH₂-CH₂-O)ₕ-CH₂-CH₂-OH, mit n=0 bis 20, steht,
und worin R6 und R7 unabhängig voneinander stehen für einen Rest ausgewählt aus
- H,
- (CH₂-CH₂-O)ₙ-CH₂-CH₂-OH, mit n= 0 bis 20,
- geradkettige oder verzweigten Alkylgruppe mit 1 bis 20 C-Atomen,
- geradkettige oder verzweigte Alkenyl- oder Alkinylgruppe mit 2 bis 20 C-Atomen und einer oder mehreren Doppel- bzw. Dreifachbindungen,
wobei die Alkyl-, Alkenyl- oder Alkinylgruppe auch eine oder mehrere gesättigte oder ungesättigte C₃- bis C₁₂-Cycloalkylgruppen enthalten kann,
als antimikrobieller Wirkstoff in Lebensmitteln,
Haushaltsprodukten,Kunststoffen, Papier und/oder Farben.

Unter einem antimikrobiellen Wirkstoff versteht man erfindungsgemäß eine Substanz, die das Wachstum von Mikroorganismen reduziert, oder die Mikroorganismen abtötet oder inaktiviert.

Die erfindungsgemäßen Verbindungen der Formel I können verwendet werden, um das Wachstum und die Vermehrung der Mikroorganismen zu hemmen. Unter Mikroorganismen werden beispielsweise Bakterien (gram-positive und gram-negative Bakterien), Hefen, Pilze oder Viren verstanden. Beispiele von Mikroorganismen sind Mikroorganismen ausgewählt aus *Staphylococcus, Micrococcus, Escherichia, Pseudomonas, Bacillus, Salmonella, Serratia, Shigella, Porphyromonas, Prevotella, Wolinella, Campylobacter, Propionibacterium, Streptococcus, Corynebacterium, Treponema, Fusobacterium, Bifidobacterium, Lactobacillus, Actinomyces, Candida, Malassezia, Aspergillus, Herpes simplex 1 und 2.*

Im Speziellen sind erfindungsgemäße Verbindungen antimikrobiell wirksam gegen *Staphylococcus epidermidis, Staphylococcus aureus, Corynebacterium xerosis, Malassezia furfur, Propionibacterium acnes, Pseudomonas aeruginosa, Salmonella enteric, Serratia marcescens, Aspergillus niger* und *Candida albicans.*

Die antimikrobiellen Verbindungen der Formel I zeichnen sich durch eine gute antimikrobielle Aktivität aus, das bedeutet, dass die Zahl der Keime in einem Medium reproduzierbar reduziert werden kann oder die Keimvermehrung unterdrückt wird (siehe Beispiele).

Die erfindungsgemäßen antimikrobiellen Wirkstoffe können in einer Vielzahl von Formulierungen oder Anwendungen zum Einsatz kommen, wie beispielsweise kosmetische/ pharmazeutische Formulierungen, Medizinprodukte, Lebensmittel, Haushaltsprodukte, Kunststoffe, Papier und/oder Farben. Im Speziellen kann es sich zum Beispiel um antimikrobielle Reinigungsprodukte, Seifen, Desinfektionsmittel, Prothesen oder Knochenzement mit antimikrobieller Aktivität, Zahnfüllungen und - prothesen, Zahn- und Mundpflegeprodukte, Körperpflegeprodukte (Cremes, Shampoos, Lotionen, Waschprodukte, Deodorants, Antitranspirantien, antimikrobielle Handspülungen etc.), Hygieneartikel, Küchen- und Badezimmerartikel, Geschirrspülprodukte oder Lebensmittel und Getränke handeln.

Die Verbindungen der Formel I können vorteilhaft zur Konservierungsverbesserung eingesetzt werden.

Vorteilhaft ist beispielsweise die Verwendung der erfindungsgemäßen antimikrobiellen Wirkstoffe in Zahn- oder Mundpflegeprodukten, z.B. zur Behandlung oder Prophylaxe von Plaque, Karies oder Mundgeruch, ausgelöst durch Mikroorganismen wie *Streptococcus sobrinus, Streptococcus mutans, Streptococcus gordonii, Streptococcus salivaris, Streptococcus sanguis, Actinomyces, Lactobacilli, Fusobacterium, Veillonella, Treponema. denticola, Porphyromonas. gingivalis, Bacteroides* oder *Peptostreptococcus.*

Als multifunktionelle Stoffe eignen sich die Verbindungen der Formel I zur Verwendung als duftgebende Geruchsstoffe zur Desodorierung und Maskierung unerwünschter Eigengerüche der Inhaltsstoffe in Formulierungen. Insbesondere ist in dieser Hinsicht eine Kombination mit weiteren Antioxidantien und Duftstoffen denkbar. Dies umfaßt z.B. alle Duftstoffe wie beschrieben in "S. Arctander, Perfume and Flavor Materials, Vol. I and II, Montclair, N.J., 1969, Selbstverlag" oder in "K. Bauer, D. Garbe and H. Surburg, Common Fragrance and Flavor Materials, 4th Ed., Wiley-VCH, Weinheim 2001" oder wie beschrieben in US7354893 B2, insbesondere beschrieben in US 7354893, Spalte 2, Zeilen 37 bis 67.

Erfindungsgemäße Stoffe eignen sich auf Grund ihrer Stoffeigenschaften hervorrragend zur Einarbeitung in Emulsionen und tensidische Zubereitungen wie z,.B. Reinigungsmittel und sog. Rinse-off-Zubereitungen wie z.B. Duschgele. Erfindungsgemäße Stoffe liegen z.B. bei Raumtemperatur in flüssiger Form vor und eignen sich zum einen als Lösungsmittel für Feststoffe, zum anderen zeigen sie Emollienteigenschaften mit gutem Spreitverhalten, welches ein angenehmes Hautgefühl der Formulierung bewirkt.

Insbesondere in der Kombination mit traditionellen Konservierungsmitteln lassen sich Verbesserungen im Konservierungsergebnis erzielen. Die Wirkung kosmetischer Alkohole, wie z.B. Glycole, kann durch die Stoffe verstärkt werden.

Ein weiterer Gegenstand der vorliegenden Erfindung ist die Verwendung der Verbindungen der Formel I als Anti-Akne-, Antischuppen-, Antitranspirant- oder desodorierender Wirkstoff.

Insbesondere eignen sich die Verbindungen der Formel I zur Behandlung oder Prophylaxe von Akne, welche durch Mikroorganismen wie *Propionibacterium acnes, Propionibacterium granulosum* oder *Staphylococcus epidermidis* ausgelöst wird.
Geeignete Formulierungen hierfür sind weiter unten beschrieben.

Weiterhin ist die Verwendung von Verbindungen der Formel I als Antischuppen-Wirkstoff vorteilhaft, sowohl zur Behandlung als auch zur Prophylaxe. Geeignete Formulierungen, beispielsweise Shampoos, sind weiter unten beschrieben.

Vorteilhaft ist auch die Verwendung als Wirkstoff in Antitranspirantien oder Deodorants. Zum Einen haben einige der erfindungsgemäßen Verbindungen einen angenehmen Eigengeruch, welcher unangenehme Gerüche überdecken und so desodorierend wirken kann. Beispielsweise riecht Verbindung I-2 nach Apfel, während Verbindung I-20 nach Himbeere riecht (Definitionen der Verbindungen siehe weiter unten).

Die Verwendung in Deodorants und Antitranspirantien ist weiterhin vorteilhaft, da die Verbindungen der Formel I eine antimikrobielle Wirkung gegen die Bakterien aufweisen, die für die Zersetzung des Schweißes und damit für die Entstehung des Geruchs verantwortlich sind. Besonders von Vorteil ist hierbei, dass die Verbindungen je nach Testkeim bakteriostatisch oder bakterizid wirken können. Eine bakteriostatische Wirkung wird erzielt, wenn die Vermehrung eines Bakteriums zwar gehemmt wird, wodurch die Geruchsentstehung unterdrückt wird, jedoch das Bakterium nicht abgetötet wird. Dadurch kann die natürliche Hautflora vorteilhaft aufrechterhalten werden.

So weisen beispielsweise die Verbindungen der Formel I-2, I-9 und I-10 eine bakteriostatische Wirkung gegen *Staphylococcus epidermidis* auf, während die Wirkung der Verbindung der Formel I-20 auf *S. epidermidis* bakterizid ist (Beispiel 5).

Mögliche Formulierungen für Deodorants und Antitranspirantien sind weiter unten beschrieben.

Die erfindungsgemäße Verwendung der Verbindungen der allgemeinen Formel I kann sowohl im kosmetischen Sinn als auch im pharmazeutischen Sinn erfolgen. Eine pharmazeutische Anwendung ist beispielsweise bei Anti-Akne-Mitteln denkbar.

Die Verbindungen der Formel I weisen eine unerwartet gute antimikrobielle Wirkung auf, wie in den Beispielen dargelegt wird. Weiterhin sind die Verbindungen der Formel I vorteilhaft in Formulierungen verwendbar, da es sich um klare Flüssigkeiten handelt, welche gut in die entsprechenden Formulierungen eingearbeitet werden können.

Erfindungsgemäß werden von den Verbindungen der Formeln I alle möglichen Ringkonfigurationsisomere umfasst, d.h. sowohl cis-, als auch trans-Isomere sind denkbar.

Ebenso ist für den Fachmann bekannt, dass die Atome der Verbindungen auch durch andere Isotope ersetzt sein können, so ist zum Beispiel ein Austausch der H-Atome durch D möglich.

Bevorzugt wird eine Verbindung der Formel I verwendet, wobei die Reste R1, R2, R3, R4 und R5 unabhängig voneinander für H oder OH stehen, wobei 1, 2 oder 3 der Reste R1, R2, R3, R4 und R5 für OH stehen.

Beispiele für solche Verbindungen sind im Folgenden aufgelistet:

| | | | |
|---|---|---|---|
| I-1 | 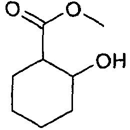 | I-2 | 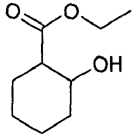 |
| I-3 | 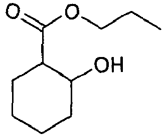 | I-4 | 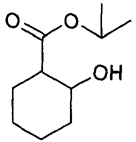 |
| I-5 | 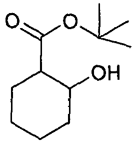 | I-6 | 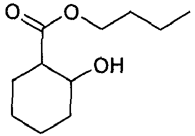 |
| I-7 | 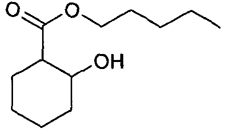 | I-8 | 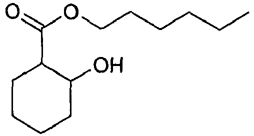 |
| I-9 | 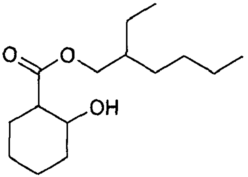 | I-10 | 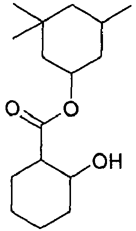 |
| I-11 | 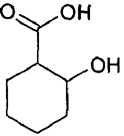 | I-12 | 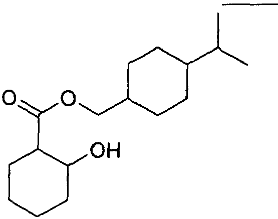 |
| I-13 | 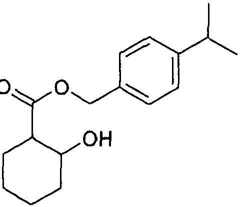 | I-14 | 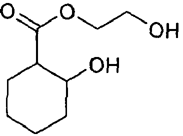 |
| I-15 | 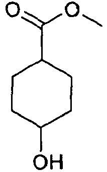 | I-16 | 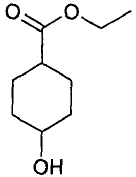 |
| I-17 | 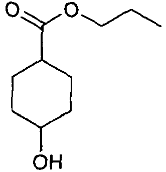 | I-18 | 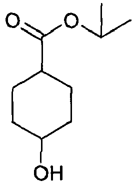 |
| I-19 | 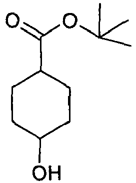 | I-20 | 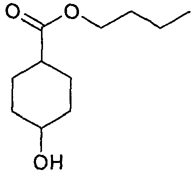 |
| I-21 | 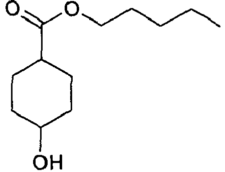 | I-22 | 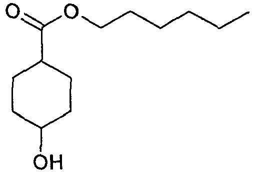 |
| I-23 | 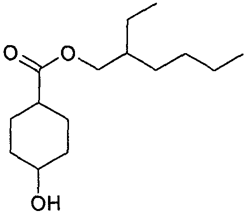 | I-24 | 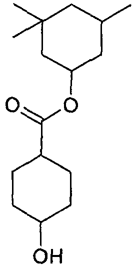 |
| I-25 | 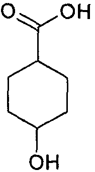 | I-26 | 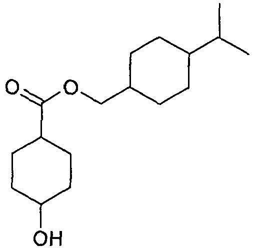 |
| I-27 | 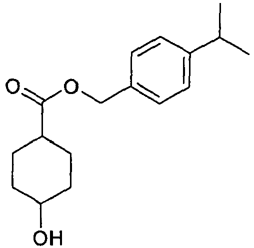 | I-28 | 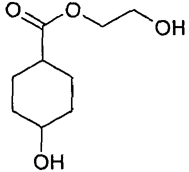 |
| I-29 | 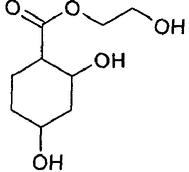 | I-30 | 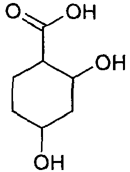 |
| I-31 | 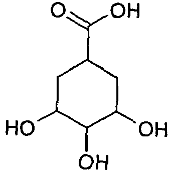 | I-32 | 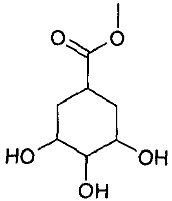 |
| I-33 | 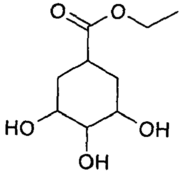 | I-34 | 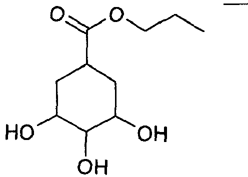 |
| I-35 | 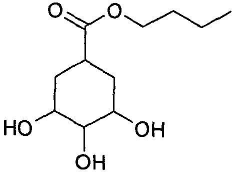 | | |

Beispiele für Verbindungen mit anderen Resten R1, R2, R3, R4 und R5 sind:

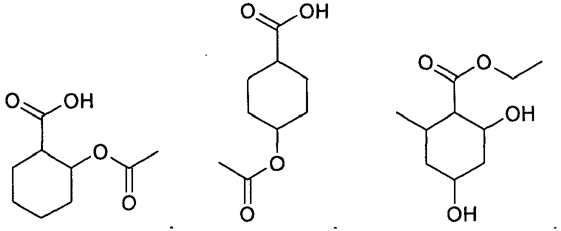

Weiterhin bevorzugt steht genau einer der Reste R1, R2, R3, R4 und R5 für OH, während die anderen Reste für H stehen. Besonders bevorzugt befindet sich die OH-Gruppe dann in p- oder m-Position, d.h. besonders bevorzugt steht in diesem Fall einer der Reste R1, R3 und R5 für OH.

Für die erfindungsgemäße Verwendung sind daher Verbindungen der Formel I bevorzugt, ausgewählt aus den Verbindungen der Formel Ia, oder Ib 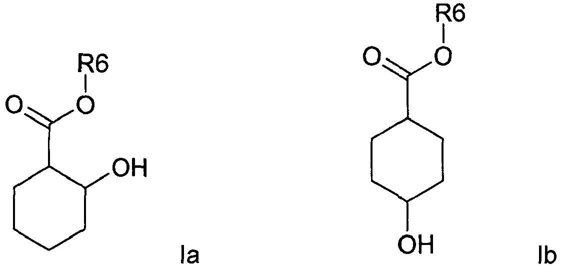 worin R6 und R7 unabhängig voneinander stehen für einen Rest ausgewählt aus
- H,
- (CH₂-CH₂-O)ₙ-CH₂-CH₂-OH, mit n= 0 bis 20,
- geradkettige oder verzweigten Alkylgruppe mit 1 bis 20 C-Atomen,
- geradkettige oder verzweigte Alkenyl- oder Alkinylgruppe mit 2 bis 20 C-Atomen und einer oder mehreren Doppel- bzw. Dreifachbindungen,
wobei die Alkyl-, Alkenyl- oder Alkinylgruppe auch eine oder mehrere gesättigte oder ungesättigte C₃- bis C₁₂-Cycloalkylgruppen enthalten kann.

Die Reste R6 und R7 stehen in den vorstehenden Definitionen unabhängig voneinander bevorzugt für einen Rest ausgewählt aus
- H,
- CH₂-CH₂-OH,
- geradkettige oder verzweigten Alkylgruppe mit 1 bis 10 C-Atomen, welche auch eine gesättigte oder ungesättigte C₆-Cycloalkylgruppe enthalten kann.

Besonders bevorzugt wird erfindungsgemäß eine Verbindung der Formel I verwendet, ausgewählt aus den Verbindungen der Formel I-1 bis I-28

| | | | |
|---|---|---|---|
| I-1 | 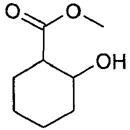 | I-2 | 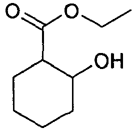 |
| I-3 | 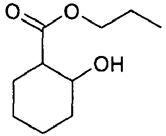 | I-4 | 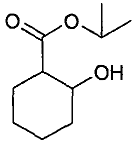 |
| I-5 | 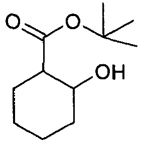 | I-6 | 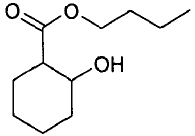 |
| I-7 | 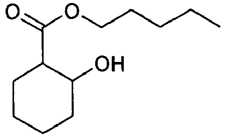 | I-8 | 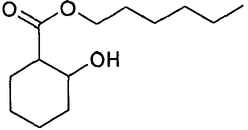 |
| I-9 | 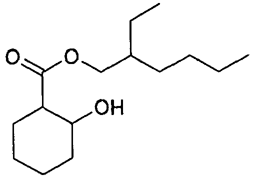 | I-10 | 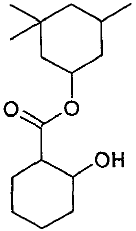 |
| I-11 | 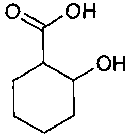 | I-12 | 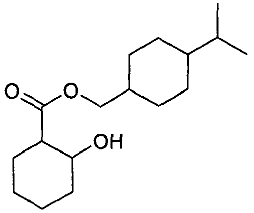 |
| I-13 | 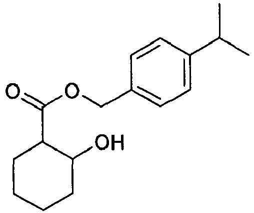 | I-14 | 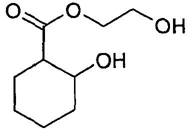 |
| I-15 | 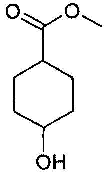 | I-16 | 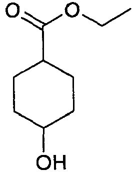 |
| I-17 | 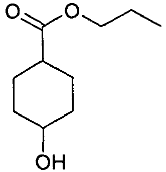 | I-18 | 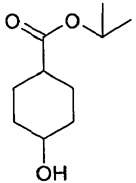 |
| I-19 | 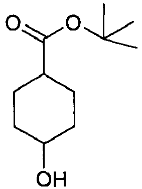 | I-20 | 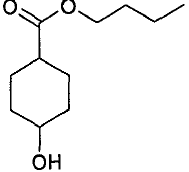 |
| I-21 | 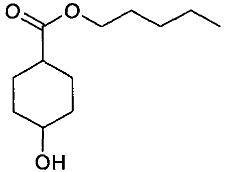 | I-22 | 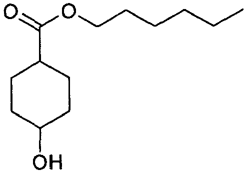 |
| I-23 | 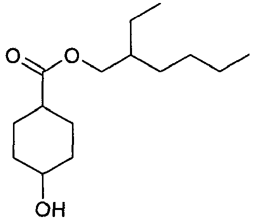 | I-24 | 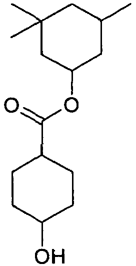 |
| I-25 | 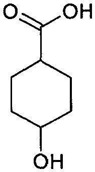 | I-26 | 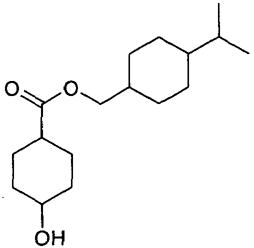 |
| I-27 | 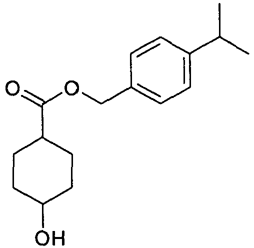 | I-28 | 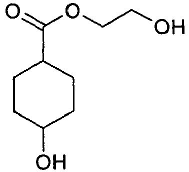 |

Ganz besonders bevorzugt sind die Verbindungen I-2, I-9 und I-10.

Im Sinne der vorliegenden Erfindung handelt es sich bei einer geradkettigen oder verzweigten C₁- bis C₁₀-Alkylgruppe um einen Alkylrest mit 1 bis 10 C-Atomen, beispielsweise um Methyl, Ethyl, Isopropyl, Propyl, Butyl, sek.-Butyl oder tert.-Butyl, Pentyl, Isopentyl, 1-, 2- oder 3-Methylbutyl, 1,1-, 1,2- oder 2,2-Dimethylpropyl, 1-Ethylpropyl, 1-Ethyl-1-methylpropyl, 1-Ethyl-2-methylpropyl, 1,1,2- oder 1,2,2-Trimethylpropyl, 1,1-, 1,2-, 1,3-, 2,2-, 2,3- oder 3,3-Dimethylbutyl, 1- oder 2-Ethylbutyl, 1-, 2-, 3- oder 4-Methylpentyl, Hexyl, Heptyl, 1-Ethyl-pentyl, Octyl, 1-Ethyl-hexyl, Nonyl oder Decyl.

Bei einer C₁- bis C₂₀-Alkylgruppe kann es sich neben den oben gelisteten Resten beispielsweise auch um Undecyl, Dodecyl, Tridecyl, Tetradecyl, Pentadecyl, Hexadecyl, Heptadecyl, Octadecyl, Nonadecyl oder Eicosyl handeln.

Erfindungsgemäß kann eine Alkenylgruppe eine oder mehrere Doppelbindungen enthalten. Eine geradkettige oder verzweigte C₂- bis C₂₀-Alkenylgruppe ist beispielsweise Allyl, Vinyl, Propenyl, 2- oder 3-Butenyl, Isobutenyl, sek.-Butenyl, 2-Methyl-1- oder 2-Butenyl, 3-Methyl-1-butenyl, 1,3-Butadienyl, 2-Methyl-1,3-butadienyl, 2,3-Dimethyl-1,3-butadienyl, 1-, 2-, 3- oder 4-Pentenyl, iso-Pentenyl, Hexenyl, Heptenyl oder Octenyl, -C₉H₁₇,-C₁₀H₁₉ bis -C₂₀H₃₉.

Eine Alkinylgruppe kann eine oder mehrere Dreifachbindungen enthalten. Beispiele für eine verzweigte oder nicht verzweigte C₂- bis C₂₀-Alkinylgruppe sind Ethinyl, 1- oder 2-Propinyl, 2- oder 3-Butinyl, ferner 4-Pentinyl, 3-Pentinyl, Hexinyl, Heptinyl, Octinyl, -C₉H₁₅, -C₁₀H₁₇ bis -C₂₀H₃₇.

Eine C₃- bis C₁₂-Cycloalkylgruppe bezeichnet im Sinne der Erfindung gesättigte und teilweise oder vollständig ungesättigte (d.h. auch aromatische) cyclische Kohlenwasserstoffgruppen, die 3 bis 12 C-Atome umfassen und auch durch -(CH₂)ₙ-Gruppen, mit n=1, 2 oder 3, überbrückt sein können. Die Bindung an den jeweiligen Rest kann über jedes Ringmitglied der Cycloalkylgruppe erfolgen. Beispiele für geeignete Cycloalkylreste sind Cyclopropyl, Cyclobutyl, Cyclopentyl, Cyclohexyl, Cycloheptyl, Cyclooctyl, Cyclopentenyl, Cyclopentadienyl, Cyclohexenyl, Cyclooctadienyl oder Phenyl.

Ein cyclischer Alkylrest mit 6 C-Atomen ist bevorzugt Cyclohexyl, Cyclohexenyl oder Phenyl.

Die Verbindungen der Formel I können mittels Hydrierung entsprechender aromatische Ausgangsprodukte nach dem Fachmann bekannten Methoden mit Wasserstoff und unter Verwendung eines geeigneten Ni-, Co-, Pt-, Pd- oder Rh-Katalysators hergestellt werden. (siehe beispielsweise GB 286201, Beispiel 3).

Die Edukte, sowie die weiteren notwendigen Stoffe in der Synthese sind kommerziell erhältlich oder durch Synthesen zugänglich, die dem Fachmann aus der Literatur bekannt sind. Dabei bereitet es dem Fachmann keine Schwierigkeiten, die geeigneten Reaktionsbedingungen wie Lösungsmittel oder Temperatur auszuwählen.

Typischerweise erfolgt die Reaktion bei Temperaturen unter 100°C und einem Druck kleiner 200 bar, z.B. bei 100 bar oder 5 bar.

Beispiele für mögliche Lösungsmittel sind 2-Propanol oder Tetrahydrofuran. Die Reaktionszeit beträgt typischerweise mehrere Stunden, beispielsweise 3 bis 5 Stunden.

Alternativ kann die Hydrierung auch mittels dem in WO 2011/001041 A1 beschriebenen Verfahren durchgeführt werden.

Ein weiterer Gegenstand der vorliegenden Erfindung ist auch eine Zubereitung enthaltend mindestens eine Verbindung der Formel I wie in den vorliegenden Ansprüchen definiert, und mindestens einen geeigneten Träger.

Bei der Zubereitung kann es sich sowohl um eine kosmetische als auch um eine pharmazeutische Zubereitung handeln. Bevorzugt handelt es sich um eine kosmetische Zubereitung.

Bevorzugte Ausführungsformen der Reste R1 bis R7 der Formel I sind hierbei wie zuvor beschrieben definiert.

Bei den Zubereitungen handelt es sich dabei beispielsweise um topisch anwendbare Zubereitungen, beispielsweise kosmetische oder dermatologische Formulierungen oder Medizinprodukte. Die Zubereitungen enthalten in diesem Fall einen topisch kosmetisch oder dermatologisch geeigneten Träger und je nach gewünschtem Eigenschaftsprofil optional weitere geeignete Inhaltsstoffe. Handelt es sich um pharmazeutische Zubereitungen, so enthalten die Zubereitungen in diesem Fall einen pharmazeutisch verträglichen Träger und optional weitere pharmazeutische Wirkstoffe.

Zubereitungen zur Anwendung im Mund enthalten einen Träger, der für diese Anwendungen geeignet ist.

Im Sinne der vorliegenden Erfindung wird neben dem Begriff Zubereitung gleichbedeutend auch der Begriff Mittel oder Formulierung verwendet.

Topisch anwendbar bedeutet im Sinne der Erfindung, dass die Zubereitung äußerlich einschließlich der Mundhöhle und örtlich angewendet wird, d.h. dass die Zubereitung geeignet sein muss, um beispielsweise auf die Haut aufgetragen werden zu können.

Die Zubereitungen können die genannten notwendigen oder optionalen Bestandteile umfassen oder enthalten, daraus im wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.

Die mindestens eine Verbindung der Formel I wird in den erfindungsgemäßen Zubereitungen typischerweise in Mengen von 0,01 bis 20 Gew.-%, bevorzugt in Mengen von 0,05 bis 10 Gew.-%, besonders bevorzugt in Mengen von 0,1 Gew.-% bis 5 Gew.-% und ganz besonders bevorzugt in Mengen von 0,5 bis 2 Gew.-%, bezogen auf die Gesamtmenge der Zubereitung, eingesetzt. Dabei bereitet es dem Fachmann keinerlei Schwierigkeiten die Mengen abhängig von der beabsichtigten Wirkung der Zubereitung entsprechend auszuwählen.

Bevorzugt handelt es sich bei der Zubereitung um ein Deodorant, ein Antitranspirant, ein Anti-Schuppen- oder Anti-Akne-Mittel oder eine antibakterielle Zubereitung, insbesondere zur Zahn- oder Mundpflege.

Anti-Akne-Mittel enthaltend die erfindungsgemäßen Verbindungen können in der Form von Seifen, Reiniger, Lösungen, Suspensionen, Emulsionen, Cremes, Gels, Pasten, Lotionen, Pudern, Ölen, Stiften oder Sprays vorliegen. Weitere mögliche Inhaltsstoffe in den Formulierungen sind weiter unten im Detail beschrieben.

Anti-Schuppen-Mittel enthaltend die erfindungsgegmäßen Verbindungen können beispielsweise in der Form eines Shampoos oder einer Spülung vorliegen, die vor oder nach dem Waschen, dem Färben oder dem Bleichen der Haare angewandt werden kann. Alternativ ist auch eine Formulierung möglich, in Form einer Lotion oder eines Gels zum Haarstyling, zur Haarbehandlung oder zur Haartrocknung, in Form eines Haarlacks, einer Formulierung für Dauerwelle, oder einer Formulierung zur Haarfärbung oder -bleichung. Eine solche kosmetische Formulierung kann eine Reihe von weiteren Inhaltsstoffen enthalten, wie oberflächenaktive Stoffe, Verdickungsmittel, Polymere, Weichmacher, Konservierungsstoffe, Schaumstabilisatoren, Elektrolyte, organische Lösungsmittel, Silikonderivate, Antifettwirksoffe, Farbstoffe oder Pigmente, die die Formulierung oder das Haar färben, oder andere Inhaltsstoffe, die gewöhnlich in Haarpflegeprodukten verwendet werden. Weitere Inhaltsstoffe werden im folgenden der Anmeldung detailliert beschrieben.

Deodorants und Antitranspirantien können beispielsweise in der Form von Cremes, Gelen, Lotionen, Emulsionen, Deodorantstiften, -rollern, Sprays oder Pumpsprays vorliegen. Die Verbindungen der Formel I und/oder II werden gewöhnlich mit für Deodorants und Antitranspirantien geeigneten Trägermaterialien, Inhaltsstoffen und Wirkstoffen kombiniert. Beispielhaft seien hier Kombinationen mit allen in WO 2011/131474 genannten Trägermaterialien, Inhaltsstoffen und Wirkstoffen genannt. Als besonders bevorzugt werden Kombinationen mit weiteren Deo-Adjuvantien erachtet, wie zum Beispiel aus den Gruppen der Silbersalze und/oder Silberkomplexe und/oder Mineralen vulkanischer Herkunft und/oder Zeolithen und/oder Alaun und/oder Haarwuchsinhibierenden Stoffen. Beispielhafte Silbersalze und/oder Silberkomplexe werden auf Seite 6, Zeile 7 bis Seite 10, Zeile 14 der WO 2011/131474 beschrieben. Mineralien vulkanischer Herkunft sind beispielhaft auf Seite 10, Zeile 15 bis Seite 11, Zeile 5 der WO 2011/131474 beschrieben. Zeolithe sind beispielhaft auf Seite 11, Zeile 7 bis Zeile 27 der WO 2011/131474 beschrieben. Salze vom Alaun-Typ sind beispielhaft auf Seite 11, Zeile 28 bis Seite 13, Zeile 17 der WO 2011/131474 beschrieben. Beispielhafte Haarwuchs inhibierende Substanzen sind auf Seite 13, Zeile 29 bis Seite 21, Zeile 11 der WO 2011/131474 beschrieben.

Beispiele für geeignete Trägermaterialien sind Glycerylstearat, Aluminiumchlorhydrat, Propylenglycol, Carbomer, Glycerin, Dicaprylether, Ethanol, Glycerylcocoat, Cylomethicone, Dimethicone, Dipropylenglycol, Stearylalcohol, Mineralöl, Phenyltrimethicone oder Natriumstearat.

In den oben genannten Formulierungen können die Verbindungen der Formel I vorteilhaft mit allen weiteren bekannten

Konservierungsstoffen oder antimikrobiellen Wirkstoffen kombiniert werden, wie zum Beispiel Anissäure, Alkohol, Ammomiumbenzoat, Ammoniumpropionat, Benzoesäure, Bronopol, Butylparaben, Benzethoniumchlorid, Benzalkoniumchlorid, 5-Brom-5-nitro-1,3-dioxan, Benzylalkohol, Borsäure, Benzisothiazolinon, Benzotriazol, Benzylhemiformat, Benzylparaben, 2-Brom-2-Nitropropan-1,3-Diol, Butylbenzoat, Chlorphenesin, Capryl/Capringlyceride, Caprylylglycol, Camellia Sinensis Blattextrakt, Candida Bombicola/glucose/methyl rapeseedate, Chlorxylenol, Chloracetamid, Chlorhexidin, Chlorbutanol, Calciumbenzoat, Calciumparaben, Calciumpropionat, Calciumsalicylat, Calciumsorbat, Captan, Chloramin T, Chlorhexidindiacetat, Chlorhexidindigluconat, Chlorhexidindithydrochlorid, Chloracetamin, p-Chlor-m-Cresol, Chlorophen, p-Chlorphenol, Chlorthymol, Citrus Grandis (Grapefruit) Fruchtextract, Citrus Grandis (Grapefruit) Samenextract, m-Cresol, o-Cresol, p-Cresol, gemischte Cresole, 1,2-Decanediol (INCI Decylene Glycol), Diazolidinylharnstoff, Dichlorobenzylalcohol, Dimethyloxazolidin, DMDM Hydantoin, Dimethylhydroxmethylpyrazol, Dehydroessigsäure, Diazolidinylharnstoff, DEDM Hydantoin, DEDM Hydantoin Dilaurat, Dibrompropamidindiisothionat, Dimethylolethylenthioharnstoff, Dithiomethylbenzamid, DMHF, Domiphenbromid, 7-Ethylbicyclooxazolidin, Ethylparaben, Ethylhexylglycerin, Ethanol, Ethylferulat, Formaldehyd, Ferulasäure, Glycerylcaprate, Glutaral, Glycerolformat, Glyoxal, Hexamidindiisethionat, Hexandiol, Hexetidin, Hexamidin, Hexamidindiparaben, Hexamidinparaben, 4-Hydroxybenzoesäure, Hydroxymethyldioxazabicyclooctan, Imidazolidinylharnstoff, Imidiazolidinylharnstoff NF, Isobutylparaben, Isothiazolinon, lodpropynylbutylcarbamat, Isodecylparaben, Isopropylcresol, Isopropylparaben, Isopropylsorbat, Kaliumsorbat NF FCC, Kupferusnat, Kaliumbenzoat, Kaliumethylparaben, Kaliummethylparaben, Kaliumparaben, Kaliumphenoxid, Kalium o-Phenylphenat, Kaliumpropionat, Kaliumpropylparaben, Kaliumsalicylat, Kaliumsorbat, Methylparaben, Methylisothiazolinon, Methylbenzethoniumchloridphenol, Methyldibromglutaronitril, Methenammoniumchlorid, Methylbromglutaronitril, Magnesiumbenzoat, Magnesiumpropionat, Magnesiumsalicylat, MDM Hydantoin, MEA-Benzoat, MEA o-Phenylphenat, MEA-Salicylat, Methylchloristhiazolinon, Natriumbenzoat NF FCC, Natriumcaprylat, Natriumdehydroacetat, Natriumdehydroacetate FCC, Natriumhydroxymethylglycinat, Natriummethylparaben, Natriumpropylparaben, Natriumiodat, Niembaumsamenöl, Nisin, Natriumbenzoat, Natriumbutylparaben, Natrium-p-chlor-m-Cresol, Natriumethylparaben, Natriumformat, Natriumhydroxymethansulfonat, Natriumisobutylparaben, Natriumparaben, Natriumphenolsulfonat, Natriumphenoxid, Natrium-o-phenylphenat, Natriumpropionat, Natriumpropylparaben, Natriumpyrithion, Natriumsalicylat, Natriumsorbat, Ortholphenylphenol, Phenoxyethanol, Propylparaben, Polymethoxybicyclicoxazolidin, Pinus Pinaster Rindenextrakt, Poloxamer 188, PVP-Iodine, Parabene, Pircotone olamine, Phenethylalkohol, Polyaminopropylbiguanid, Polyquarternium-42, PEG-5 DEDM Hydantoin, PEG-15 DEDM Hydantoin, PEG-5 Hydantoin Oleat, PEG-15 DEDM Hydantoin Stearat, Phenethylalcohol, Phenol, Phenoxyethylparaben, Phenoxyisopropanol, Phenylbenzoat, Phenyl Quecksilberacetat, Phenyl Quecksilberbenzoat, Phenyl Quecksilberborat, Phenyl Quecksilberbromid, Phenylquecksilberchlorid, Phenylparaben, o-Phenylphenol, Polyaminopropylbiguanidstearat, Propionsäure, Propylbenzoat, Quaternium-15, Quaternium-8, Quaternium-14, Rosmarinus officinalis Blattextrakt, Sorbinsäure NF FCC, Selenium disulphin, Sorbinsäure, Salicylsäure, Silberborsilicat, Silbermagnesiumaluminiumphosphat, Triclosan, di-alpha-Tocopherol, Tocopherolacetat, Thimersal, Triclocarban, TEA-Sorbat, Thimerosal, Usnic acid, Undecylenoyl PEG-5 Paraben, Vitis vinifera Samenextrakt, Teebaumöl, Wasserstoffperoxid, Zinkpyrithion, Zinkoxid, Zinkphenolsulphonat oder Kombinationen davon.

In den oben genannten Formulierungen können die Verbindungen der Formel I vorteilhaft mit einem oder mehreren Insektenschutzmitteln (Insect Repellants) kombiniert werden. Wichtige Insektenschutzmittel sind z.B. N,N-diethyl-m-toluamid (DEET), p-Menthan-3,8-diol (PMD) oder IR3535 (3-[N-Butyl-N-acetyl]-aminopropionic acid, ethyl ester). Erfindungsgemäße Stoffe können auch selbst Insekten abwehrende Eigenschaften haben oder zur verbesserten Abwehr in Kombination mit weiteren Insektenschutzmitteln beitragen.

Die Verbindungen der Formel I können auch mit Antibiotika kombiniert werden. Erfindungsgemäß können alle bekannten Antibiotika verwendet werden, zum Beispiel beta-Lactam, Vancomycin, Macrolide, Tetracycline, Quinolone, Fluorquinolone, nitrierte Verbindungen (wie Nitroxolin, Tilboquinol oder Nitrofurantoin), Aminoglycoside, Phenicole, Lincosamida, Synergistine, Fosfomycin, Fusidinsäure, Oxazolidinone, Rifamycine, Polymixyne, Gramicidine, Tyrocydine, Glycopeptide, Sulfonamide oder Trimethoprime.

Formulierungen zur Mund- oder Zahnpflege können beispielsweise in Form einer Zahncreme, eines Mundwassers, eines Zahnpulvers, eines Kaugummis, einer Pastille, eines Mundsprays, Zahnseide, Zahnzement oder -farbe sein.

Ensprechende Formulierungen können weitere gewöhnliche Inhaltsstoffe enthalten, wie zum Beispiel Feuchthaltemittel, oberflächenaktive Mittel, Strukturbildner, Gelbildner, Schleifmittel, Fluoridquellen, Desensibilisierungsmittel, Geschmacksstoffe, Farbstoffe, Süßstoffe, Konservierungsstoffe, antimikrobielle Stoffe oder Mittel gegen Zahnbelag oder Zahnstein enthalten.

Geeignete Feuchthaltemittel zur Verwendung in Zahncremes sind beispielsweise Mehrwertige Alkohole, wie Xylitol, Sorbitol, Glycerin, Propylenglycol oder Polyethylenglycol. Mischungen von Glycerin und Sorbitol sind besonders geeignet. Ein Feuchthaltemittel trägt dazu bei, dass Zahncremeformulierungen bei Luftkontakt nicht austrocknen und dass das Mundgefühl der Creme (weiche Beschaffenheit, Fließfähigkeit, Süße) angenehm ist. Typischerweise sind diese Feuchthaltemittel in Mengen von 0-85 Gewichts-%, bevorzugt von 0-60 Gewichts-% in der Zubereitung enthalten.

Geeignete oberflächenaktive Stoffe in Zahncremes, Mundwässern etc. sind typischerweise wasserlösliche organische Verbindungen und können anionisch, nicht-ionisch, kationisch oder amphoter sein. Bevorzugt sollte ein angemessen stabiler oberflächenaktiver Stoff ausgewählt werden. Anionische oberflächenaktive Stoffe sind zum Beispiel wasserlösliche Salze von C₁₀₋₁₈ Alkylsulphaten (z.B. Natriumlaurylsulfat), wasserlösliche Salze von C₁₀₋₁₈ ethoxylierten Alkylsulphaten, wasserlösliche Salze von C₁₀₋₁₈ Alkylsarcosinaten, wasserlösliche Salze von sulfonierten Monoglyceriden von C₁₀₋₁₈ Fettsäuren (z.B. Natrium Kokosfettsäure Monoglyceridsulfonat), Alkylarylsulfonate (z.B. Natriumdodecylbenzensulfonat) und Natriumsalze des Kokosfettsäureamids von N-methyltaurin.

Nichtionische oberflächenaktive Stoffe, welche für Mundpflegemittel geeignet sind, sind beispielsweise die Kondensationsprodukte der Alkylenoxidgruppen mit aliphatischen oder alkylaromatischen Verbindungen, wie Polyethylenoxidekondensate von Alkylphenolen, Ethylenoxid/propylenoxid Copolymere (erhältlich unter dem Namen 'Pluronic'), Ethylenoxid/Ethylendiamin Copolymere, Ethylenoxidkondensate von aliphatischen Alkoholen, langkettige tertiäre Aminoxide, langkettige tertiäre Phosphinoxide, langkettige Dialkylsulfoxides und Mischungen davon. Alternativen hierzu sind ethoxylierte Sorbitanester, welche zum Beispiel über ICI unter dem Namen "Tween" erhältlich sind.

Kationische oberflächenaktive Mittel sind typischerweise quaternäre Ammoniumverbindungen mit einer C₈₋₁₈ Alkylkette, wie zum Beispiel Lauryltrimethylammoniumchlorid, Cetyltrimethylammoniumbromid, Cetylpyridiniumchlorid, di-Isobutylphenoxyethoxyethyldimethylbenzylammoniumchlorid, Kokosfettsäurealkyltrimethylammoniumnitrit und Cetylpyridiniumfluorid.

Auch geeignet sind Benzylammoniumchlorid, Benzyldimethylstearylammoniumchlorid und tertiäre Amine mit einer C₁₋₁₈ Kohlenwasserstoffgruppe und zwei (Poly)oxyethylengruppen. Amphoterische oberflächenaktive Mittel sind typischerweise aliphatische sekundäre und tertiäre Amine, wobei die aliphatischen Reste geradkettig oder verzweigt sein können und worin einer der aliphatischen Reste eine C₈₋₁₈ Gruppe ist und der andere Rest eine anionische hydrophile Gruppe enthält, zum Beispiel Sulfonat, Carboxylat, Sulfate, Phosphonat oder Phosphat.

Normalerweise wird das oberflächenaktive Mittel in einer Menge von 0-20 Gew.-%, bevorzugt 0-10 Gew.-% in die Mundpflegeformulierung eingearbeitet.

Strukturbildner können in Zahncremes oder Kaugummis notwendig sein, um die gewünschten strukturellen Eigenschaften und gewünschte "Mundgefühl" zu ermöglichen. Geeignete Stoffe sind zum Beispiel natürliche Gummis wie Tragantgummi, Xanthangummi, Karayagummi und Gummiarabicum, Algenderivate wie Perlmoos und Alginate, Smektiterde wie Bentonit oder Hectorit, Carboxyvinylpolymere und wasserlösliche Cellulosederivate wie Hydroxyethylcellulose und Natriumcarboxymethylcellulose. Verbesserte Strukturen können auch erreicht werden, wenn zum Beispiel colloidales Magnesiumaluminiumsilicat verwendet wird. Typischerweise ist der Strukturbildner in einer Menge von 0-5 Gew.-%, bevorzugt 0-3 Gew.-% in der Mundhygieneformulierung enthalten.

Schleifmittel sollten bevorzugt in der Lage sein, die Zähne zu putzen und/oder zu polieren, ohne dabei den Zahnschmelz oder das Dentin zu beschädigen. Meist werden sie in Zahncremes oder Zahnpulvern verwendet, jedoch können sie auch in Mundwässern etc. zum Einsatz kommen. Geeignete Schleifmittel sind beispielsweise Silikaschleifmittel, wie hydratisierte Silikate oder Silikagele, insbesondere Silicaxerogele (z.B. 'Syloid' erhältlich bei W. R. Grace and Company). Ebenso geeignet sind die Silikamaterialien erhältlich unter dem Namen 'Zeodent' von J. M. Huber Corporation, und Diateomeenerde wie 'Celite' erhältlich von Johns-Manville Corporation. Alternative Schleifmittel sind Alumina, unlösliche Metaphosphate wie unlösliches Natriummetaphosphat, Calciumcarbonat, Dicalciumphosphat (in Dihydrat und wasserfreie Formen), Calciumpyrophosphat, Polymethoxylate und spezielle partikuläre aushärtbare polymerisierte Harze, wie Melaminharnstoffe, Melaminformaldehyde, Harnstoffformaldehyde, Melaminharnstoffformaldehyde, vernetzte Epoxide, Melamine, Phenolharze, hochreine Cellulosen (z.B. 'Elcema' erhältlich von Degussa AG), und vernetzte Polyester. Typischerweise werden Schleifmittel in einer Menge von 0-80 Gew.-%, bevorzugt 0-60 Gew.-% in der Mundhygieneformulierung eingearbeitet.

Geeignete Fluoridquellen sind zum Beispiel Natriumfluorid, Zincfluorid, Kaliumfluorid, Aluminiumfluorid, Lithiumfluorid, Natriummonofluorphosphat, Zinnfluorid, Ammoniumfluorid, Ammoniumbifluorid und Aminfluorid. Die Fluoridquellen sind bevorzugt in geeigneten Mengen enthalten, um bei der Verwendung ca. 50 ppm bis ca. 4,000 ppm Fluoridion bereitzustellen.

Geeignete Desensibilisierungsmittel sind zum Beispiel Formaldehyd, Kaliumnitrat, Trikaliumcitrat, Kaliumchlorid und Strontiumchlorid, Strontiumacetat und Natriumcitrat.

Geschmacksstoffe können beispielsweise ausgewählt sein aus Ölen der Pfefferminze, der Grünen Minze, der Moosbeere, der Sassafraswurzel und der Nelke. Süßstoffe können auch verwendet werden, zum Beispiel D-tryptophan, Saccharin, Dextrose, Aspartam, Levulose, Acesulfam, Dihydrochalcone und Natriumcyclamat. Typischerweise sind alle diese Geschmacksstoffe in Mengen von 0-5 Gew.-%, bevorzugt von 0-2 Gew.-% enthalten. Farbstoffe und Pigmente können hinzugefügt werden, um die Formulierung optisch attraktiver zu gestalten. Häufig wird Titandioxid verwendet um eine starke weiße Farbe zu erhalten.

Wie oben beschrieben können die erfindungsgemäßen Formulierungen zur Zahn- und Mundpflege auch ein oder mehrere weitere antimikrobielle Wirkstoffe enthalten. Geeignete Beispiele hierfür sind Zinksalze (wie Zinkcitrat), Cetylpyridiniumchlorid, bis-Biguanide (wie Chlorhexidin), aliphatische Amines, Bromchlorophene, Hexachlorophene, Salicylanilide, quaternäre Ammoniumverbindungen und Triclosan. Alternativ können enzymatische Systeme eingesetzt werden, z.B. kann ein System enthaltend Lactoperoxidase und Glucoseoxidase verwendet werden um antimikrobiell wirksame Mengen von Wasserstoffperoxid in Gegenwart von Glucose, Wasser und Sauerstoff zu generieren.

Die Formulierung kann auch Alkohol enthalten. Dies ist besonders vorteilhaft in Mundwässern.

Die erfindungsgemäßen Zubereitungen können auch neben den Verbindungen der Formel I und den oben beschriebenen Inhaltsstoffen, weitere Inhaltsstoffe enthalten. Im Folgenden werden weitere mögliche Inhaltsstoffe, insbesondere für kosmetische Zubereitungen, beschrieben.

Die erfindungsgemäßen Zubereitungen können zusätzlich mindestens einen UV-Filter enthalten.

Organische UV-Filter, sogenannte hydrophile oder lipophile Sonnenschutzfilter, sind im UVA-Bereich und/oder UVB-Bereich und/oder IR und/oder VIS-Bereich (Absorber) wirksam. Diese Substanzen können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben. Im Folgenden werden die genannten UV-Filter meist nach der INCI-Nomenklatur benannt.

Insbesondere für eine Kombination geeignet sind: para-Aminobenzoesäure und deren Derivate: PABA, Ethyl PABA, Ethyl dihydroxypropyl PABA, Ethylhexyl dimethyl PABA, z. B. vetrieben unter dem Namen "Escalol 507" von der Fa. ISP, Glyceryl PABA, PEG-25 PABA, z. B. vetrieben unter dem Namen "Uvinul P25" von der Fa. BASF.

Salicylate: Homosalate vetrieben unter dem Namen "Eusolex HMS" von der Fa. Merck; Ethylhexyl salicylate, z. B. vetrieben unter dem Namen "Neo Heliopan OS" von der Fa. Symrise, Dipropylene glycol salicylate, z. B. vetrieben unter dem Namen "Dipsal" von der Fa. Scher, TEA salicylate, z. B. vetrieben unter dem Namen "Neo Heliopan TS" von der Fa. Symrise.

β,β-Diphenylacrylate Derivate: Octocrylene, z. B. vetrieben unter dem Namen "Eusolex® OCR" von der Firma Merck", "Uvinul N539" von der Fa. BASF, Etocrylene, z. B. vetrieben unter dem Namen "Uvinul N35" von der BASF.

Benzophenone Derivate: Benzophenone-1, z. B. vetrieben unter dem Namen "Uvinul 400"; Benzophenone-2, z. B. vetrieben unter dem Namen "Uvinul D50" ; Benzophenone-3 oder Oxybenzone, z. B. vetrieben unter dem Namen "Uvinul M40";Benzophenone-4, z. B. vetrieben unter dem Namen "Uvinul MS40" ; Benzophenone-9, z. B. vetrieben unter dem Namen "Uvinul DS-49" von der Fa. BASF, Benzophenone-5, Benzophenone-6, z. B. vetrieben unter dem Namen "Helisorb 11" von der Fa. Norquay, Benzophenone-8, z. B. vetrieben unter dem Namen "Spectra-Sorb UV-24" von der Fa. American Cyanamid, Benzophenone-12 n-hexyl 2-(4-diethylamino-2-hydroxybenzoyl)benzoate oder 2-Hydroxy-4-methoxybenzophenon, vertrieben von der Fa. Merck, Darmstadt unter dem Namen Eusolex® 4360.

Benzylidencampher Derivate: 3-Benzylidenecamphor, z. B. vetrieben unter dem Namen "Mexoryl SD" von der Fa. Chimex, 4-Methylbenzylidenecamphor, z. B. vetrieben unter dem Namen "Eusolex 6300" von der Fa. Merck, Benzylidenecamphorsulfonsäure, z. B. vetrieben unter dem Namen "Mexoryl SL" von der Fa. Chimex, Camphor benzalkonium methosulfate, z. B. vetrieben unter dem Namen "Mexoryl SO" von der Fa. Chimex, Terephthalylidenedicamphorsulfonsäure, z. B. vetrieben unter dem Namen "Mexoryl SX" von der Fa Chimex, Polyacrylamidomethylbenzylidenecamphor vetrieben unter dem Namen "Mexoryl SW" von der Fa. Chimex.

Phenylbenzimidazol Derivate: Phenylbenzimidazolesulfonsäure, z. B. vetrieben unter dem Namen "Eusolex 232" von der Fa. Merck, Dinatrium phenyl dibenzimidazole tetrasulfonat, z. B. vetrieben unter dem Namen "Neo Heliopan AP" von der Fa. Symrise.

Phenylbenzotriazol Derivate: Drometrizole trisiloxane, z. B. vetrieben unter dem Namen "Silatrizole" von der Fa. Rhodia Chimie, Methylenebis(benzotriazolyl)tetramethylbutylphenol in fester Form, z. B. vetrieben unter dem Namen "MIXXIM BB/100" von der Fa. Fairmount Chemical, oder in mikronisierter Form als wässrige Dispersion, z. B. vetrieben unter dem Namen "Tinosorb M" von der Fa. BASF.

Triazin Derivate: Ethylhexyltriazone, z. B. vetrieben unter dem Namen "Uvinul T150" von der Fa. BASF, Diethylhexylbutamidotriazone, z. B. vetrieben unter dem Namen "Uvasorb HEB" von der Fa. Sigma 3V. Weitere Triazinderivate sind exemplarisch 2,4,6-tris(diisobutyl 4'-aminobenzalmalonate)-s-triazine, oder 2,4,6-Tris-(biphenyl)-1,3,5-triazine, Butyl 4-({4-{[4-(butoxycarbonyl)phenyl]amino}-6-[(3-{1,3,3,3-tetramethyl-1-[(trimethylsilyl)oxy]disiloxanyl} propyl)amino]-1,3,5-triazin-2-yl}amino)benzoate, vertrieben unter dem namen Mexoryl SBS. Struktur von Mexoryl SBS: 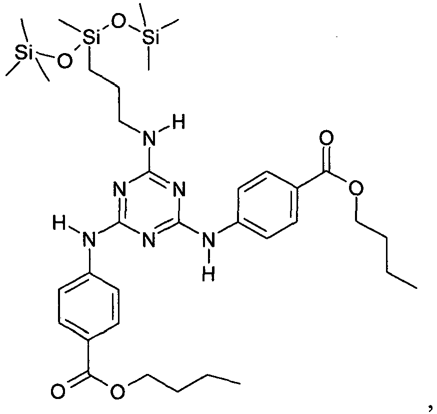 sowie Bis-ethylhexyloxyphenol methoxyphenyl triazine, z.B. vertrieben unter dem Namen Tinosorb S durch die Firma BASF.

Anthranilin Derivate: Menthyl anthranilate, z. B. vetrieben unter dem Namen "Neo Heliopan MA" von der Fa. Symrise.

Imidazol Derivate: Ethylhexyldimethoxybenzylidenedioxoimidazoline propionat.

Benzalmalonat Derivate: Polyorganosiloxane enthaltend funktionelle benzalmalonate Gruppen, wie z.B. Polysilicone-15, z. B. vetrieben unter dem Namen "Parsol SLX" von der Hoffmann LaRoche.

4,4-Diarylbutadien Derivate: 1,1-Dicarboxy(2,2'-dimethylpropyl)-4,4-diphenylbutadiene.

Benzoxazole Derivate: 2,4-bis[5-(1-dimethylpropyl)benzoxazol-2-yl(4-phenyl)imino]-6-(2-ethylhexyl)imino-1,3,5-triazine, z. B. vetrieben unter dem Namen Uvasorb K2A von der Fa. Sigma 3V und Mischungen dieses enthaltend.

Piperazinderivate wie beispielsweise die Verbindung 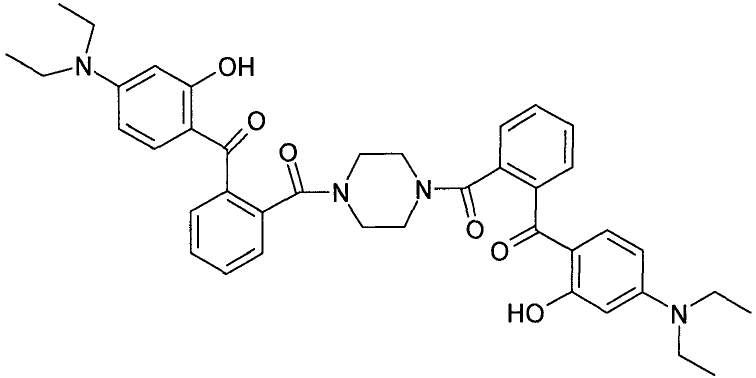 oder die UV-Filter der folgenden Strukturen 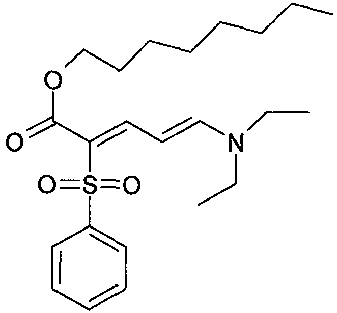 oder

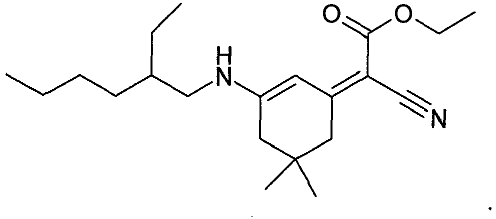

Es können auch UV-Filter auf Basis von Polysiloxancopolymeren mit einer statistischen Verteilung gemäß nachfolgender Formel verwendet werden, wobei z.B. a = 1,2; b= 58 und c=2,8 sind:

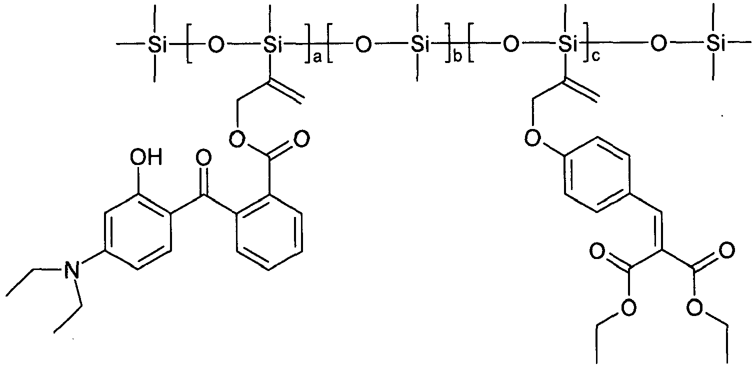

Die in der Liste aufgeführten Verbindungen sind nur als Beispiele aufzufassen. Selbstverständlich können auch andere UV-Filter verwendet werden.

Geeignete organischen UV-schützende Substanzen sind bevorzugt aus der folgenden Liste auszuwählen: Ethylhexyl salicylate, Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl)imino]-6-(2-ethylhexyl)imino-1,3,5-triazine und Mischungen davon.

Diese organischen UV-Filter werden in der Regel in einer Menge von 0,01 Gewichtsprozent bis 20 Gewichtsprozent, vorzugsweise 1 Gew.-% - 10 Gew.-%, in Formulierungen eingearbeitet.

Die Zubereitungen können neben den Verbindungen der Formel I sowie den gegebenenfalls organischen UV-Filtern, wie zuvor beschrieben, weitere anorganische UV-Filter, sogenannte partikuläre UV-Filter, enthalten.

Diese Kombinationen mit partikulären UV-Filtern sind sowohl als Pulver als auch als Dispersion oder Paste der folgenden Typen möglich.

Hierbei sind sowohl solche aus der Gruppe der Titandioxide wie z.B. gecoatetes Titandioxid (z.B. Eusolex® T-2000, Eusolex®T-AQUA, Eusolex®T-AVO, Eusolex®T-OLEO), Zinkoxide (z.B. Sachtotec®), Eisenoxide oder auch Ceroxide und/oder Zirkonoxide bevorzugt.

Ferner sind auch Kombinationen mit pigmentärem Titandixoxid oder Zinkoxid möglich, wobei die Partikelgröße dieser Pigmente größer oder gleich 200 nm sind, beispielsweise Hombitan® FG oder Hombitan® FF-Pharma.

Weiter kann es bevorzugt sein, wenn die Zubereitungen anorganische UV-Filter enthalten, die mit üblichen Methoden, wie beispielsweise in Cosmetics & Toiletries, February 1990, Vol. 105, pp. 53 64 beschrieben, nachbehandelt wurden. Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Bevorzugt eingesetzte partikuläre UV-Filter sind dabei:
- unbehandelte Titandioxide wie z.B. die Produkte Microtitanium Dioxide MT 500 B der Fa. Tayca; Titandioxd P25 der Fa. Degussa,
- Nachbehandelte mikronisierte Titandioxide mit Aluminiumoxid und Siliciumdioxid Nachbahandlung wie z.B. das Produkt "Microtitanium Dioxide MT 100 SA der Tayca; oder das Produkt "Tioveil Fin" der Fa. Uniqema,
- Nachbehandelte mikronisierte Titandioxide mit Aluminiumoxid und/oder Aluminiumstearate/laurate Nachbehandlung wie z.B. Microtitanium Dioxide MT 100 T der Fa. Tayca, Eusolex T-2000 der Firma Merck,
- Nachbehandelte mikronisierte Titandioxide mit Eisenoxid und/oder Eisenstearate Nachbehandlung wie z.B. das Produkt "Microtitanium Dioxide MT 100 F" der Fa. Tayca,
- Nachbehandelte mikronisierte Titandioxide mit Siliciumdioxide, Aluminiumoxid und Silicon Nachbehandlung wie z.B. das Produkt "Microtitanium Dioxide MT 100 SAS",der Fa. Tayca,
- Nachbehandelte mikronisierte Titandioxide mit Natrumhexameta¬phosphate, wie z.B. das Produkt "Microtitanium Dioxide MT 150 W" der Fa. Tayca.

Die zur Kombination eingesetzten behandelten mikronisierten Titandioxide können auch nachbehandelt sein mit:
- Octyltrimethoxysilane; wie z.B. das Produkt Tego Sun T 805 der Fa. Degussa,
- Siliciumdioxid; wie z.B. das Produkt Parsol T-X der Fa. DSM,
- Aluminiumoxid und Stearinsäure; wie z.B. das Produkt UV-Titan M160 der Fa. Sachtleben,
- Aluminium und Glycerin; wie z.B. das Produkt UV-Titan der Fa. Sachtleben
- Aluminium und Silikonölen, wie z.B. das Produkt UV-Titan M262 der Fa. Sachtleben,
- Natriumhexamethaphosphat und Polyvinylpyrrolidon,
- Polydimethylsiloxane, wie z.B. das Produkt 70250 Cardre UF TiO2SI3" der Fa. Cardre,
- Polydimethylhydrogensiloxane, wie z.B. das Produkt Microtitanium Dioxide USP Grade Hydrophobic" der Fa. Color Techniques.

Ferner kann auch die Kombination mit folgenden Produkten vorteilhaft sein:
- Unbehandelte Zinkoxide wie z. B. das Produkt Z-Cote der Fa. BASF (Sunsmart), Nanox der Fa. Elementis
- Nachbehandelte Zinkoxide wie z.B die folgenden Produkte:
   - "Zinc Oxide CS-5" der Fa. Toshibi (ZnO nachbehandelt mit polymethylhydrogenosiloxane)
   - Nanogard Zinc Oxide FN der Fa. Nanophase Technologies
   - "SPD-Z1" der Fa Shin-Etsu (ZnO nachbehandelt mit einem Silikongepfropften Acrylpolymer, dispergiert in Cyclodimethylsiloxane
   - "Escalol Z100" der Fa ISP (Aluminiumoxid nachbehandeltes ZnO dispergiert in einer ethylhexyl methoxycinnamate/PVP-hexadecene/ methicone copolymer Mischung)
   - "Fuji ZNO-SMS-10" der Fa. Fuji Pigment (ZnO nachbehandelt mit Siliciumdioxid und Polymethylsilesquioxan);
   - Unbehandeltes Ceroxide Mikropigment z.B. mit der Bezeichnung "Colloidal Cerium Oxide" der Fa Rhone Poulenc
   - Unbehandelte und/oder nachbehandelte Eisenoxide mit der Bezeichnung Nanogar der Fa. Arnaud.

Beispielhaft können auch Mischungen verschiedener Metalloxide , wie z.B. Titandioxid und Ceroxid mit und ohne Nachbenhandlung eingesetzt werden, wie z.B. das Produkt Sunveil A der Fa. Ikeda. Außerdem können auch Mischungen von Aluminiumoxid, Siliciumdioxid und Silikonnachbehandeltem Titandioxid, Zinkoxid-Mischungen wie z.B. das Produkt UV-Titan M261 der Fa. Sachtleben verwendet werden.

Diese anorganischen UV-Filter werden in der Regel in einer Menge von 0,1 Gewichtsprozent bis 25 Gewichtsprozent, vorzugsweise 2 Gew.-% - 10 Gew.-%, in die Zubereitungen eingearbeitet.

Durch Kombination von einer oder mehrerer der genannten Verbindungen mit UV-Filterwirkung kann die Schutzwirkung gegen schädliche Einwirkungen der UV-Strahlung optimiert werden.

Alle genannten UV-Filter können auch in verkapselter Form eingesetzt werden. Insbesondere ist es von Vorteil organische UV-Filter in verkapselter Form einzusetzen.

Dabei sind die Kapseln in erfindungsgemäß einzusetzenden Zubereitungen vorzugsweise in solchen Mengen enthalten, die gewährleisten, dass die verkapselten UV-Filter in den oben angegebenen Gewichtsprozentverhältnissen in der Zubereitung vorliegen.

In den beschriebenen Zubereitungen, die erfindungsgemäß mindestens eine Verbindung der Formel I enthalten, können weiterhin auch Farbpigmente enthalten sein, wobei der Schichtaufbau der Pigmente nicht limitiert ist.

Vorzugsweise sollte das Farbpigment bei Einsatz von 0,5 bis 5 Gew.-% hautfarben oder bräunlich sein. Die Auswahl eines entsprechenden Pigments ist für den Fachmann geläufig.

Bevorzugte Zubereitungen können ebenfalls mindestens einen weiteren kosmetischen Wirkstoff enthalten, beispielsweise ausgewählt aus Antioxidantien, Anti-Ageing-, Anti-Falten-, weiteren Anti-Schuppen-, weiteren Anti-Akne-, Anti-Cellulite-Wirkstoffen, weiteren Deodorants, hautaufhellenden Wirkstoffen, Selbstbräunungssubstanzen oder Vitaminen.

Hinsichtlich der Anti-Aknewirkung sind synergistische Kombinationen mit weiteren Anti-Aknewirkstoffen, wie z.B. in WO2009/098139 auf Seite 47, Zeile 2 bis Seite 48, Zeile 27 und DE10324567 offenbart, denkbar. Exemplarische weitere Anti-Aknewirkstoffe sind Silberpartikel und Silbersalze wie Silberlactat und Silbercitrat, Azelainsäure, Ellaginsäure, Milchsäure, Glycolsäure, Salicylsäure, Glycyrrhizinsäure, Triclosan, Phenoxyethanol, Hexamidinisethionat, Ketoconazol, Peroxide wie Wasserstoffperoxid oder Benzoylperoxid, 3-Hydroxybenzoesäure, 4-hydroxybenzoesäure, Phytinsäure, Arachidonsäure, Caprylylglycol, Ethylhexylglycerin, Farnesol, Cetylpyridiniumsalze, 6-Trimethylpentyl-2-pyridon (Piroctone Olamine) und Lipohydroxysäure (LHA).

Hinsichtlich der Anti-Schuppenwirkung sind synergistische Kombinationen mit weiteren Anti-Schuppenwirkstoffen denkbar, wie z.B. Zinkpyrithion, Piroctone Olamine, Selendisulfid, Climbazole, Triclosan, Butylparaben, 1,3-Bis(hydroxymethyl)-5,5-dimethylimidazolidine-2,4-dione (DMDM Hydantoin), Fumarsäure, Methylchloroisothiazolinone oder Methylisothiazolinone (MIT),

In ihrer Verwendung in Desodorantien und Antitranspirantien lassen sich erfindungsgemäße Stoffe synergistisch mit weiteren Deo-Adjuvantien kombinieren. Hierzu sei auf die in WO2011/131474 aufgeführten Wirkstoffe verwiesen. Exemplarisch werden an dieser Stelle folgende kombinierbare Wirkstoffe aufgeführt: 2-Methyl 5-Cyclohexylpentanol, Aluminium Chlorohydrate, Ethylhexlglycerin, Farnesol, Aluminum Zirconium Tetrachlorohydrex GLY, Aluminium Chlorohydrate, Aluminium zirconium tetrachlorohydrate, Aluminum Sesquichlorohydrate (Aluminumhydroxidchlorid), Triclosan, Aluminum tetrachloride, Zinc Ricinoleate, Aluminum Zirconium Pentachlorohydrate, Polyaminopropyl Biguanide Stearate, Benzyl Salicylate, Aluminum Sesquichlorohydrat, Zinc PCA (Zinksalz der Pyrrolidoncarbonsäure), Zinkgluconat Triethylcitrat, Aluminum Chloride, Aluminum Chlorohydrex Polyethylene Glycol Complex, Aluminum Chlorohydrex Propylene Glycol Complex, Aluminum Dichlorohydrate, Aluminum Dichlorohydrex Polyethylene Glycol Complex, Aluminum Dichlorohydrex Propylene Glycol Complex, Aluminum Sesquichlorohydrate, Aluminum Sesquichlorohydrex Polyethlene Glycol Complex, Aluminum Sesquichlorohydrex Propylene Glycol Complex, Aluminum Sulfate Buffered, Aluminum Zirconium Octachlorohydrate, Aluminum Zirconium Octachlorohydrex Glycine Complex, Aluminum Zirconium Pentachlorohydrate, Aluminum Zirconium Pentachlorohydrex Glycine Complex, Aluminum Zirconium Tetrachlorohydrate, Aluminum Zirconium Tetrachlorohydrex Glycine Complex, Aluminum Zirconium Trichlorhydrate, Aluminum Zirconium Trichlorohydrex Glycine Complex, Aluminum Zirconium Trichlorohydrex Glycine Complex, Aluminum Sulfate Buffered With Sodium Aluminum Lactate.

Die schützende Wirkung von Zubereitungen gegen oxidativen Stress bzw. gegen die Einwirkung von Radikalen kann verbessert werden, wenn die Zubereitungen ein oder mehrere Antioxidantien enthalten, wobei es dem Fachmann keinerlei Schwierigkeiten bereitet geeignet schnell oder zeitverzögert wirkende Antioxidantien auszuwählen.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA, Pentasodium ethylenediamin tetramethylen phosphonat und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Phytantriol, Coenzym Q10, Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selen¬methionin), Stilbene und deren Derivate (z.B. Stilbenoxid, trans-Stilbenoxid).

Geeignete Antioxidantien sind auch Verbindungen der Formeln A oder B 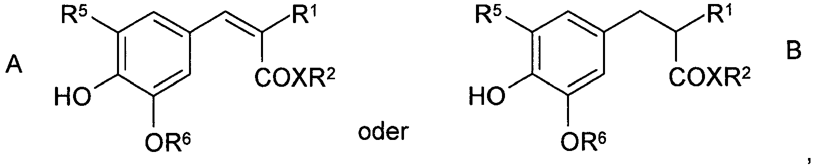 worin
- R¹: aus der Gruppe -C(O)CH₃, -CO₂R³, -C(O)NH₂ und -C(O)N(R⁴)₂ ausgewählt werden kann,
- X: O oder NH,
- R²: lineares oder verzweigtes Alkyl mit 1 bis 30 C-Atomen,
- R³: lineares oder verzweigtes Alkyl mit 1 bis 20 C-Atomen,
- R⁴: jeweils unabhängig voneinander H oder lineares oder verzweigtes Alkyl mit 1 bis 8 C-Atomen,
- R⁵: H, lineares oder verzweigtes Alkyl mit 1 bis 8 C-Atomen oder lineares oder verzweigtes Alkoxy mit 1 bis 8 C-Atomen und
- R⁶: lineares oder verzweigtes Alkyl mit 1 bis 8 C-Atomen bedeutet,
vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex® ST Liquid) und/oder 2-(4-Hydroxy-3,5-dimethoxybenzyl)-malonsäure-bis-(2-ethylhexyl)ester (z.B. RonaCare® AP).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure, natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L (+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit den erfindungsgemäßen Verbindungen in solchen Zusammensetzungen üblicherweise in Gewichtsprozentverhältnissen im Bereich von 1000:1 bis 1:1000, bevorzugt in Gewichtsprozentverhältnissen von 100:1 bis 1:100 eingesetzt.

Unter den Phenolen, die erfindungsgemäß verwendet werden können, sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydroxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers und I.M.C.M. Rietjens (Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Die Zubereitungen können auch neben den Verbindungen der Formel I ein oder mehrere weitere Anti-Ageing-Wirkstoffe enthalten. Geeignete Anti-Ageing Wirkstoffe, insbesondere für hautpflegende Zubereitungen, sind vorzugsweise sogenannte kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin- Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myo-inositol-phosphat (DIP), cyclisches 2,3-diphosphoglycerat (cDPG), 1,1-Diglycerin-Phosphat (DGP), β-Mannosylglycerat (Firoin), β-Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure) und deren Derivate zu nennen.

Additional können als Anti-Ageing Wirkstoffe Produkte der Firma Merck wie z.B. 5,7-Dihydroxy-2-methyl-chromon, vermarktet unter dem Handelsnamen RonaCare®Luremine, Ronacare®Isoquercetin, Ronacare®Tilirosid oderRonacare®Cyclopeptide 5 verwendet werden.

Die Zubereitungen können auch ein oder mehrere hautaufhellende Wirkstoffe oder synonym Depigmentierungswirkstoffe oder Melanogeneseinhibitoren enthalten. Hautaufhellende Wirkstoffe können prinzipiell alle dem Fachmann bekannte Wirkstoffe sein. Beispiele von Verbindungen mit hautaufhellender Aktivität sind Hydrochinon, Kojisäure, Arbutin, Aloesin, Niacinamide, Azelainsäure, Elaginsäure, Maulbeerbaumextract, Magnesium-ascorbyl-phosphat, Süßholzwurzelextrakt, Emblica, Ascorbinsäure oder Rucinol.

Ferner können die erfindungsgemäßen Zubereitungen mindestens eine Selbstbräunungssubstanz als weiteren Inhaltsstoff enthalten. Als vorteilhafte Selbstbräunungssubstanzen können unter anderem eingesetzt werden:
1,3-Dihydroxyaceton, Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon) oder 2-Hydroxy-1,4-naphtochinon (Lawson). Ganz besonders bevorzugt ist 1,3-Dihydroxyaceton, Erythrulose oder deren Kombination. Bevorzugt ist die mindestens eine weitere Selbstbräunungssubstanz in der Zubereitung in einer Menge von 0,01 bis 20 Gew.-%, besonders bevorzugt in einer Menge von 0,5 bis 15 Gew.-% und ganz besonders bevorzugt in einer Menge von 1 bis 8 Gew.-%, bezogen auf die Gesamtmenge der Zubereitung, enthalten.

Die einzusetzenden Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂), insbesondere bevorzugt Vitamin-A-Palmitat, Vitamin C und dessen Derivate, DL-α-Tocopherol, Tocopherol-E-Acetat, Nicotinsäure, Pantothensäure und Biotin. Vitamine werden mit den flavonoidhaltigen Vormischungen oder Zubereitungen üblicherweise bei kosmetischer Anwendung in Bereichen von 0,01 bis 5,0 Gew.-%, bezogen auf das Gesamtgewicht, zugesetzt.

Gegenstand der vorliegenden Erfindung ist auch ein Verfahren zur Herstellung einer Zubereitung, wie in den vorliegenden Ansprüche beschrieben, dadurch gekennzeichnet, dass mindestens eine Verbindung der Formel I mit einem geeigneten Träger und optional mit Hilfs- und oder Füllstoffen vermischt wird. Geeignete Trägerstoffe sowie Hilfs- oder Füllstoffe sind im nachfolgenden Teil ausführlich beschrieben.

Die genannten Bestandteile der Zubereitung können in der üblichen Weise eingearbeitet werden, mit Hilfe von Techniken, die dem Fachmann wohl bekannt sind.

Die kosmetischen und dermatologischen Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W) oder O/W/O, ein Gel, einen festen Stift, eine Salbe oder auch ein Aerosol darstellen. Bevorzugt werden Emulsionen. O/W-Emulsinen werden besonders bevorzugt. Emulsionen, W/O-Emulsionen und O/W-Emulsionen sind in üblicher Weise erhältlich.

Als Anwendungsform der einzusetzenden Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole, Pflaster, Umschläge, Verbände und Sprays.

Bevorzugte Hilfsstoffe stammen aus der Gruppe der Konservierungsstoffe, Stabilisatoren, Lösungsvermittler, Färbemittel, Geruchsverbesserer.

Salben, Pasten, Cremes und Gele können die üblichen Trägerstoffe enthalten, die für die topische Verabreichung geeignet sind, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlenwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3 Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Ein bevorzugter Lösungsvermittler generell ist 2-Isopropyl-5-methylcyclohexancarbonyl-D-Alaninmethylester.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Puder-, Emulsions- und Wachs-Make up sowie Sonnenschutz-, Prä-Sun- und After-Sun-Präparate.

Zu den bevorzugten Zubereitungsformen gehören insbesondere auch Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C Zahl, z.B. mit Isopropanol, Propylenglykol oder Glycerin, oder Ester von Fettalkoholen mit Alkansäuren niedriger C Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C Atomen und gesättigten und/oder ungesättigten, verzweigten und/oder unverzweigten Alkoholen einer Kettenlänge von 3 bis 30 C Atomen, aus der Gruppe der Ester aus aromatischen Carbonsäure und gesättigten und/oder ungesättigten, verzweigten und/oder unverzweigten Alkoholen einer Kettenlänge von 3 bis 30 C Atomen. Solche Esteröle können dann vorteilhaft gewählt werden aus der Gruppe Isopropylmyristat, Isopropylpalmitat, Isopropylstearat, Isopropyloleat, n Butylstearat, n-Hexyllaurat, n Decyloleat, Isooctylstearat, Isononylstearat, Isononylisononanoat, 2 Ethylhexylpalmitat, 2 Ethylhexyllaurat, 2 Hexaldecylstearat, 2 Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Die wässrige Phase der einzusetzenden Zubereitungen enthält gegebenenfalls vorteilhaft Alkohole, Diole oder Polyole niedriger C Zahl, sowie deren Ether, vorzugsweise Ethanol, Isopropanol, Propylenglykol, Glycerin, Ethylenglykol, Ethylenglykolmonoethyl oder -monobutylether, Propylenglykolmonomethyl, -monoethyl oder -monobutylether, Diethylenglykolmonomethyl oder -monoethylether und analoge Produkte, ferner Alkohole niedriger C Zahl, z. B. Ethanol, Isopropanol, 1,2 Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

In einer bevorzugten Ausführungsform enthalten die einzusetzenden Zubereitungen hydrophile Tenside. Die hydrophilen Tenside werden bevorzugt gewählt aus der Gruppe der Alkylglucoside, der Acyllactylate, der Betaine sowie der Cocoamphoacetate.

Als Emulgatoren können beispielsweise die bekannten W/O- und O/W-Emulgatoren verwendet werden. Es ist vorteilhaft, weitere übliche Co-Emulgatoren in den bevorzugten O/W-Emulsionen zu verwenden.

Vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen.

Es ist ferner von Vorteil, die Fettsäureethoxylate aus folgender Gruppe zu wählen:
Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
PoIyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol(20)glyceryloleat, Polyethylenglycol(20)glycerylisostearat, Polyethylenglycol(18)glyceryloleat(cocoat) zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:
Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C Atome, Diglycerinester gesättigter und/oder ungesättigter, verzweigter und/oder unverzweigter Alkancarbonsäuren einer Kettenlänge von 8 bis 24, insbesondere 12-18 C¬ Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat oder PEG-30-dipolyhydroxystearat.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die öligalkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane , bevorzugt Alkane.

Die Verbindungen der Formel I können erfindungsgemäß auch in Nahrungsmitteln verwendet werden. Die weiteren zu Nahrungsmitteln ausgeführten Erläuterungen gelten sinngemäß auch für Nahrungsergänzungsmittel und für "functional food".

Die Nahrungsmittel, die nach der vorliegenden Erfindung mit einer oder mehreren Verbindungen der Formel I angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren ". Die Nahrungsmittel können fest sein aber auch in flüssiger Form, also als Getränk, vorliegen.

Nahrungsmittel, die nach der vorliegenden Erfindung mit einer oder mehreren Verbindungen der Formel I angereichert werden können, sind beispielsweise Nahrungsmittel, die aus einer einzigen natürlichen Quelle stammen (z.B. Zucker, ungesüßter Saft, Korn, Getreide, Getreidesirup), Mischungen von derartigen Nahrungsmitteln (z.B. Multivitaminpräparate, Mineralstoffmischungen oder gezuckerter Saft) oder Nahrungsmittelzubereitungen (z.B. zubereitete Cerealien, Gebäck, Mischgetränke, Joghurt, Diätnahrungsmittel, kalorienarme Nahrungsmittel oder Tierfutter).

Die Nahrungsmittel, die nach der vorliegenden Erfindung mit einer oder mehreren Verbindungen der Formel angereichert werden können, umfassen somit alle genießbaren Kombinationen von Kohlehydraten, Lipiden, Proteinen, anorganischen Elementen, Spurenelementen, Vitaminen, Wasser oder aktiven Metaboliten von Pflanzen und Tieren.

Die mit einer oder mehreren Verbindungen der Formel I und/oder II angereicherten erfindungsgemäßen Nahrungsmittel können mit Hilfe von Techniken hergestellt werden, die dem Fachmann wohl bekannt sind.

Ein weiterer Gegenstand der vorliegenden Erfindung sind auch Verbindungen der Formel Ia, Ib, 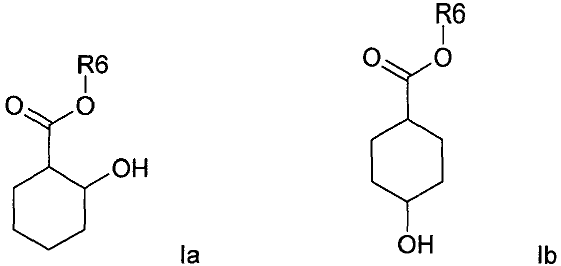 worin R6 und R7 unabhängig voneinander stehen für einen Rest ausgewählt aus
- H,
- (CH₂-CH₂-O)ₙ-CH₂-CH₂-OH, mit n= 0 bis 20,
- geradkettige oder verzweigten Alkylgruppe mit 1 bis 20 C-Atomen,
- geradkettige oder verzweigte Alkenyl- oder Alkinylgruppe mit 2 bis 20 C-Atomen und einer oder mehreren Doppel- bzw. Dreifachbindungen,
wobei die Alkyl-, Alkenyl- oder Alkinylgruppe auch eine oder mehrere gesättigte oder ungesättigte C₃- bis C₁₂-Cycloalkylgruppen enthalten kann, wobei Verbindungen der Formel Ia ausgeschlossen sind, in denen R6 für H (Verbindung I-11), Methyl (Verbindung I-1), Ethyl (Verbindung I-2), Isopropyl (Verbindung I-4), tert-Butyl (Verbindung I-5), n-Butyl (Verbindung I-6), n-Hexyl (Verbindung I-8), CH₂CH(CH₂CH₃)₂, CH₂CH₂CH(CH₃)₂, C(=CH₂)CH₃ oder Cyclopentyl steht,
wobei Verbindungen der Formel Ib ausgeschlossen sind, in denen R6 für H (Verbindung I-25), Methyl (Verbindung I-15), Ethyl (Verbindung I-16), Propyl (Verbindung I-17), Isopropyl (Verbindung I-18) oder tert-Butyl (Verbindung I-19) steht.

Bevorzugt sind die Reste R6 und R7 wie oben beschrieben definiert.

Besonders bevorzugt sind die Verbindungen ausgewählt aus den nachstehenden Verbindungen:

| | | | |
|---|---|---|---|
| I-3 | 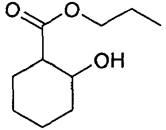 | | |
| I-7 | 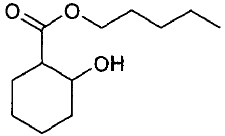 | I-9 | 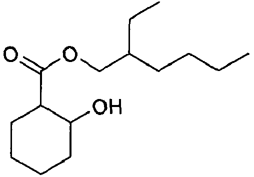 |
| I-10 | 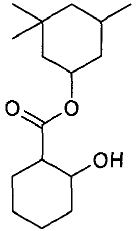 | I-12 | 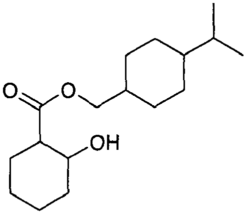 |
| I-13 | 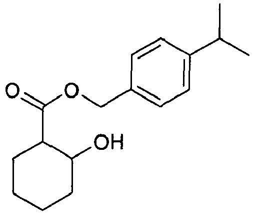 | I-14 | 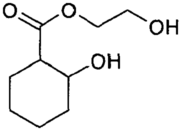 |
| I-20 | 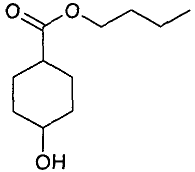 | I-21 | 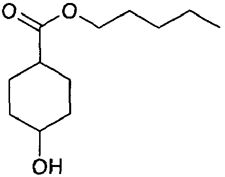 |
| I-22 | 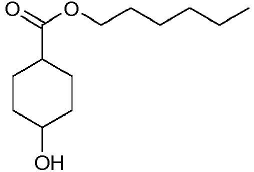 | I-23 | 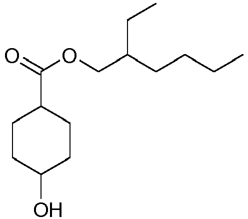 |
| I-24 | 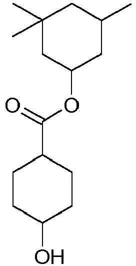 | I-26 | 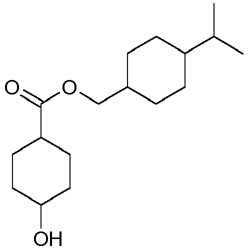 |
| I-27 | 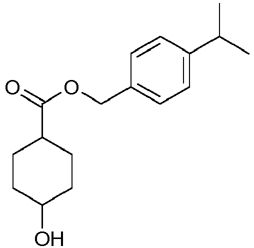 | I-28 | 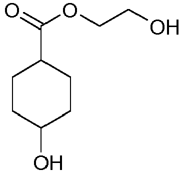 |

Ebenfalls Gegenstand der vorliegenden Erfindung ist ein Verfahren zur Herstellung einer Verbindung der Formel Ia oder Ib, wie zuvor beschrieben, dadurch gekennzeichnet, dass eine Verbindung der Formel Ia-x oder Ib-x 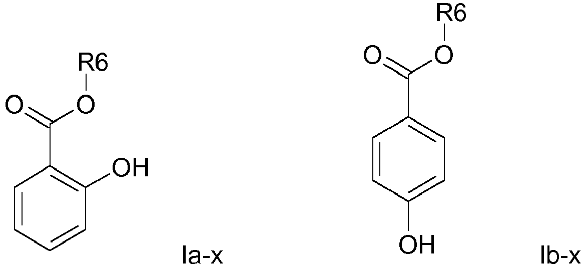 mittels Hydrierung zum entsprechenden hydrierten Produkt der Formel Ia oder Ib umgesetzt wird.

Dabei kann die Hydrierung wie weiter oben beschrieben nach dem Fachmann bekannten Methoden mit Wasserstoff und unter Verwendung eines geeigneten Katalysators erfolgen.

Im folgenden wird die Erfindung anhand von Beispielen näher erläutert. Die Erfindung ist im gesamten beanspruchten Bereich ausführbar und nicht auf die hier genannten Beispiele beschränkt.

### Beispiele:

### Beispiel 1: Antioxidative Wirkung (nicht beansprucht)

Die antioxidative Kapazität der Substanzen I-9, I-10 und I-2 wird im sog. Rancimat-Assay bestimmt. Als Vergleichssubstanzen dienen 4-t-Butyl-1-Cyclohexanol und Tocopherol. Als Positivstandard wird BHT eingesetzt. Meßgröße ist die Zeitdauer (Induktionszeit) des Anstieges der Leitfähigkeit von unstabilisiertem Sojaölder bei Luftexposition in der Wärme. Je länger die Induktionszeit, desto größer ist die stabilisierende bzw. antioxidierende Wirkung. Folgende Rahmenbedingungen wurden eingehalten: Lufteintrag 20L/h; 120°C Temperatur und Probenkonzentrationen von je 0,1% w/w im Sojaöl. Für jede Prüfsubstanz wird ein relativer Antioxidationsschutzfaktor bestimmt, der dem Verhältnis der Induktionszeiten mit und ohne Prüfsubstanz entspricht:

| | |
|---|---|
| Antioxidationschutzfaktor Tocopherol: | 1,00 |
| Antioxidationschutzfaktor 4-t-Butyl-1-cyclohexanol: | 1,05 |
| Antioxidationschutzfaktor Substanz I-9: | 1,12 |
| Antioxidationschutzfaktor Substanz I-10: | 1,14 |
| Antioxidationschutzfaktor Substanz I-2: | 1,15 |
| Antioxidationschutzfaktor Positivstandard BHT: | 1,30 |

### Beispiel 2: Desodorierende Wirkung

Die Substanzen I-2 (nach Apfel) und I-20 (nach Himbeere) weisen einen angenehmen Eigengeruch auf und eignen sich zur Geruchsüberdeckung in Deodorantien.

### Beispiel 3: Antimikrobielle Wirkung gegen Staphylococcus epidermis (Geruchskontrolle, Deowirksamkeit)

Zur Probenvorbereitung werden die Untersuchungssubstanzen zu je 1,0% im Wasser-Ethanolgemisch (12% Ethanol) gelöst bzw. homogen dispergiert und anschließend mit Bakteriensuspension im Verhältnis 1 zu 2 verdünnt. *S. epidermidis* (ATCC 12228) wird für 24h bei 35°C auf CASO-Agar (Merck 1.05458) aerob vorinkubiert. Anschließend wird in CASO-Bouillon (Merck 1.05459, doppelt konzentriert) eine Bakteriensuspension mit einer optischen Dichte (OD) von ca. 1,0 hergestellt und nochmals 1:10 in CASO-Bouillon verdünnt. Pro Well werden zu 150µl Bakteriensuspension 150µl frisch vorbereitete Probe gegeben. Die optische Dichte wird im zeitlichen Verlauf bei 37°C mit einem Bioscreen C™ Analyzer im Vergleich zur Neagtivkontrolle (= Vehicle ohne Prüfsubstanz) bestimmt. Im Vergleich zur Negativkontrolle reduzierte optische Dichten belegen wachstumsinhibierende Substanzwirkungen Ergebnis als Optische Dichte der Bakteriensuspension nach 20 Stunden Einwirkzeit:
1. Versuch:
   OD₂₀ₕ(Negativkontrolle): 0,80
   OD₂₀ₕ(Substanz I-2): 0,30
   OD₂₀ₕ(Substanz I-10): 0,30
2. Versuch:
   OD₂₀ₕ(Negativkontrolle): 0,65
   OD₂₀ₕ(Substanz I-9): 0,30
   OD₂₀ₕ(Substanz I-20): 0,20

### Beispiel 4: Antimikrobielle Wirkung gegen Propionibacterium acnes (Aknekontrolle)

Zur Probenvorbereitung werden die Untersuchungssubstanzen zu je 1,0% im Wasser-Ethanolgemisch (12% Ethanol) gelöst bzw. homogen dispergiert und anschließend mit Bakteriensuspension im Verhältnis 1 zu 2 verdünnt. *P. acnes* (ATCC 6919) wird für 48h bei 35°C auf RMC-Agar (Merck 1.05410) anaerob vorinkubiert. Anschließend wird in RMC-Bouillon (Merck 1.05411, doppelt konzentriert) eine Bakteriensuspension mit einer optischen Dichte (OD) von ca. 1,5 hergestellt. Pro Well werden zu 150µl Bakteriensuspension 150µl frisch vorbereitete Probe sowie 100µL Paraffin gegeben. Die optische Dichte wird im zeitlichen Verlauf bei 37°C mit einem Bioscreen C™ Analyzer im Vergleich zur Neagtivkontrolle (= Vehicle ohne Prüfsubstanz) bestimmt. Im Vergleich zur Negativkontrolle reduzierte optische Dichten belegen wachstumsinhibierende Substanzwirkungen.

Ergebnis als Optische Dichte der Bakteriensuspension nach 20 Stunden Einwirkzeit:
OD₂₀ₕ(Negativkontrolle): 1,0
OD₂₀ₕ(Substanz I-2): 0,45
OD₂₀ₕ(Substanz I-9): 0,50
OD₂₀ₕ(Substanz I-10): 0,45
OD₂₀ₕ(Substanz I-20): 0,70

### Beispiel 5: Keimzahlbestimmung zum Nachweis der bakteriostatischen bzw. bakteriziden Wirkung

Zur Probenvorbereitung werden die Untersuchungssubstanzen zu je 1,0% im Wasser-Ethanolgemisch (12% Ethanol) gelöst bzw. homogen dispergiert (= Probe). Eine Bakteriensuspension von *S. epidermidis* (ATCC 12228) mit einer optischen Dichte von 1,0 in CASO-Bouillon (Merck 1.05459) wird 1:10000 mit CASO-Bouillon verdünnt. Man gibt zu 500µL Bakteriensuspension 500µL Probe und inkubiert bei 35°C für 24h. Nach der Inkubation werden die Testansätze in 10er-Schritten bis -6 verdünnt und je 0,1mL auf CASO-Agar (Merck 1.05458) ausplattiert und bei 35°C für 24h bebrütet.

Zur Bestimmung der Startkeimzahl werden 0,1 mL der verwendeten Bakteriensuspension auf CASO-Agar (Merck 1.05458) ausplattiert und für 24h bei 35°C inkubiert.

Anschließend werden die Kolonien gezählt und die Keimzahl pro mL im Vergleich zur Negativkontrolle (12% Ethanol in Wasser) ermittelt.Ergebnis:

| Testsubstanz | Startkeimzahl KBE/mL | Endkeimzahl KBE/mL | Bermerkung |
|---|---|---|---|
| I-10 | 3,8 x 10⁴ | 2,0 x 10³ | bakteriostatisch |
| I-9 | 3,8 x 10⁴ | 2,0 x 10³ | bakteriostatisch |
| I-2 | 3,8 x 10⁴ | 2,9 x 10⁴ | bakteriostatisch |
| I-20 | 3,8 x 10⁴ | 0 | bakterizid |
| Negativkontrolle (12%ige wässrige Ethanollösung) | 3,8 x 10⁴ | 5,0 x 10⁷ | - |

### Beispiel 6: Antimikrobielle Wirkung

Die Vorgehensweise entspricht der in den Beispielen 4 und 5 beschriebenen unter Verwendung der nachfolgend aufgeführten Mikroorganismen. Die angegebenen Konzentrationen sind die finalen Einsatzkonzentrationen. Zur Herstellung der Stammlösung wurden die doppelte finale Konzentration im angegebenen Lösungsmittel hergestellt. Diese wurde anschließend 1:2 mit der entsprechenden Keimsuspension verdünnt. Bei Aspergillus brasiliensis erfolgte die Keimzahlbestimmung abweichend nach 48h.

| *Substanz* | *Konz.* | *Lösungsmittel* | *Keim* | *Keimzahl t₀* | *Keimzahl t₂₄ₕ* | *Δlog* |
|---|---|---|---|---|---|---|
| I-2 | 0,5% | H₂O _{(12% Ethanol)} | Propionibacterium acnes | 2,0 x 10⁴ | 1,2 x 10⁴ | -0,22 |
| I-9 | 0,5% | H₂O _{(12% Ethanol)} | Propionibacterium acnes | 2,0 x 10⁴ | 8,0 x 10² | -1,40 |
| - | - | H₂O _{(12% Ethanol)} | Propionibacterium acnes | 2,0 x 10⁴ | 4,3 x 10⁶ | +2,33 |
| I-20 | 1,0% | H₂O | Propionibacterium acnes | 7,8 x 10⁵ | 1,3 x 10⁴ | -1,78 |
| I-2 + Phenoxy ethanol | 1,0% + 0,5% | H₂O | Propionibacterium acnes | 7,8 x 10⁵ | 0 | -5,89 |
| - | - | H₂O | Propionibacterium acnes | 7,8 x 10⁵ | 3,9 x 10⁸ | +2,70 |
| I-2 | 0,5% | H₂O _{(12% Ethanol)} | Staphylococcus epidermidis | 3,4 x 10⁴ | 2,0 x 10⁴ | -0,23 |
| I-9 | 0,5% | H₂O _{(12% Ethanol)} | Staphylococcus epidermidis | 3,4 x 10⁴ | 6,0 x 10³ | -0,75 |
| - | - | H₂O _{(12% Ethanol)} | Staphylococcus epidermidis | 3,4 x 10⁴ | 6,6 x 10⁷ | +3,29 |
| I-2 + Phenoxy ethanol | 1,0% + 0,5% | H₂O | Staphylococcus epidermidis | 9,4 x 10⁴ | 1,2 x 10⁴ | -0,89 |
| - | - | H₂O | Staphylococcus epidermidis | 9,4 x 10⁴ | 1,9 x 10⁸ | +3,31 |
| I-2 | 0,5% | H₂O _{(12% Ethanol)} | Corynebacterium xerosis | 3,5 x 10⁴ | 0 | -4,54 |
| I-9 | 0,5% | H₂O _{(12% Ethanol)} | Corynebacterium xerosis | 3,5 x 10⁴ | 0 | -4,54 |
| - | - | H₂O _{(12% Ethanol)} | Corynebacterium xerosis | 3,5 x 10⁴ | 1,7 x 10⁶ | +1,69 |
| I-2 + Phenoxy ethanol | 1,0% + 0,5% | H₂O | Corynebacterium xerosis | 6,9 x 10⁴ | 0 | -4,84 |
| I-2 + I-9 | 0,5% + 0,5% | H₂O | Corynebacterium xerosis | 6,9 x 10⁴ | 0 | -4,84 |
| - | - | H₂O | Corynebacterium xerosis | 6,9 x 10⁴ | 8,7 x 10⁶ | +2,10 |
| I-9 | 0,125% | H₂O | Corynebacterium xerosis | 1,0 x 10⁶ | 5,0 x 10⁶ | +0,70 |
| I-20 | 0,5% | H₂O | Corynebacterium xerosis | 1,0 x 10⁶ | 0 | -6,00 |
| - | - | H₂O | Corynebacterium xerosis | 1,0 x 10⁶ | 6,0 x 10⁸ | +2,78 |
| I-2 | 0,5% | H₂O _{(12% Ethanol)} | Pseudomonas aeruginosa | 6,8 x 10⁴ | 0 | -4,83 |
| I-9 | 0,5% | H₂O _{(12% Ethanol)} | Pseudomonas aeruginosa | 6,8 x 10⁴ | 9,0 x 10³ | -0,88 |
| - | - | H₂O _{(12% Ethanol)} | Pseudomonas aeruginosa | 6,8 x 10⁴ | 1,0 x 10⁶ | +1,17 |
| I-2 + Phenoxy ethanol | 1,0% + 0,5% | H₂O | Pseudomonas aeruginosa | 3,8 x 10⁵ | 0 | -5,58 |
| - | - | H₂O | Pseudomonas aeruginosa | 3,8 x 10⁵ | 2,9 x 10⁹ | +3,88 |
| I-2 | 0,5% | H₂O _{(12% Ethanol)} | Salmonella enterica | 6,0 x 10⁴ | 0 | -4,78 |
| I-9 | 0,5% | H₂O _{(12% Ethanol)} | Salmonella enterica | 6,0 x 10⁴ | 1,8 x 10⁴ | -0,52 |
| - | - | H₂O _{(12% Ethanol)} | Salmonella enterica | 6,0 x 10⁴ | 1,3 x 10⁸ | +3,34 |
| Phenoxy ethanol | 0,4% | H₂O _{(12% Ethanol)} | Salmonella enterica | 1,3 x 10⁴ | 8,2 x 10² | -1,20 |
| I-2 + Phenoxy ethanol | 0,8% + 0,4% | H₂O _{(12% Ethanol)} | Salmonella enterica | 1,3 x 10⁴ | 0 | -4,11 |
| I-2 + I-9 | 0,4% + 0,4% | H₂O _{(12% Ethanol)} | Salmonella enterica | 1,3 x 10⁴ | 0 | -4,11 |
| I-20 | 0,8% | H₂O _{(12% Ethanol)} | Salmonella enterica | 1,3 x 10⁴ | 0 | -4,11 |
| - | - | H₂O _{(12% Ethanol)} | Salmonella enterica | 1,3 x 10⁴ | 4,9 x 10⁷ | +3,58 |
| I-2 | 0,5% | H₂O | Salmonella enterica | 1,0 x 10⁶ | 5,0 x 10⁴ | -1,30 |
| - | - | H₂O | Salmonella enterica | 1,0 x 10⁶ | 1,1 x 10⁹ | +3,04 |
| I-2 | 0,5% | H₂O _{(12% Ethanol)} | Serratia marcescens | 6,8 x 10⁴ | 0 | -3,83 |
| I-9 | 0,5% | H₂O _{(12% Ethanol)} | Serratia marcescens | 6,8 x 10⁴ | 9,0 x 10³ | -0,88 |
| - | - | H₂O _{(12% Ethanol)} | Serratia marcescens | 6,8 x 10⁴ | 2,0 x 10⁷ | +2,47 |
| Phenoxy ethanol | 0,5% | H₂O | Serratia marcescens | 2,5 x 10⁵ | 1,7 x 10³ | -2,17 |
| I-2 + Phenoxy ethanol | 1,0% + 0,5% | H₂O | Serratia marcescens | 2,5 x 10⁵ | 0 | -5,40 |
| - | - | H₂O | Serratia marcescens | 2,5 x 10⁵ | 8,8 x 10⁸ | +3,55 |
| I-2 | 0,5% | H₂O | Serratia marcescens | 2,9 x 10⁵ | 9,0 x 10⁵ | +0,49 |
| - | - | H₂O | Serratia marcescens | 2,9 x 10⁵ | 9,7 x 10⁸ | +3,52 |
| I-2 | 0,5% | H₂O _{(12% Ethanol)} | Candida albicans | 3,4 x 10⁴ | 3,0 x 10² | -2,05 |
| I-9 | 0,5% | H₂O _{(12% Ethanol)} | Candida albicans | 3,4 x 10⁴ | 1,5 x 10¹ | -3,36 |
| - | - | H₂O _{(12% Ethanol)} | Candida albicans | 3,4 x 10⁴ | 1,8 x 10⁶ | +1,72 |
| I-20 | 1,0% | H₂O _{(12% Ethanol)} | Candida albicans | 6,0 x 10³ | 0 | -3,78 |
| - | - | H₂O _{(12% Ethanol)} | Candida albicans | 6,0 x 10³ | 5,0 x 10⁵ | +1,92 |
| I-2 | 0,5% | H₂O | Candida albicans | 3,8 x 10⁵ | 2,5 x 10⁵ | -0,18 |
| I-9 | 0,5% | H₂O | Candida albicans | 3,8 x 10⁵ | 8,0 x 10⁶ | +1,32 |
| I-20 | 0,5% | H₂O | Candida albicans | 3,8 x 10⁵ | 0 | -5,58 |
| - | - | H₂O | Candida albicans | 3,8x10⁵ | 3,0 x 10⁷ | +1,90 |
| I-2 | 0,5% | H₂O _{(12% Ethanol)} | Echerichia coli | 4,6 x 10⁴ | 1,8 x 10³ | -1,41 |
| I-9 | 0,5% | H₂O _{(12% Ethanol)} | Echerichia coli | 4,6 x 10⁴ | 3,8 x 10² | -2,08 |
| - | - | H₂O _{(12% Ethanol)} | Echerichia coli | 4,6 x 10⁴ | 1,5 x 10⁷ | +2,51 |
| Methylpa raben | 0,2% | H₂O _{(12% Ethanol)} | Echerichia coli | 6,3 x 10⁴ | 4,8 x 10³ | -1,12 |
| I-2 + Methylpa raben | 0,4% + 0,2% | H₂O _{(12% Ethanol)} | Echerichia coli | 6,3 x 10⁴ | 0 | -4,80 |
| Phenoxy ethanol | 0,4% | H₂O _{(12% Ethanol)} | Echerichia coli | 6,3 x 10⁴ | 2,0 x 10¹ | -3,50 |
| I-2 + Phenoxy ethanol | 0,8% + 0,4% | H₂O _{(12% Ethanol)} | Echerichia coli | 6,3 x 10⁴ | 0 | -4,80 |
| I-20 | 0,8% | H₂O _{(12% Ethanol)} | Echerichia coli | 6,3 x 10⁴ | 0 | -4,80 |
| - | - | H₂O _{(12% Ethanol)} | Echerichia coli | 6,3 x 10⁴ | 5,8 x 10⁷ | +2,96 |
| I-9 | 0,5% | H₂O _{(12% Ethanol)} | Staphylococcus aureus | 4,2 x 10⁴ | 1,4 x 10² | -2,48 |
| - | - | H₂O _{(12% Ethanol)} | Staphylococcus aureus | 4,2 x 10⁴ | 2,0 x 10⁸ | +3,68 |
| Phenoxy ethanol | 0,5% | H₂O _{(12% Ethanol)} | Staphylococcus aureus | 7,2 x 10⁴ | 7,0 x 10¹ | -3,01 |
| I-2 + Phenoxy ethanol | 1,0% + 0,5% | H₂O _{(12% Ethanol)} | Staphylococcus aureus | 7,2 x 10⁴ | 0 | -4,86 |
| I-2 + I-9 | 0,5% + 0,5% | H₂O _{(12% Ethanol)} | Staphylococcus aureus | 7,2 x 10⁴ | 6,0 x 10¹ | -3,80 |
| - | - | H₂O _{(12% Ethanol)} | Staphylococcus aureus | 7,2 x 10⁴ | 9,0 x 10⁷ | +3,10 |
| I-20 | 0,5% | H₂O _{(12% Ethanol)} | Staphylococcus aureus | 1,1 x 10⁴ | 0 | -4,04 |
| - | - | H₂O _{(12% Ethanol)} | Staphylococcus aureus | 1,1 x 10⁴ | 3,0 x 10⁷ | +3,44 |
| I-2 | 0,5% | H₂O _{(12% Ethanol)} | Aspergillus brasiliensis | 2,0 x 10⁴ | 1,0 x 10⁴ | -0,30 |
| I-9 | 0,5% | H₂O _{(12% Ethanol)} | Aspergillus brasiliensis | 2,0 x 10⁴ | 8,0 x 10² | -1,40 |
| - | - | H₂O _{(12% Ethanol)} | Aspergillus brasiliensis | 2,0 x 10⁴ | 3,0 x 10⁵ | +1,18 |
| Phenoxy ethanol | 0,5% | H₂O _{(12% Ethanol)} | Aspergillus brasiliensis | 8,0 x 10² | 4,0 x 10² | -0,30 |
| I-2 + Phenoxy ethanol | 1,0% + 0,5% | H₂O _{(12% Ethanol)} | Aspergillus brasiliensis | 8,0 x 10² | 3,0 x 10¹ | -1,43 |
| I-20 | 1,0% | H₂O _{(12% Ethanol)} | Aspergillus brasiliensis | 8,0 x 10² | 2,0 x 10² | -0,60 |
| - | - | H₂O _{(12% Ethanol)} | Aspergillus brasiliensis | 8,0 x 10² | 1,2 x 10⁴ | +1,18 |
| I-2 | 1,0% | H₂O | Malassezia furfur | 4,0 x 10² | 0 | -2,60 |
| I-20 | 1,0% | H₂O | Malassezia furfur | 4,0 x 10² | 0 | -2,60 |
| - | - | H₂O | Malassezia furfur | 4,0 x 10² | 3,0 x 10⁴ | +1,86 |

### Beispiel 7: Synthese von 2-Hydroxy-cyclohexanecarboxylic acid 2-ethyl-hexyl ester (Substanz I-9)

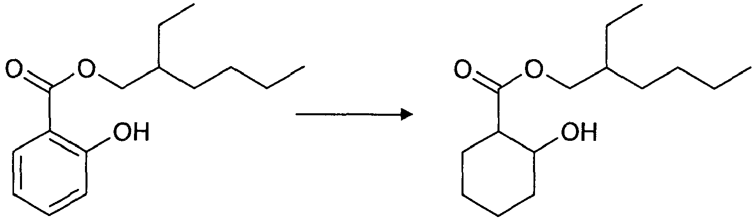

50g 2-Hydroxy-benzoic acid 2-ethylhexylester (Eusolex® OS) werden in 318mL 2-Propanol gelöst. Anschließend werden 5g Katalysator Rh-C-5% (53,6% Wasser) zugegeben. Die Hydrierung erfolgt mit Wasserstoff 3.0 bei 65°C und 5bar Druck. Nach vollständiger Hydrierung wird der Katalysator durch Filtration abgetrennt. Das Filtrat wird im Vakuum vom Lösungsmittel befreit und über einen Spritzenfilter (0,2µm) filtriert. Man erhält das analysenreine Produkt als farbloses Öl im Gemisch von cis- und trans-Isomeren im Verhältnis von ca. 5/1.

### Beispiel 8: Synthese von 2-Hydroxy-cyclohexanecarboxylic acid 3,3,5-trimethyl-cyclohexyl ester (Substanz I-10)

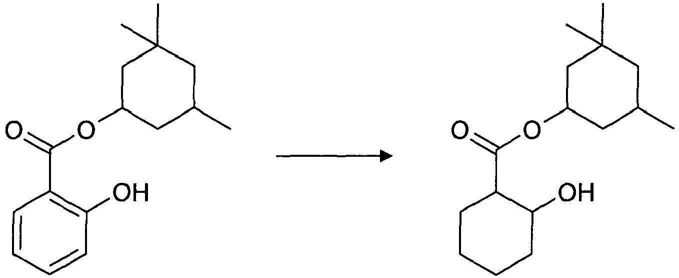

50g 3,3,5-trimethyl-cyclohexyl ester (Eusolex® HMS) werden in 318mL 2-Propanol gelöst. Anschließend werden 5g Katalysator Rh-C-5% (53,6% Wasser) zugegeben. Die Hydrierung erfolgt mit Wasserstoff 3.0 bei 80°C und 5bar Druck. Nach vollständiger Hydrierung wird der Katalysator durch Filtration abgetrennt. Das Filtrat wird über Seitzfilter (a) 0.5µm, anschließend b) 0,2µm) filtriert. Das Filtrat wird im Vakuum vom Lösungsmittel befreit. Man erhält analysenreines Produkt als farbloses Öl im Gemisch von cis- und trans-Isomeren im Verhältnis von ca. 5/1.

### Beispiel 9: Synthese von 4-Hydroxy-cyclohexanecarboxylic acid butyl ester (Substanz I-20)

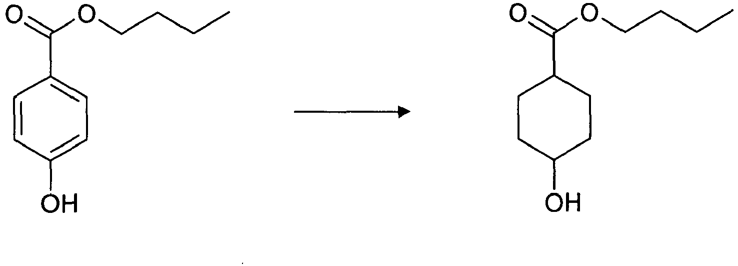

30g 4-Hydroxy-benzoic acid butylyl ester werden in 100mL Tetrahydrofuran gelöst. Anschließend werden 3,0g Katalysator Rh-C-5% (ca. 50% Wasser) zugegeben. Die Hydrierung erfolgt mit Wasserstoff 3.0 bei 65°C und 5bar. Nach vollständiger Hydrierung wird der Katalysator durch Filtration abgetrennt. Das Filtrat wird im Vakuum vom Lösungsmittel befreit. Man erhält analysenreines Produkt als farbloses Öl im Gemisch von cis- und trans-Isomeren von ca. 5/2.

**Beispiel 10:** Messung der dynamischen Viskositäten zur Abschätzung der Spreitbarkeit bei 25°C

Nach Messung der Dichten und kinematischen Viskositäten mit dem Ubbelohde-Viskosimeter ergeben sich folgende dynamische Viskositäten:
Kinematische Viskosität von I-2: 15,5 mm²/s
   Dichte von I-2: 1,0564 g/cm³; 25°C
   Dynamische Viskosität I-2 = Kinematische Viskosität I-2 x Dichte I-2 = 16,4 [mPas]
Kinematische Viskosität von I-9: 33,0 mm²/s
   Dichte von I-9: 0,9752 g/cm³; 25°C
   Dynamische Viskosität I-9 = Kinematische Viskosität I-9 x Dichte I-9 = 32,2 [mPas]
Da Spreitbarkeiten und Viskositäten kosmetischer Emollients gut korrelieren, läßt sich aus den ermittelten Viskositäten auf gute Spreitbarkeiten schließen.

### Beispiel A: Antischuppenschampoo

| INCI | % (w/w) | % (w/w) | % (w/w) | % (w/w) | % (w/w) | % (w/w) |
|---|---|---|---|---|---|---|
| Acrylates/C10-30 Alkyl Acrylate Crosspolymer | 1,0 | 1,0 | 1,0 | 1,0 | 1,0 | 1,0 |
| Propylenglycol | 5,0 | 5,0 | 5,0 | 5,0 | 5,0 | 5,0 |
| Sodium Lauryl Sulfate (30%) | 15,0 | 15,0 | 15,0 | 15,0 | 15,0 | 15,0 |
| Sodium Laureth Sulfate (28%) | 15,0 | 15,0 | 15,0 | 15,0 | 15,0 | 15,0 |
| Cocamidopropyl betaine (35%) | 5,0 | 5,0 | 5,0 | 5,0 | 5,0 | 5,0 |
| Substanz I-2 | 0,5 | 0,5 | | 1,0 | | |
| Substanz I-9 | | 0,5 | | | 0,75 | 2,0 |
| Substanz I-10 | | | 0,5 | 1,0 | | |
| Substanz I-20 | | | 0,5 | | 0,75 | |
| Polyquaternium-10 | 0,30 | 0,30 | 0,30 | 0,30 | 0,30 | 0,30 |
| DMDM Hydantoin (and) Iodopropynyl Butylcarbamate | 0,3 | 0,3 | 0,3 | 0,3 | 0,3 | 0,3 |
| Polyquaternium-39 | 1,0 | 1,0 | 1,0 | 1,0 | 1,0 | 1,0 |
| Dimethicone PEG-7 Isostearate | 0,5 | 0,5 | 0,5 | 0,5 | 0,5 | 0,5 |
| Zinc Pyrithione (48%) | 2,0 | 1,0 | 1,0 | | 0,5 | 0,5 |
| Fragrance | 1,0 | 1,0 | 1,0 | 1,0 | 1,0 | 1,0 |
| Dye | 0,1 | 0,1 | 0,1 | 0,1 | 0,1 | 0,1 |
| Aqua | Ad 100 | Ad 100 | Ad 100 | Ad 100 | Ad 100 | Ad 100 |
| Sodium Hydroxide | q.s. to pH 6 | q.s. to pH 6 | q.s. to pH 6 | q.s. to pH 6 | q.s. to pH 6 | q.s. to pH 6 |

**Beispiel B: Antiacne Gesichtsreinigungsgel**

| INCI | % (w/w) | % (w/w) | % (w/w) | % (w/w) | % (w/w) | % (w/w) |
|---|---|---|---|---|---|---|
| Propylene Glycol | 2,5 | 2,5 | 2,5 | 2,5 | 2,5 | 2,5 |
| Dehydroxanthan Gum | 1,5 | 1,5 | 1,5 | 1,5 | 1,5 | 1,5 |
| Cocamidopropyl Betaine | 10,0 | 10,0 | 10,0 | 10,0 | 10,0 | 10,0 |
| Salicylic acid | 2,0 | 0,5 | 0,5 | 1,0 | | |
| Sodium Lactate | 0,5 | 1,0 | | | | 1,0 |
| Triclosan | 0,2 | | | | 0,2 | |
| 1,3.Butylene glycol | 3,5 | 3,5 | 3,5 | 3,5 | 3,5 | 3,5 |
| Substanz I-2 | 0,5 | 0,5 | | 1,0 | 1,0 | |
| Substanz I-9 | | 0,5 | | | 0,75 | 2,0 |
| Substanz I-10 | | | 0,5 | 1,0 | | |
| Substanz I-20 | | | 0,5 | | | |
| Menthyl Lactate | 0,2 | 0,2 | 0,2 | 0,2 | 0,2 | 0,2 |
| PEG-40 Hydrogenated Castor Oil | 0,5 | 0,5 | 0,5 | 0,5 | 0,5 | 0,5 |
| C8-16-decyl Glucoside | 5,0 | 5,0 | 5,0 | 5,0 | 5,0 | 5,0 |
| DMDM Hydantoin (and) Iodopropynyl Butylcarbamate | 0,5 | 0,5 | 0,5 | 0,5 | 0,5 | 0,5 |
| Lactose (and) Cellulose (and) Hydroxypropyl Methylcellulose (and) Ultrmarine Blue (C177007) (and) Triclosan | 0,4 | 0,4 | 0,4 | 0,4 | 0,4 | 0,4 |
| Fragrance | 0,5 | 0,5 | 0,5 | 0,5 | 0,5 | 0,5 |
| Aqua | Ad 100 | Ad 100 | Ad 100 | Ad 100 | Ad 100 | Ad 100 |

### Beispiel C: Antitranspirant Spray (Aerosol)

### a) Suspensionsherstellung

| INCI | % (w/w) | % (w/w) | % (w/w) | % (w/w) | % (w/w) | % (w/w) |
|---|---|---|---|---|---|---|
| Aluminium Chlorohydrate | 35,0 | 25,0 | 20,0 | 10,0 | 25,0 | |
| Ethylhexylglycerin | 0,5 | 0,5 | 0,5 | 0,5 | 0,5 | 0,5 |
| C12-15 Alkylbenzoate | 5,0 | 5,0 | 5,0 | 5,0 | 5,0 | 5,0 |
| Diasteardimonium Hectorite | 5,0 | 5,0 | 5,0 | 5,0 | 5,0 | 5,0 |
| Silver citrate | 0,0015 | 0,0025 | | | | |
| Silver lactate | | | 0,0025 | 0,0015 | | |
| Propylencarbonate | 1,0 | 1,0 | 1,0 | 1,0 | 1,0 | 1,0 |
| Substanz 2 | | | | 1,0 | 1,5 | |
| Substanz 9 | 0,5 | 1,0 | 2,0 | 1,0 | | 3,5 |
| Substanz 10 | 2,0 | 1,5 | 0,5 | 1,0 | | 1,0 |
| Substanz 21 | | | | 1,0 | 1,5 | |
| Fragrance | 4,0 | 4,0 | 4,0 | 4,0 | 4,0 | 4,0 |
| Cyclopentasiloxane | Ad 100 | Ad 100 | Ad 100 | Ad 100 | Ad 100 | Ad 100 |

### b) Treibgasgemisch aus Propan/Isobutan/Butan (1/1/1)

### c) Die Aerosolherstellung erfolgt durch Verpackung der Suspension in eine

Aerosoldose zusammen mit dem Treibgas im Verhältnis
Suspension/Treibgas = 20/80.

### Beispiel D: Antitranspirant / Deodorant (Gel)

| Ingredients | % (w/w) | % (w/w) | % (w/w) | % (w/w) | % (w/w) | % (w/w) |
|---|---|---|---|---|---|---|
| Aluminium Zirconium Octachlorohydrex GLY | 10,0 | 15,0 | 20,0 | 10,0 | 15,0 | |
| Alcohol denat. | 5,0 | 5,0 | 5,0 | 5,0 | 5,0 | 5,0 |
| Cyclomethicone | 15,0 | 15,0 | 10,0 | 20,0 | 10,0 | 20,0 |
| Propylene Glycol | 10,0 | 5,0 | 5,0 | 15,0 | 5,0 | 15,0 |
| Dimethicone | 2,5 | 2,5 | 2,5 | 2,5 | 2,5 | |
| Trisiloxane | 5,0 | 2,5 | 2,5 | 5,0 | 2,5 | 5,0 |
| Substanz 2 | | | | 1,0 | 1,5 | |
| Substanz 9 | 0,5 | 1,0 | 2,0 | 1,0 | | 3,5 |
| Substanz 10 | 2,0 | 1,5 | 0,5 | 1,0 | | 1,0 |
| Substanz 21 | | | | 1,0 | 1,5 | |
| Maltosin | 0,1 | 0,1 | 0,1 | 0,1 | 0,1 | 0,5 |
| Calcium Chloride | 0,3 | 0,3 | 0,3 | 0,3 | 0,3 | 0,3 |
| PEG/PPG-18/18 Dimethicone | 0,75 | 0,75 | 0,75 | 0,75 | 0,75 | 0,75 |
| Fragrance | qs | qs | qs | qs | qs | qs |
| Water | Ad 100 | Ad 100 | Ad 100 | Ad 100 | Ad 100 | Ad 100 |

### Beispiel E: Antitranspirant / Deodorant (Roll-on)

| Ingredients | % (w/w) | % (w/w) | % (w/w) | % (w/w) | % (w/w) | % (w/w) |
|---|---|---|---|---|---|---|
| Aluminium Chlorohydrate | 25,0 | 25,0 | 25,0 | 25,0 | 25,0 | 25,0 |
| Isoceteth-20 | 1,5 | 1,5 | 1,5 | 1,5 | 1,5 | 1,5 |
| Mineral Oil | 3,0 | 3,0 | 3,0 | 3,0 | 3,0 | 3,0 |
| Butylene Glycol | 2,0 | 2,0 | 2,0 | 2,0 | 2,0 | 2,0 |
| Gylceryl Isostearate | 1,5 | 1,5 | 1,5 | 1,5 | 1,5 | 1,5 |
| Laureth-7 Citrate | 1,0 | 1,0 | 1,0 | 1,0 | 1,0 | 1,0 |
| Palmitamidopropyl Trimonium Chloride | 0,3 | 0,3 | 0,3 | 0,3 | 0,3 | 0,3 |
| Propylene Glycol | 0,5 | 0,5 | 0,5 | 0,5 | 0,5 | 0,5 |
| PEG-150 Distearate | 0,3 | 0,3 | 0,3 | 0,3 | 0,3 | 0,3 |
| Substanz 2 | | | | 1,0 | 1,5 | |
| Substanz 9 | 0,5 | 1,0 | 2,0 | 1,0 | | 3,5 |
| Substanz 10 | 2,0 | 1,5 | 0,5 | 1,0 | | 1,0 |
| Substanz 21 | | | | 1,0 | 1,5 | |
| Calcium Chloride | 0,3 | 0,3 | 0,3 | 0,3 | 0,3 | 0,3 |
| PEG/PPG-18/18 Dimethicone | 0,75 | 0,75 | 0,75 | 0,75 | 0,75 | 0,75 |
| Fragrance | qs | qs | qs | qs | qs | qs |
| Water | Ad 100 | Ad 100 | Ad 100 | Ad 100 | Ad 100 | Ad 100 |

### Beispiel F: Anti-Aknemittel

Nachfolgende Zusammensetzungsvarianten enthalten erfindungsgemäße Stoffe in Einsatzkonzentration von jeweils zwischen 0,1 und 10 Gewichtsprozenten. Inhaltsstoffe werden nach INCI angegeben.

### Variante a)

water, TEA-cocoate, cocamide DEA, potassium cocoate, TEA-laurate/myristate, butylene glycol, glycerin, glycol distearate, PEG-55 stearate, sodium cocoamphoacetate, chocolate vine (akebia quinata) root extract, scutellaria baicalensis root extract, chinese blackberry extract, angelica acutiloba root extract, coix lacryma-jobi (job's tears) seed extract, sodium polyaspartate, melothria heterophylla extract, disodium EDTA, trisodium EDTA, ethanol, xanthan gum, citric acid, sodium methyl cocoyl taurate, squalane, Hydroxypropyl Methylcellulose, polyquaternium-10, lauryl betaine, sodium bisulfite, sodium chloride, phenoxyethanol, methylparaben, fragrance

### Variante b)

Aqua, Cyclohexasiloxane, Isononyl Isononanoate, Propylene Glycol, Isohexadecane, Niacinamide, PEG_100 stearate, Glyceryl Stearate, Cetyl Alcohol, Kaolin, Salicylic Acid, proctone olamine, Acrylates Copolymer, PEG-4, PEG-4 Dilaurate, PEG-4 Laurate, Sodium Carbomer, Capryloyl Glycine, Capryloyl Salicylic Acid, Xanthan Gum, Isobutane, Sodium Sulfate, lodopropynyl Butylcarbamate, Chlorhexidine Digluconate, Parfum (Perfuming)

### Variante c)

Aqua, Nylon-66, Glycerin, Cyclohexasiloxane, aluminum starch octenylsucinate, PEG-2 Stearate, Prunus Armeniaca Fruit, dydrogenated polyisobutene, Cetearyl Alcohol, Triethanolamine, Salicylic Acid, Silica, Kaolin, PEG-100 Stearate, C13-14 Isoparaffin, Stearyl Alcohol, Hamamelis Virginiana Extract, hamaelis virginiana, glyceril stearate, Sarcosine, c'glycine soja, Methylparaben, Arginine PCA, Phenoxyethanol, Cinnamomum Zeylanicum Leaf Extract, Tocopherol, Alcohol, Disodium EDTA, Capryloyl Glycine, Laureth-7, oleth-1, Acrylates/stearyl Acrylate/dimethicone Methacrylate Copolymer, Polyacrylamide, Butylene Glycol, CI 42090, Parfum (Perfuming)

### Variante d)

Aqua, Glycerin, Cyclohexasiloxane, Hydrogenated Polyisobutene (Hydrogenated), Niacinamide, Isopropyl Lauroyl Sarcosinate, Ammonium Polyacryloyldimethyl Taurate, Silica, Methyl Methacrylate Crosspolymer, Sodium Hydroxide, Salicylic Acid, Nylon-12, Zinc PCA, Linoleic Acid, Pentaerythrityl Tetra-di-t-butyl Hydroxyhydrocinnamate, Capryloyl Glycine, Capryloyl Salicylic Acid, Caprylyl Glycol, Piroctone Olamine, Myristyl Myristate, Potassium Cetyl Phosphate, Glyceryl Stearate SE, Parfum (Perfuming), F.I.L B36611/1

### Variante e)

Salicylic Acid, (Aqua), D1-C 12-13 alkyl malate, Cyclohexasiloxane, Propylene Glycol, Aluminum Starch Octenylsuccinate, PEG-100 Stearate, Glyceryl Stearate, Cetyl Alcohol, PEG-4 Dilaurate, PEG-4 Laurate, Zinc PCA, Sodium Hydroxide, Capryloyl Salicylic Acid, Xanthan Gum, acrylates C10-30 alky acrylate crosspolymer, lodopropynyl Butylcarbamate, PEG-4, Parfum (Perfuming)

### Variante f)

Resorcinol, Tocopherol, acetate, Stearyl Glycyrrhetinate, Pyridoxine HCl, Cinchona Succirubra Bark Extract (Extract), Glycerin (Concentrate), Butylene Glycol, Carbomer, Hydroxypropyl Methylcellulose, Propylene Glycol Alginate, polyoxyethylene hydrogenated castor oil, Alcohol, Triethanolamine, Aqua (Purified), Disodium EDTA, Methylparaben, Propylparaben, BHT, Parfum (Perfuming)

### Variante g)

biosulfur fluid, Dipotassium Glycyrrhizate, Aqua, Butylene Glycol, hydrolysed rice leaf extract, Phellodendron Amurense Bark Extract (Extract), Hydroxyproline, Salvia Officinalis Leaf Extract (Extract), Camellia Sinensis Leaf (Extract), Rosmarinus Officinalis Extract (Extract), Glycerin (Cosmetic Grade), Pentylene Glycol, Carbomer, Xanthan Gum, Camphor, Menthol, Potassium Hydroxide
Alcohol Denat., Aqua, Acrylates/C10-30 Alkyl Acrylate Crosspolymer, Hamamelis Virginiana Extract, Laminaria Saccharina Extract (Extract), Salicylic Acid, Sucrose, Caffeine, Butylene Glycol, Benzalkonium Chloride, ILN 32308

### Variante h)

Aqua, Alcohol Denat., Glycerin, Acrylates/C10-30 Alkyl Acrylate Crosspolymer, Triethanolamine, Salicylic Acid, Dipotassium Glycyrrhizate, Copper Gluconate, Zinc Gluconate, Potassium Alum, Vitreoscilla Ferment (Extract), Pentylene Glycol, Sodium Citrate, Parfum (Perfuming), Limonene, Benzyl Benzoate, Linalool, Alpha-isomethyl Ionone, F.I.L. B28365/2

### Variante i)

Alcohol, Aqua, Polyacrylamide, C13-14 Isoparaffin, Laureth-7, Hamamelis Virginiana Water, Glycerin, Salicylic Acid, Cyclopentasiloxane, C12-15 Alkyl Lactate, Phenoxyethanol, Cetyl Lactate, dipropylene glycol isoceteth-20 acetate, Cocamidopropyl PG-dimonium Chloride Phosphate, Polysorbate 20, Phenethyl Dimethicone, Dehydroxanthan Gum, Parfum (Perfuming), Benzalkonium Chloride, Propylene Glycol, Ammonium Hydroxide, T-butyl Alcohol, Tetrasodium EDTA, Capryloyl Glycine, Sarcosine, Cinnamomum Zeylanicum Bark Extract (Extract), Portulaca Oleracea Extract (Extract), Cedrus Atlantica Bark Extract (Extract), Denatonium Benzoate

### Variante j)

Alcohol, Aqua, Propylene Glycol, PEG-6, Salicylic Acid, PPG-33 Butyl Ether, Potassium Hydroxide, Benzophenone-1, Parfum (Perfuming), BHT, Isopropyl Myristate, Disodium EDTA, Lysine Carboxymethyl Cysteinate, Isododecane, Aloe Barbadensis Leaf Juice, Sodium C8-16 Isoalkylsuccinyl Lactoglobulin Sulfonate, Polystyrene/hydrogenated Polyisopentene Copolymer (Hydrogenated), Chamomilla Recutita Flower Extract (Extract), CI 42090, CI 17200

### Variante k)

Aqua, Polyethylene, Hydrogenated Vegetable Oil (Hydrogenated, Vegetable Based), Hydrogenated Polydecene (Hydrogenated), Stearyl Alcohol, Glycerin, Glyceryl Stearate, Alcohol, PEG-40 Stearate, C12-15 Pareth-12, Cetyl Alcohol, Hamamelis Virginiana Extract (Extract), Menthol, Parfum (Perfuming), Benzethonium Chloride, Sorbic Acid, BHT, CI 15985, Eucalyptus Globulus Leaf Extract (Extract), CI 47005, CI 42090, Disodium EDTA

### Variante I)

Aqua, Alcohol, PPG-5-ceteth-20, Glycerin, Salicylic Acid, Parfum (Perfuming), Methyl Lactate, Denatonium Benzoate

### Variante m)

Aqua, Alcohol Denat., Dipropylene Glycol, Glycerin, Salicylic Acid, Laminaria Saccharina Extract

### Variante n)

Polyoxyethylene ethers, Lauramine Oxide, Polyethylene (Granules), Propylene Glycol, benzalconium chlorate, Alcohol, Potassium Sorbate, Silica, yellow CI 47005, Parfum (Perfuming), Aqua (Purified)

### Variante o)

Aqua, sodium sulfonate C14-16 olefin, Cocamidopropyl Betaine, salicilic acid, Glycerin, Decyl Glucoside, alkyl C12-13 potassium phosphate, Acrylates Copolymer, glycol distearate/ laureth-4/ cocamidopropyl betaine, Parfum (Perfuming), Citric Acid, portulaca oleracea (little hogweed) extract, Cedrus Atlantica Bark Extract (Extract), caprylyl glycine / sarcosine / cinnamomum zeylanicum (ceylon cinnamon) bark extract, Sodium Hydroxide

### Variante p)

Aqua, Kaolin, Glycerin, Butylene Glycol, Zea Mays Starch, CI 77891, Decyl Glucoside, Polyethylene, Sodium Laureth Sulfate, Chondrus Crispus, PEG-7 Glyceryl Cocoate, Jojoba Esters, Phenoxyethanol, Salicylic Acid, Tetrasodium EDTA, Xanthan Gum, Triethanolamine, Methylparaben, Zinc Gluconate, Menthol, Butylphenyl Methylpropional, CI 77007, Propylene Glycol, Limonene, Linalool, Eucalyptus Globulus Leaf Extract (Extract), Parfum (Perfuming), F.I.L. B32440

### Variante q)

Aqua, Sodium C14-16 Olefin Sulfonate, Glycerin, Cocamidopropyl Betaine, Salicylic Acid, Sodium Methyl Cocoyl Taurate, Acrylates Copolymer, Sodium Lauroamphoacetate, Parfum (Perfuming), Citric Acid, Glycol Distearate, Sodium Chloride, Menthyl Lactate, Cocamidopropyl PG-dimonium Chloride Phosphate

### Variante r)

Aqua, Aqua, Decyl Glucoside, Polysorbate 20, Ceteareth-60 Myristyl Glycol, Disodium Cocoamphodiacetate, Glycerin, Sodium Lauroyl Sarcosinate, Methyl Gluceth-20, Benzoic Acid, Cetrimonium Bromide, curcurbita pepo seed oil, Disodium EDTA, Parfum (Perfuming), CI 61570, Lactic Acid, Phenoxyethanol, CI 19140, Zinc Gluconate

### Variante s)

Aqua, Cyclomethicone, Propylene Glycol, Glycerin, Polyacrylamide, Polymethyl Methacrylate, Aqua, C13-14 Isoparaffin, Zinc Gluconate, Butylparaben, Cetrimonium Bromide, cucurbita pepo (pumpkin) seed oil (cucurbita pepo), Dimethiconol, Disodium EDTA, Parfum (Perfuming), Laureth-7, Pyridoxine HCl, Salicylic Acid, Triethanolamine

### Variante t)

Aqua, Cetyl Alcohol, Cyclomethicone, Polysorbate 60, Glycolic Acid, Aqua, SD Alcohol 39-C, Polymethyl Methacrylate, Sodium Hydroxide, Cetearyl Alcohol, Arginine HCl, BHT, Ceteareth-33, Dimethiconol, Parfum (Perfuming), Potassium Sorbate, CI 17200, retinal undecyl rhamnoside

### Variante u)

Aqua, Cyclohexasiloxane, Isononyl Isononanoate, Propylene Glycol, Isohexadecane, Niacinamide, PEG_100 stearate, Glyceryl Stearate, Cetyl Alcohol, Kaolin, Salicylic Acid, proctone olamine, Acrylates Copolymer, PEG-4, PEG-4 Dilaurate, PEG-4 Laurate, Sodium Carbomer, Capryloyl Glycine, Capryloyl Salicylic Acid, Xanthan Gum, Isobutane, Sodium Sulfate, lodopropynyl Butylcarbamate, Chlorhexidine Digluconate, Parfum (Perfuming)

### Variante v)

Aqua, Dipropylene Glycol, SD Alcohol 39-C, Zinc Gluconate, cucurbita pepo pump-kin seed oil (cucurbita pepo), Parfum (Perfuming), PEG-40 Hydrogenated Castor Oil (Hydrogenated), PPG-26-buteth-26, Salicylic Acid, Silica, Stearalkonium Hectorite, Triethanolamine, Aqua

### Variante w)

Aqua, Glycerin, Kaolin, Bentonite, Sodium Methyl Cocoyl Taurate, CI 77891, Trideceth-9, Salicylic Acid, C12-15 Alkyl Lactate, Sodium Chloride, Menthol, PEG-5 Ethylhexanoate, Xanthan Gum, Cetyl Lactate, Cocamidopropyl PG-dimonium Chloride Phosphate, Coconut Acid (Coconut Derived), Citric Acid, Sodium Citrate, Lactic Acid, Disodium EDTA, Methylparaben, Chlorphenesin, Propylparaben, Benzalkonium Chloride, Ethylparaben, Parfum (Perfuming)

### Variante x)

Aqua, Cocamidopropyl Betaine, Sodium Myreth Sulfate, Acrylates Copolymer, Lactic Acid, Maris Limus Extract, ostera, Lauryl Glucoside, PEG-40 Hydrogenated Castor Oil (Hydrogenated), PEG-200 Hydrogenated Glyceryl Palmate (Hydrogenated), Polyethylene, Mannitol, Trisodium EDTA, Polyquaternium-10, Benzophenone-4, Microcrystalline Cellulose, Propylene Glycol, Phenoxyethanol, Methylparaben, Propylparaben, Alpha-isomethyl Ionone, Citronellol, Hexyl Cinnamal, Benzyl Salicylate, Butylphenyl Methylpropional, Parfum (Perfuming), CI 42090, CI 74160

### Variante y)

Aqua, Sodium Laureth Sulfate, PEG-8, Coco-betaine, Hexylene Glycol, Sodium Chloride, PEG-120 Methyl Glucose Dioleate, Zinc PCA, Sodium Hydroxide, Citric Acid, Sodium Benzoate, Phenoxyethanol, Caprylyl Glycol, Parfum (Perfuming), F.I.L. B32026/1

### Variante aa)

Aqua, Kaolin, Glycerin, Butylene Glycol, Zea Mays Starch, CI 77891, Decyl Glucoside, Polyethylene, Sodium Laureth Sulfate, Chondrus Crispus, PEG-7 Glyceryl Cocoate, Salicylic Acid, Eucalyptus Globulus Leaf Extract (Extract), Menthol, Zinc Gluconate, Jojoba Esters, Propylene Glycol, Triethanolamine, Xanthan Gum, Tetrasodium EDTA, Methylparaben, Phenoxyethanol, CI 7707

### Variante ab)

Aqua, Alcohol Denat., Sorbitol, Cocamidopropyl Betaine, Parfum (Perfuming), Allantoin, Sodium Chloride, Propylene Glycol, Laminaria Digitata Extract (Extract)

### Variante ac)

Aqua, Sodium Myreth Sulfate, Disodium Laureth Sulfosuccinate, Cocamidopropyl Betaine, PEG-150 Distearate, Coco-glucoside, Glyceryl Oleate, Hexylene Glycol, Sorbitol, Cymbopogon Schoenanthus Extract (Extract), 1,10-decanediol, 10-hydroxydecanoic Acid, Sebacic Acid, Parfum (Perfuming), PEG-7 Glyceryl Cocoate, Tocopherol, Hydrogenated Palm Glycerides Citrate (Hydrogenated), Propylene Glycol, Butylene Glycol, Citric Acid, Sodium Chloride, Sodium Salicylate, Sodium Benzoate

### Variante ad)

Aqua, PPG-15 Stearyl Ether, Glycerin, Stearyl Alcohol, Cetyl Betaine, Salicylic Acid, Distearyldimonium Chloride, Polyethylene, Sodium Lauryl Sulfate, Cetyl Alcohol, Alcohol, Steareth-21, Sodium Chloride, Sodium Hydroxide, Synthetic Wax (Artificial), Behenyl Alcohol, PPG-30, Steareth-2, Parfum (Perfuming), Dipropylene Glycol, Mica, Benzyl Salicylate, Limonene, Disodium EDTA, BHT, CI 77891, CI 77510

### Variante ae)

Aqua, Alcohol Denat., Glucosamine, Sorbitol, Sea Whip Extract (Extract), CI 77120, 10-hydroxydecanoic Acid, silica disodium EDTA, benzalkonium chloride (ILN32341) nylon-12, Salicylic Acid, Butylene Glycol, Hamamelis Virginiana Extract, Laminaria Saccharina Extract (Extract), Caffeine, Sucrose, Glycerin, acetyl

### Variante af)

Aqua, Glycerin, Butylene Glycol, Sodium Methyl Cocoyl Taurate, Sucrose, Salicylic Acid, Disodium Phosphate, Arginine Cocoate, Laminaria Saccharina Extract (Extract), Cola Nitida Seed Extract (Extract, Seed), Caffeine, Algae Extract (Extract), Mentha Piperita Leaves, Sea Whip Extract (Extract), PEG/PPG-18/18 Dimethicone, Sodium Hyaluronate, PPG-6-decyltetradeceth-30, Lactobacillus Ferment, Stearamidopropyl Dimethylamine, Longifolene, Acetyl Glucosamine, Capryloyl Glycine, perilla aldehyde, 10-hydroxydecanoic Acid, Polyquaternium-7, beta-caryophylene, Phospholipids, Sodium Salicylate, Sodium Stearate, Disodium EDTA, Phenoxyethanol, chloroxylenol (ILN 32338)

### Variante ag)

Benzoyl Peroxide 2.5%. Inactive: Water, cyclopentasiloxane, butylene glycol, ceteareth-20, dimenthicone, sucrose, green tea leaf extract, barley extract, acetyl glucosamine, lactobacillus ferment, poria cocos sclerotium extract, laminaria saccharina extract, polymethyl methacrylate, gentian root extract, sunflower seedcake, saccharomyces lysate extract, astrocaryum murumuru butter, acrylamide/sodium acryloyldimethyltaurate copolymermyristyl alcohol, glycerin

### Variante ae)

Benzoyl Peroxide, inactive (Glycerin), Petrolatum, Paraffinum Liquidum, C12-15 Alkyl Benzoate, Aqua, Sodium Cocoyl Isethionate, Sodium C14-16 Olefin Sulfonate, Zinc Lactate, Acrylates/C 10-30 Alkyl Acrylate Crosspolymer, Menthol, Parfum (Perfuming), potassium polymetaphosphate, CI 77891, Carbomer

### Variante af)

Aqua, Sodium C14-16 Olefin Sulfonate, Cocamidopropyl Betaine, Linoleamidopropyl PG-dimonium Chloride Phosphate, Polysorbate 20, Anthemis Nobilis Flower Extract (Extract), Citrus Grandis Fruit Extract (Extract), Aloe Barbadensis Flower Extract (Extract), Chamomilla Recutita Flower Extract (Extract), C12-15 Alkyl Lactate, cocamidopropyl, PG-dimonium chloride phosphate, Polyquaternium-7, Ascorbyl Palmitate, Propylene Glycol, Sodium Benzotriazolyl Butylphenol Sulfonate, PEG-120 methyl glucose dioleate PEG-80 sorbitan laurate, Disodium EDTA, Sodium Chloride, Benzalkonium Chloride, CI 16035, CI 60725, fragrance (963-277)

### Variante ag)

Salicylic Acid, (Aqua), Cyclopentasiloxane, PEG-8, Dimethicone, Dimethicone Crosspolymer, Sodium Polyacrylate, Vinyl Dimethicone/methicone Silsesquioxane Crosspolymer, cocamidopropyl PG dimonium chloride phosphate, Cucumis Sativus Fruit Extract (Extract), Camellia Sinensis Leaf Extract (Extract), Glycerin, Panthenol, Butylene Glycol, C12-15 Alkyl Lactate, Cetyl Alcohol, Glyceryl Stearate, Cetyl Lactate, PEG 75 stearate, Ceteth-20, Steareth-20, Trideceth-6, Cyclopentasiloxane, PEG/PPG-18/18 Dimethicone, Sclerotium Gum, Laureth-23, Laureth-4, Disodium EDTA, Benzalkonium Chloride, Potassium Hydroxide, CI 61570, CI 19140, CI 42090, Parfum (Perfuming)

### Variante ah)

Benzoyl Peroxide, inactive, Aqua, Bentonite, Caprylic/Capric Triglyceride, Glycerin, Emulsifying Wax (National Formulary, Emulsifying), Polysorbate 20, Glyceryl Laurate, Cetyl Dimethicone, Magnesium Aluminum Silicate, Xanthan Gum, Sodium Citrate, Disodium EDTA, Citric Acid, Methylparaben, Propylparaben

### Variante ai)

Polyquaternium-37, Silica, Aqua, Glycerin, Polysilicone-13, PEG-12 Dimethicone, Hamamelis Virginiana Extract (Extract), CI 77891, Stearyl Glycyrrhetinate, Butylene Glycol, Methylparaben

### Variante aj)

Salicylic Acid, Aqua, Sodium Cocoyl Isethionate, Cetearyl Alcohol, Laureth-3, Glycerin, Coconut Acid (Coconut Derived), Sodium Isethionate, Sodium Hydroxide, Parfum (Perfuming), Lavandula Stoechas Extract (Extract), Helichrysum Italicum Extract (Extract), Cistus Monspeliensis Extract (Extract), Disodium EDTA, PEG-40 Hydrogenated Castor Oil (Hydrogenated), Phenoxyethanol, Methylparaben, Butylparaben, Ethylparaben, Isobutylparaben, Propylparaben

### Variante ak)

Aqua, Ethylhexyl Methoxycinnamate, Ethylhexyl Salicylate, Benzophenone-3, Butyl Methoxydibenzoylmethane, Propylene Glycol, C12-15 Alkyl Benzoate, Dimethicone, Glyceryl Stearate, Thiodipropionic Acid, Retinyl Palmitate, Triticum Vulgare Germ Extract (Extract, Germs), Saccharomyces/zinc Ferment, Glycolic Acid, Salicylic Acid, Pyridoxine HCl, Tocopherol, Palmitoyl Oligopeptide, Palmitoyl Tetrapeptide-7, Glycerin, Aluminum Starch Octenylsuccinate, Hydroxyethyl Acrylate/Sodium Acryloyldimethyl Taurate Copolymer, Silica, Isohexadecane, Acrylates Crosspolymer, PEG-100 Stearate, Caprylyl Glycol, Hydrogenated Polyisobutene (Hydrogenated), Hydroxyethylcellulose, Xanthan Gum, Choleth-24, Polysorbate 60, Ceteth-24, Isododecane, Sorbitan Isostearate, Polystyrene/hydrogenated Polyisopentene Copolymer (Hydrogenated), Butylene Glycol, Carbomer, Polysorbate 20, Steareth-2, Ammonium Hydroxide, Cetyl Alcohol, Stearic Acid, Disodium EDTA, Phenoxyethanol, Parfum (Perfuming).

## Patentansprüche

1. Nicht-therapeutische Verwendung mindestens einer Verbindung der Formel I 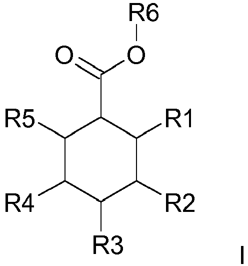 worin R1, R2, R3, R4 und R5 unabhängig voneinander stehen für einen Rest ausgewählt aus
- H, OH, OCOCH₃, O-(CH₂-CH₂-O)ₙ-CH₂-CH₂-OH, mit n=0 bis 20,
- geradkettige oder verzweigte Alkyl- oder Alkoxygruppe mit 1 bis 20 C-Atomen,
wobei mindestens einer der Reste R1, R2, R3, R4 und R5 für OH, OCOCH₃ oder O-(CH₂-CH₂-O)ₙ-CH₂-CH₂-OH, mit n=0 bis 20, steht,
und worin R6 steht für einen Rest ausgewählt aus
- H,
- (CH₂-CH₂-O)ₙ-CH₂-CH₂-OH, mit n= 0 bis 20,
- geradkettige oder verzweigten Alkylgruppe mit 1 bis 20 C-Atomen,
- geradkettige oder verzweigte Alkenyl- oder Alkinylgruppe mit 2 bis 20 C-Atomen und einer oder mehreren Doppel- bzw. Dreifachbindungen,
wobei die Alkyl-, Alkenyl- oder Alkinylgruppe auch eine oder mehrere gesättigte oder ungesättigte C₃- bis C₁₂-Cycloalkylgruppen enthalten kann,
als antimikrobieller Wirkstoff in Lebensmitteln, Haushaltsprodukten, Kunststoffen, Papier und/ oder Farben.

2. Nicht-therapeutische Verwendung mindestens einer Verbindung der Formel I 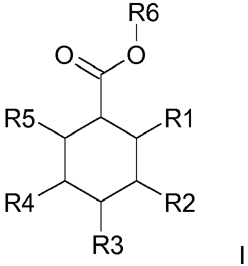 worin R1, R2, R3, R4 und R5 unabhängig voneinander stehen für einen Rest ausgewählt aus
- H, OH, OCOCH₃, O-(CH₂-CH₂-O)ₙ-CH₂-CH₂-OH, mit n=0 bis 20,
- geradkettige oder verzweigte Alkyl- oder Alkoxygruppe mit 1 bis 20 C-Atomen,
wobei mindestens einer der Reste R1, R2, R3, R4 und R5 für OH, OCOCH₃ oder O-(CH₂-CH₂-O)ₙ-CH₂-CH₂-OH, mit n=0 bis 20, steht, und worin R6 steht für einen Rest ausgewählt aus
- H,
- (CH₂-CH₂-O)ₙ-CH₂-CH₂-OH, mit n= 0 bis 20,
- geradkettige oder verzweigten Alkylgruppe mit 1 bis 20 C-Atomen,
- geradkettige oder verzweigte Alkenyl- oder Alkinylgruppe mit 2 bis 20 C-Atomen und einer oder mehreren Doppel- bzw. Dreifachbindungen,
wobei die Alkyl-, Alkenyl- oder Alkinylgruppe auch eine oder mehrere gesättigte oder ungesättigte C₃- bis C₁₂-Cycloalkylgruppen enthalten kann,
als antimikrobieller Wirkstoff in kosmetischen/ pharmazeutischen Formulierungen, Medizinprodukten, Lebensmitteln,Haushaltsprodukten, Kunststoffen, Papier und/ oder Farben zur Konservierungsverbesserung.

3. Nicht-therapeutische Verwendung mindestens einer Verbindung der
Formel I 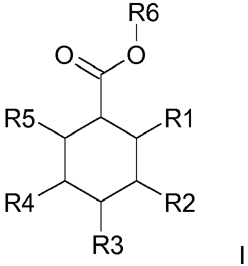 worin R1, R2, R3, R4 und R5 unabhängig voneinander stehen für einen Rest ausgewählt aus
- H, OH, OCOCH₃, O-(CH₂-CH₂-O)ₙ-CH₂-CH₂-OH, mit n=0 bis 20,
- geradkettige oder verzweigte Alkyl- oder Alkoxygruppe mit 1 bis 20 C-Atomen,
wobei mindestens einer der Reste R1, R2, R3, R4 und R5 für OH, OCOCH₃ oder O-(CH₂-CH₂-O)ₙ-CH₂-CH₂-OH, mit n=0 bis 20, steht, und worin R6 steht für einen Rest ausgewählt aus
- H,
- (CH₂-CH₂-O)ₙ-CH₂-CH₂-OH, mit n= 0 bis 20,
- geradkettige oder verzweigten Alkylgruppe mit 1 bis 20 C-Atomen,
- geradkettige oder verzweigte Alkenyl- oder Alkinylgruppe mit 2 bis 20 C-Atomen und einer oder mehreren Doppel- bzw. Dreifachbindungen,
wobei die Alkyl-, Alkenyl- oder Alkinylgruppe auch eine oder mehrere gesättigte oder ungesättigte C₃- bis C₁₂-Cycloalkylgruppen enthalten kann,
als Antitranspirant- oder desodorierender Wirkstoff.

4. Verwendung nach einem oder mehreren der Ansprüche 1 bis 3, **dadurch gekennzeichnet, dass** die Reste R1, R2, R3, R4 und R5 unabhängig voneinander für H oder OH stehen, wobei 1, 2 oder 3 der Reste R1, R2, R3, R4 und R5 für OH stehen.

5. Verwendung nach Anspruch 4, **dadurch gekennzeichnet, dass** die Verbindung der Formel I ausgewählt ist aus den Verbindungen der Formel Ia und Ib, 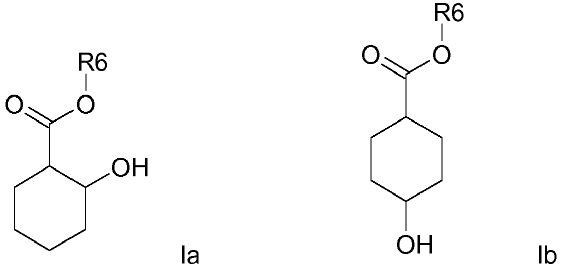 worin R6 steht für einen Rest ausgewählt aus
- H,
- (CH₂-CH₂-O)ₙ-CH₂-CH₂-OH, mit n= 0 bis 20,
- geradkettige oder verzweigten Alkylgruppe mit 1 bis 20 C-Atomen,
- geradkettige oder verzweigte Alkenyl- oder Alkinylgruppe mit 2 bis 20 C-Atomen und einer oder mehreren Doppel- bzw. Dreifachbindungen,
wobei die Alkyl-, Alkenyl- oder Alkinylgruppe auch eine oder mehrere gesättigte oder ungesättigte C₃- bis C₁₂-Cycloalkylgruppen enthalten kann.

6. Verwendung nach einem oder mehreren der Ansprüche 1 bis 5,
**dadurch gekennzeichnet, dass** der Rest R6 für einen Rest steht ausgewählt aus
- H,
- CH₂-CH₂-OH,
- geradkettige oder verzweigten Alkylgruppe mit 1 bis 10 C-Atomen, welche auch eine gesättigte oder ungesättigte C₆-Cycloalkylgruppe enthalten kann.

7. Verwendung nach Anspruch 6, **dadurch gekennzeichnet, dass** eine Verbindung der Formel I verwendet wird, ausgewählt aus den Verbindungen der Formel I-1 bis I-28
| | | | |
|---|---|---|---|
| I-1 | 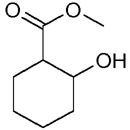 | I-2 | 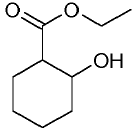 |
| I-3 | 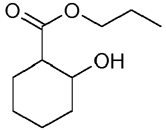 | I-4 | 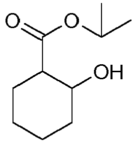 |
| I-5 | 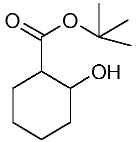 | I-6 | 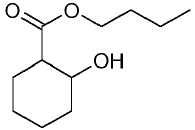 |
| I-7 | 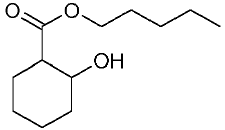 | I-8 | 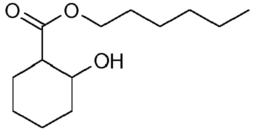 |
| I-9 | 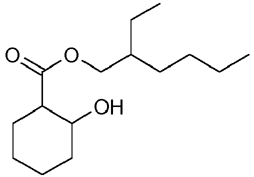 | I-10 | 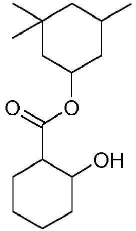 |
| I-11 | 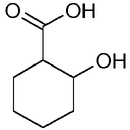 | I-12 | 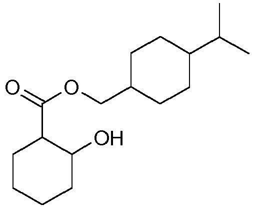 |
| I-13 | 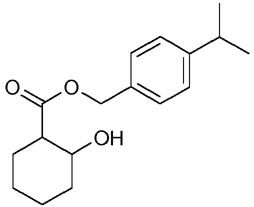 | I-14 | 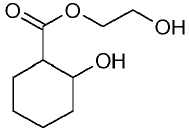 |
| I-15 | 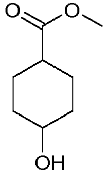 | I-16 | 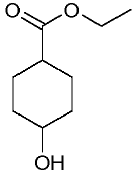 |
| I-17 | 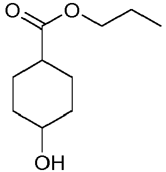 | I-18 | 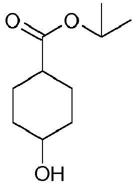 |
| I-19 | 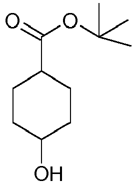 | I-20 | 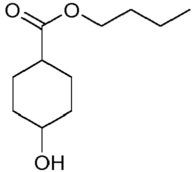 |
| I-21 | 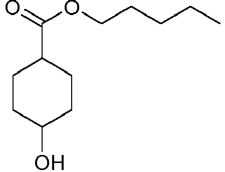 | I-22 | 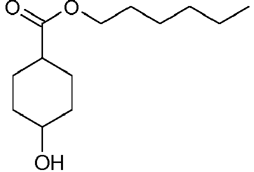 |
| I-23 | 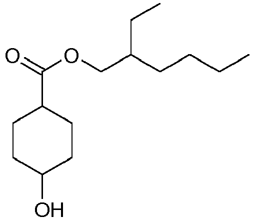 | I-24 | 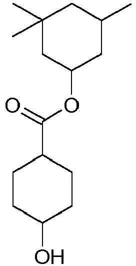 |
| I-25 | 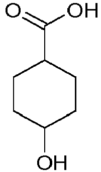 | I-26 | 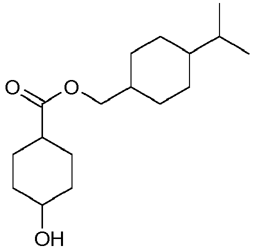 |
| I-27 | 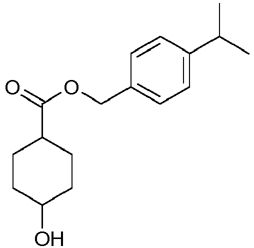 | I-28 | 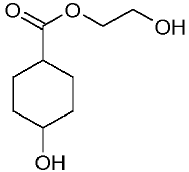 |

8. Kosmetische oder pharmazeutische Zubereitung enthaltend mindestens eine Verbindung ausgewählt aus der Gruppe der Verbindungen der Formel I-3 bis I-5, I-7 bis I-10 und I-12 bis 1-28
| | | | |
|---|---|---|---|
| I-3 | 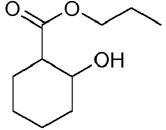 | I-4 | 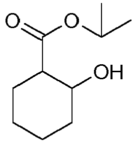 |
| I-5 | 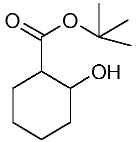 | | |
| I-7 | 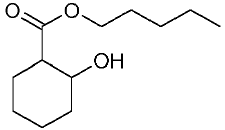 | I-8 | 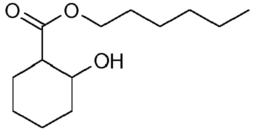 |
| I-9 | 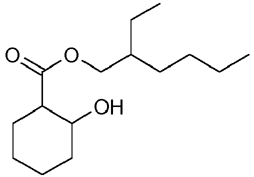 | I-10 | 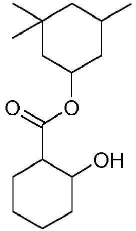 |
| | | I-12 | 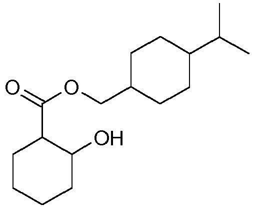 |
| I-13 | 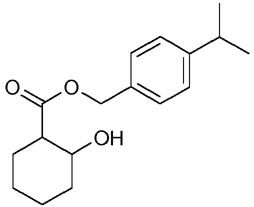 | I-14 | 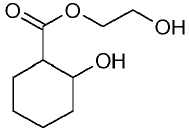 |
| I-15 | 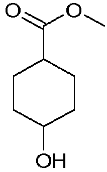 | I-16 | 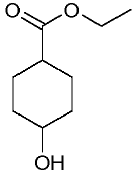 |
| I-17 | 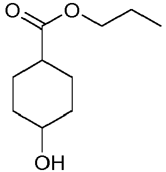 | I-18 | 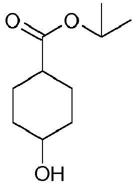 |
| I-19 | 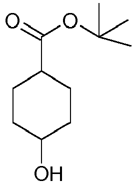 | I-20 | 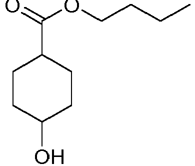 |
| I-21 | 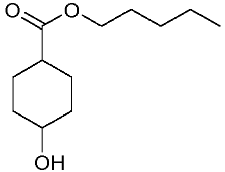 | I-22 | 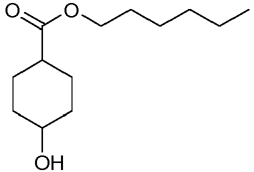 |
| I-23 | 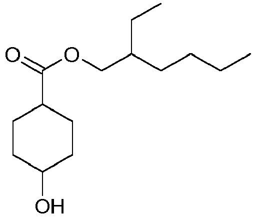 | I-24 | 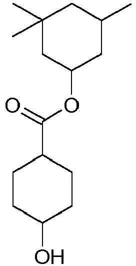 |
| I-25 | 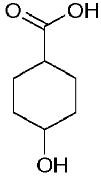 | I-26 | 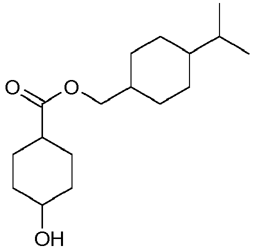 |
| I-27 | 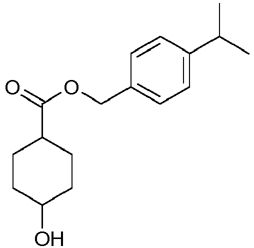 | I-28 | 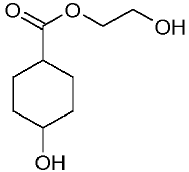 |
und mindestens einen geeigneten Träger.

9. Zubereitung nach Anspruch 8, **dadurch gekennzeichnet, dass** die Verbindung ausgewählt aus der Gruppe der Verbindungen der Formel I-3 bis I-5, I-7 bis I-10 und I-12 bis 1-28 in einer Menge von 0,01 bis 20 Gew.-%, bezogen auf die Gesamtmenge der Zubereitung, enthalten ist.

10. Zubereitung nach Anspruch 8 oder 9, **dadurch gekennzeichnet, dass** die Zubereitung ein Deodorant, ein Antitranspirant, ein Anti-Schuppen- oder Anti-Akne-Mittel oder eine antibakterielle Zubereitung, insbesondere zur Zahn- oder Mundpflege, ist.

11. Verfahren zur Herstellung einer Zubereitung nach einem oder mehreren der Ansprüche 8 bis 10, **dadurch gekennzeichnet, dass** die Verbindung ausgewählt aus der Gruppe der Verbindungen der Formel I-3 bis I-5, I-7 bis I-10 und I-12 bis 1-28 mit einem geeigneten Träger vermischt wird.

12. Verbindung der Formel Ia oder Ib, 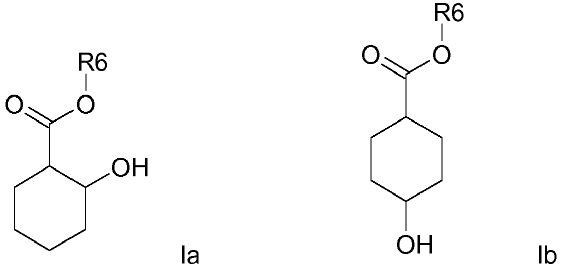 worin R6 steht für einen Rest ausgewählt aus
- H,
- (CH₂-CH₂-O)ₙ-CH₂-CH₂-OH, mit n= 0 bis 20,
- geradkettige oder verzweigten Alkylgruppe mit 1 bis 20 C-Atomen,
- geradkettige oder verzweigte Alkenyl- oder Alkinylgruppe mit 2 bis 20 C-Atomen und einer oder mehreren Doppel- bzw. Dreifachbindungen,
wobei die Alkyl-, Alkenyl- oder Alkinylgruppe auch eine oder mehrere gesättigte oder ungesättigte C₃- bis C₁₂-Cycloalkylgruppen enthalten kann,
wobei Verbindungen der Formel Ia ausgeschlossen sind, in denen R6 für H (Verbindung I-11), Methyl (Verbindung I-1), Ethyl (Verbindung I-2), Isopropyl (Verbindung I-4), tert-Butyl (Verbindung I-5), n-Butyl (Verbindung I-6), n-Hexyl (Verbindung I-8), CH₂CH(CH₂CH₃)₂, CH₂CH₂CH(CH₃)₂, C(=CH₂)CH₃ oder Cyclopentyl steht,
und wobei Verbindungen der Formel Ib ausgeschlossen sind, in denen R6 für H (Verbindung I-25), Methyl (Verbindung I-15), Ethyl (Verbindung I-16), Propyl (Verbindung I-17), Isopropyl (Verbindung I-18) oder tert-Butyl (Verbindung I-19) steht.

13. Verbindung nach Anspruch 12, **dadurch gekennzeichnet, dass** die Verbindung ausgewählt ist aus den nachstehenden Verbindungen:
| | | | |
|---|---|---|---|
| I-3 | 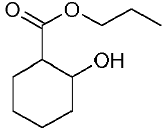 | | |
| I-7 | 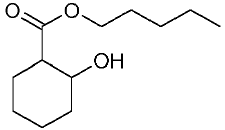 | I-9 | 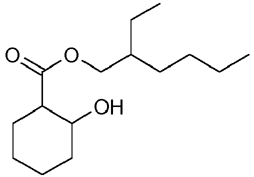 |
| I-10 | 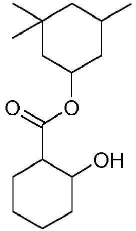 | I-12 | 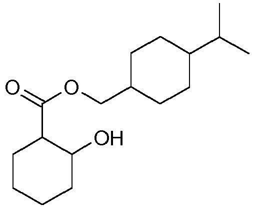 |
| I-13 | 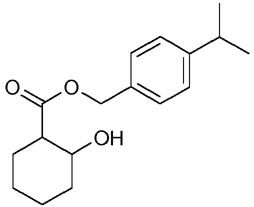 | I-14 | 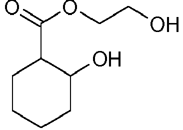 |
| I-20 | 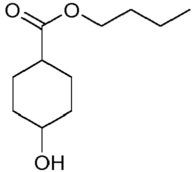 | I-21 | 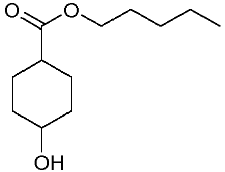 |
| I-22 | 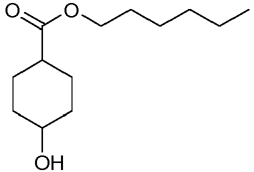 | I-23 | 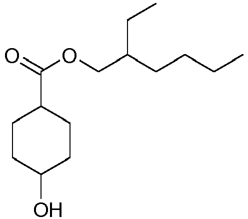 |
| I-24 | 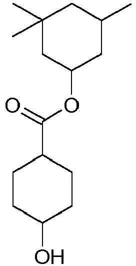 | I-26 | 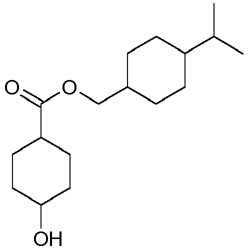 |
| I-27 | 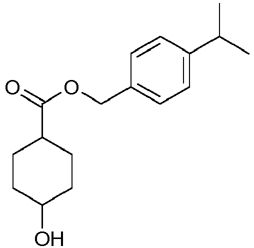 | I-28 | 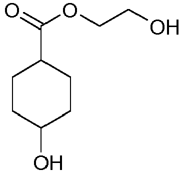 |

14. Verfahren zur Herstellung einer Verbindung der Formel Ia oder Ib, nach Anspruch 12 oder 13, **dadurch gekennzeichnet, dass** eine Verbindung der Formel Ia-x oder Ib-x 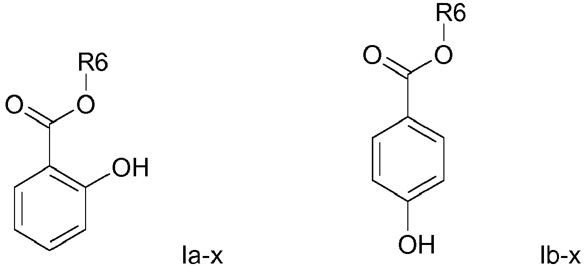 mittels Hydrierung zum entsprechenden hydrierten Produkt der Formel Ia oder Ibumgesetzt wird.

15. Verbindung der Formel I 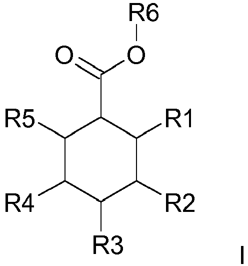 worin R1, R2, R3, R4 und R5 unabhängig voneinander stehen für einen Rest ausgewählt aus
- H, OH, OCOCH₃, O-(CH₂-CH₂-O)ₙ-CH₂-CH₂-OH, mit n=0 bis 20,
- geradkettige oder verzweigte Alkyl- oder Alkoxygruppe mit 1 bis 20 C-Atomen,
wobei mindestens einer der Reste R1, R2, R3, R4 und R5 für OH, OCOCH₃ oder O-(CH₂-CH₂-O)ₙ-CH₂-CH₂-OH, mit n=0 bis 20, steht, und worin R6 steht für einen Rest ausgewählt aus
- H,
- (CH₂-CH₂-O)ₙ-CH₂-CH₂-OH, mit n= 0 bis 20,
- geradkettige oder verzweigten Alkylgruppe mit 1 bis 20 C-Atomen,
- geradkettige oder verzweigte Alkenyl- oder Alkinylgruppe mit 2 bis 20 C-Atomen und einer oder mehreren Doppel- bzw. Dreifachbindungen,
wobei die Alkyl-, Alkenyl- oder Alkinylgruppe auch eine oder mehrere gesättigte oder ungesättigte C₃- bis C₁₂-Cycloalkylgruppen enthalten kann,
zur therapeutischen Verwendung als antimikrobieller Wirkstoff.

16. Verbindung nach Anspruch 15 zur Verwendung in Zahn- oder Mundpflegeprodukten.

17. Verbindung der Formel I 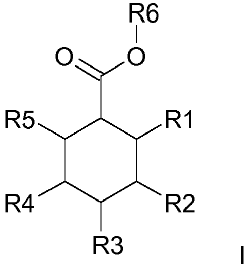 worin R1, R2, R3, R4 und R5 unabhängig voneinander stehen für einen Rest ausgewählt aus
- H, OH, OCOCH₃, O-(CH₂-CH₂-O)ₙ-CH₂-CH₂-OH, mit n=0 bis 20,
- geradkettige oder verzweigte Alkyl- oder Alkoxygruppe mit 1 bis 20 C-Atomen,
wobei mindestens einer der Reste R1, R2, R3, R4 und R5 für OH, OCOCH₃ oder O-(CH₂-CH₂-O)ₙ-CH₂-CH₂-OH, mit n=0 bis 20, steht,
und worin R6 teht für einen Rest ausgewählt aus
- H,
- (CH₂-CH₂-O)ₙ-CH₂-CH₂-OH, mit n= 0 bis 20,
- geradkettige oder verzweigten Alkylgruppe mit 1 bis 20 C-Atomen,
- geradkettige oder verzweigte Alkenyl- oder Alkinylgruppe mit 2 bis 20 C-Atomen und einer oder mehreren Doppel- bzw. Dreifachbindungen,
wobei die Alkyl-, Alkenyl- oder Alkinylgruppe auch eine oder mehrere gesättigte oder ungesättigte C₃- bis C₁₂-Cycloalkylgruppen enthalten kann,
zur Verwendung als antimikrobieller Anti-Akne-, oder Antischuppen Wirkstoff.

18. Verbindung nach einem oder mehreren der Ansprüche 15 bis 17, **dadurch gekennzeichnet, dass** die Reste R1, R2, R3, R4 und R5 unabhängig voneinander für H oder OH stehen, wobei 1, 2 oder 3 der Reste R1, R2, R3, R4 und R5 für OH stehen.

19. Verbindung nach einem oder mehreren der Ansprüche 15 bis 18, **dadurch gekennzeichnet, dass** der Rest R6 für einen Rest steht ausgewählt aus
- H,
- CH₂-CH₂-OH,
- geradkettige oder verzweigten Alkylgruppe mit 1 bis 10 C-Atomen, welche auch eine gesättigte oder ungesättigte C₆-Cycloalkylgruppe enthalten kann.

20. Verbindung nach Anspruch 19, **dadurch gekennzeichnet, dass** eine Verbindung der Formel I ausgewählt ist aus den Verbindungen der Formel I-1 bis I-28
| | | | |
|---|---|---|---|
| I-1 | 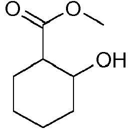 | I-2 | 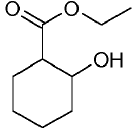 |
| I-3 | 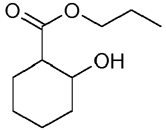 | I-4 | 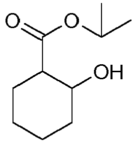 |
| I-5 | 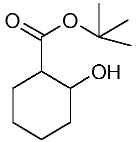 | I-6 | 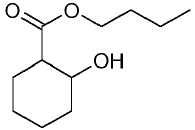 |
| I-7 | 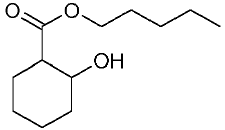 | I-8 | 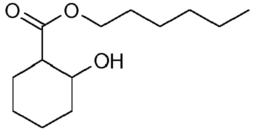 |
| I-9 | 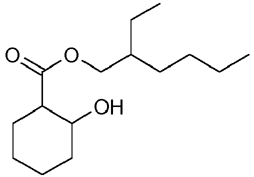 | I-10 | 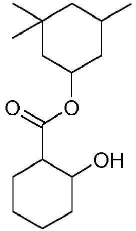 |
| I-11 | 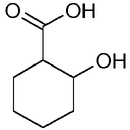 | I-12 | 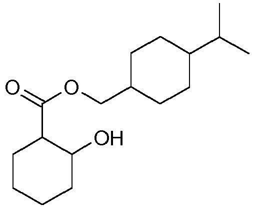 |
| I-13 | 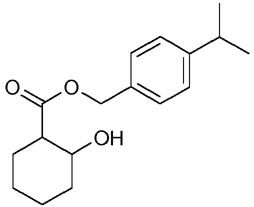 | I-14 | 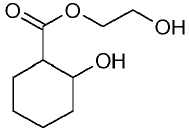 |
| I-15 | 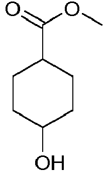 | I-16 | 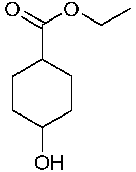 |
| I-17 | 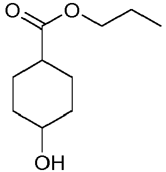 | I-18 | 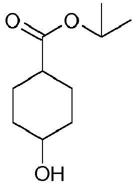 |
| I-19 | 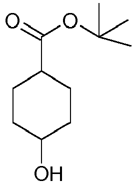 | I-20 | 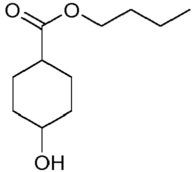 |
| I-21 | 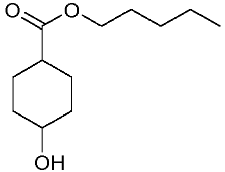 | I-22 | 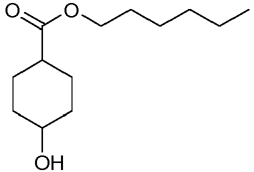 |
| I-23 | 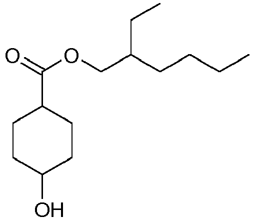 | I-24 | 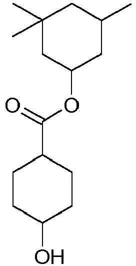 |
| I-25 | 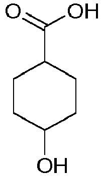 | I-26 | 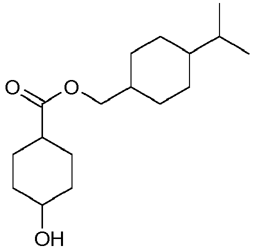 |
| I-27 | 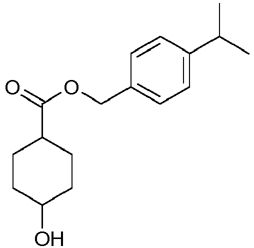 | I-28 | 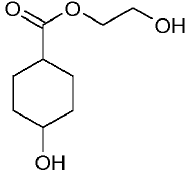 |

## Claims

1. Non-therapeutic use of at least one compound of the formula I 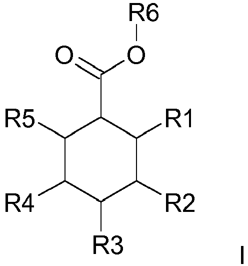 in which R1, R2, R3, R4 and R5 stand, independently of one another, for a radical selected from
- H, OH, OCOCH₃, O-(CH₂-CH₂-O)ₙ-CH₂-CH₂-OH, where n=0 to 20,
- straight-chain or branched alkyl or alkoxy group having 1 to 20 C atoms,
where at least one of the radicals R1, R2, R3, R4 and R5 stands for OH, OCOCH₃ or O-(CH₂-CH₂-O)ₙ-CH₂-CH₂-OH, where n=0 to 20,
and in which R6 stands for a radical selected from
- H,
- (CH₂-CH₂-O)ₙ-CH₂-CH₂-OH, where n= 0 to 20,
- straight-chain or branched alkyl group having 1 to 20 C atoms,
- straight-chain or branched alkenyl or alkynyl group having 2 to 20 C atoms and one or more double or triple bonds,
where the alkyl, alkenyl or alkynyl group may also contain one or more saturated or unsaturated C₃- to C₁₂-Cycloalkyl groups,
as antimicrobial active compound in foods, household products, plastics, paper and/or paints.

2. Non-therapeutic use of at least one compound of the formula I 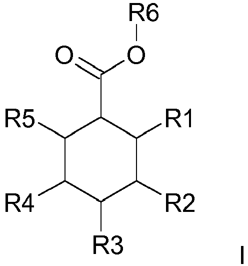 in which R1, R2, R3, R4 and R5 stand, independently of one another, for a radical selected from
- H, OH, OCOCH₃, O-(CH₂-CH₂-O)ₙ-CH₂-CH₂-OH, where n=0 to 20,
- straight-chain or branched alkyl or alkoxy group having 1 to 20 C atoms,
where at least one of the radicals R1, R2, R3, R4 and R5 stands for OH, OCOCH₃ or O-(CH₂-CH₂-O)ₙ-CH₂-CH₂-OH, where n=0 to 20,
and in which R6 stands for a radical selected from
- H,
- (CH₂-CH₂-O)ₙ-CH₂-CH₂-OH, where n= 0 to 20,
- straight-chain or branched alkyl group having 1 to 20 C atoms,
- straight-chain or branched alkenyl or alkynyl group having 2 to 20 C atoms and one or more double or triple bonds,
where the alkyl, alkenyl or alkynyl group may also contain one or more saturated or unsaturated C₃- to C₁₂-Cycloalkyl groups,
as antimicrobial active compound in cosmetic/pharmaceutical formulations, medicinal products, foods, household products, plastics, paper and/or paints for improving preservation.

3. Non-therapeutic use of at least one compound of the formula I 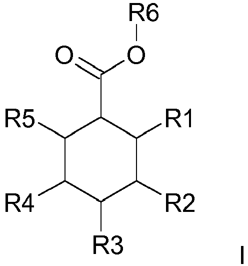 in which R1, R2, R3, R4 and R5 stand, independently of one another, for a radical selected from
- H, OH, OCOCH₃, O-(CH₂-CH₂-O)ₙ-CH₂-CH₂-OH, where n=0 to 20,
- straight-chain or branched alkyl or alkoxy group having 1 to 20 C atoms,
where at least one of the radicals R1, R2, R3, R4 and R5 stands for OH, OCOCH₃ or O-(CH₂-CH₂-O)ₙ-CH₂-CH₂-OH, where n=0 to 20,
and in which R6 stands for a radical selected from
- H,
- (CH₂-CH₂-O)ₙ-CH₂-CH₂-OH, where n= 0 to 20,
- straight-chain or branched alkyl group having 1 to 20 C atoms,
- straight-chain or branched alkenyl or alkynyl group having 2 to 20 C atoms and one or more double or triple bonds,
where the alkyl, alkenyl or alkynyl group may also contain one or more saturated or unsaturated C₃- to C₁₂-Cycloalkyl groups,
as antiperspirant or deodorising active compound.

4. Use according to one or more of Claims 1 to 3, **characterised in that** the radicals R1, R2, R3, R4 and R5 stand, independently of one another, for H or OH, where 1, 2 or 3 of the radicals R1, R2, R3, R4 and R5 stand for OH.

5. Use according to Claim 4, **characterised in that** the compound of the formula I is selected from the compounds of the formulae Ia and Ib, 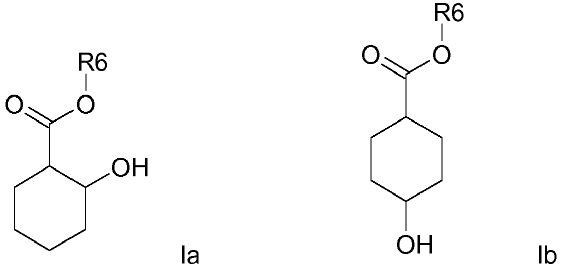 in which R6 stands for a radical selected from
- H,
- (CH₂-CH₂-O)ₙ-CH₂-CH₂-OH, where n= 0 to 20,
- straight-chain or branched alkyl group having 1 to 20 C atoms,
- straight-chain or branched alkenyl or alkynyl group having 2 to 20 C atoms and one or more double or triple bonds,
where the alkyl, alkenyl or alkynyl group may also contain one or more saturated or unsaturated C₃- to C₁₂-cycloalkyl groups.

6. Use according to one or more of Claims 1 to 5, **characterised in that** the radical R6 stands for a radical selected from
- H,
- CH₂-CH₂-OH,
- straight-chain or branched alkyl group having 1 to 10 C atoms, which may also contain a saturated or unsaturated C₆-cycloalkyl group.

7. Use according to Claim 6, **characterised in that** a compound of the formula I is used, selected from the compounds of the formulae I-1 to I-28
| | | | |
|---|---|---|---|
| I-1 | 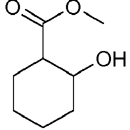 | I-2 | 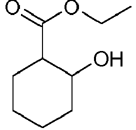 |
| I-3 | 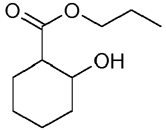 | I-4 | 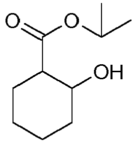 |
| I-5 | 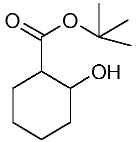 | I-6 | 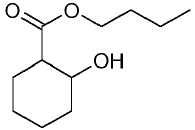 |
| I-7 | 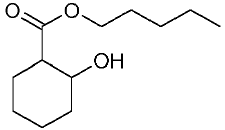 | I-8 | 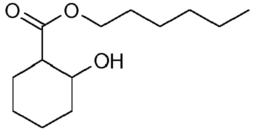 |
| I-9 | 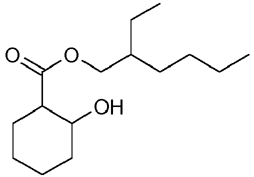 | I-10 | 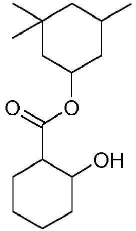 |
| I-11 | 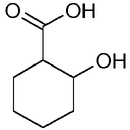 | I-12 | 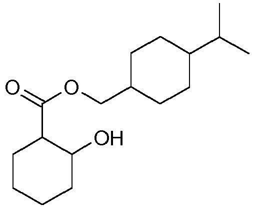 |
| I-13 | 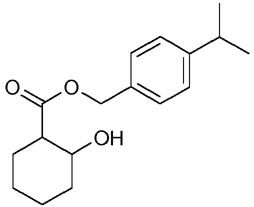 | I-14 | 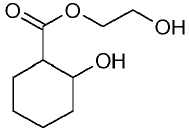 |
| I-15 | 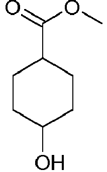 | I-16 | 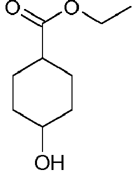 |
| I-17 | 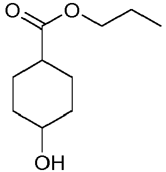 | I-18 | 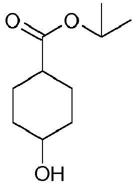 |
| I-19 | 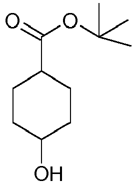 | I-20 | 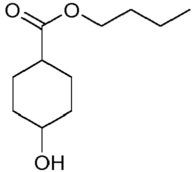 |
| I-21 | 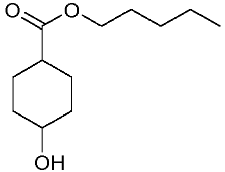 | I-22 | 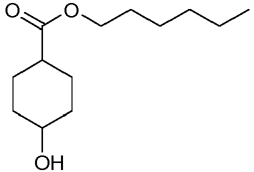 |
| I-23 | 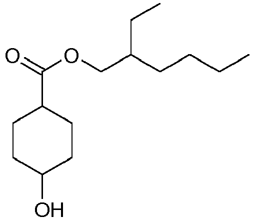 | I-24 | 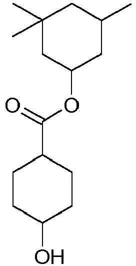 |
| I-25 | 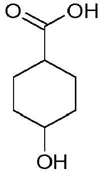 | I-26 | 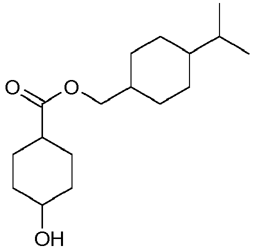 |
| I-27 | 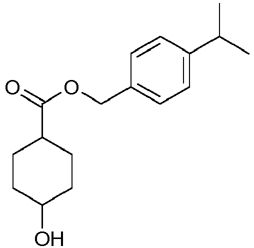 | I-28 | 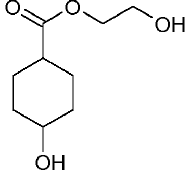 |

8. Cosmetic or pharmaceutical preparation comprising at least one compound selected from the group of the compounds of the formulae I-3 to I-5, I-7 to I-10 and I-12 to I-28
| | | | |
|---|---|---|---|
| I-3 | 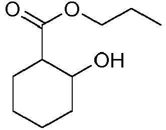 | I-4 | 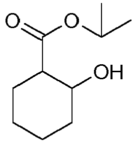 |
| I-5 | 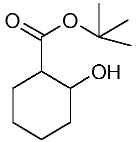 | | |
| I-7 | 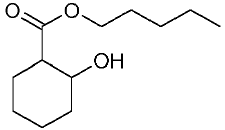 | I-8 | 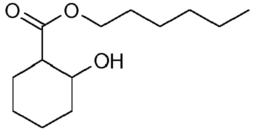 |
| I-9 | 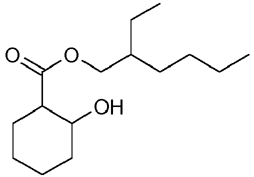 | I-10 | 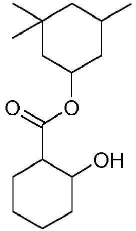 |
| | | I-12 | 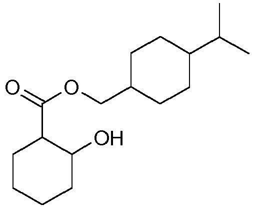 |
| I-13 | 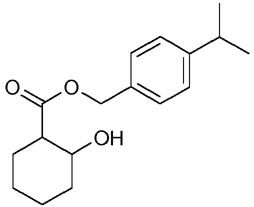 | I-14 | 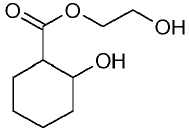 |
| I-15 | 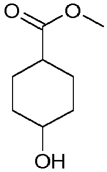 | I-16 | 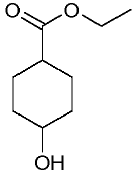 |
| I-17 | 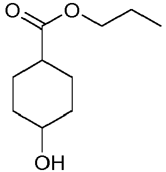 | I-18 | 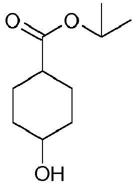 |
| I-19 | 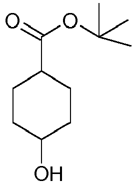 | I-20 | 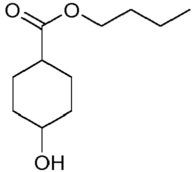 |
| I-21 | 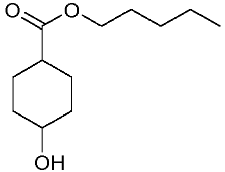 | I-22 | 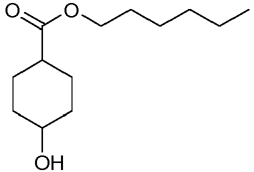 |
| I-23 | 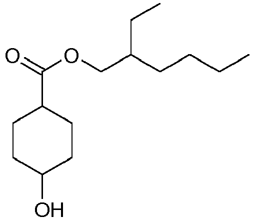 | I-24 | 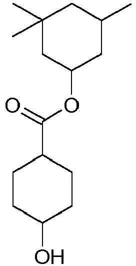 |
| I-25 | 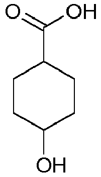 | I-26 | 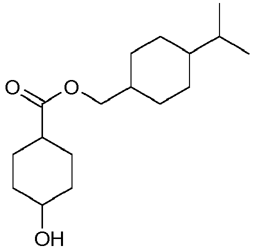 |
| I-27 | 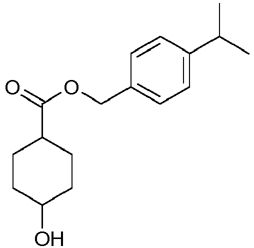 | I-28 | 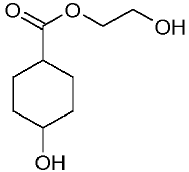 |
and at least one suitable vehicle.

9. Preparation according to Claim 8, **characterised in that** the compound selected from the group of the compounds of the formulae I-3 to I-5, I-7 to I-10 and I-12 to I-28 is present in an amount of 0.01 to 20% by weight, based on the total amount of the preparation.

10. Preparation according to Claim 8 or 9, **characterised in that** the preparation is a deodorant, an antiperspirant, an antidandruff or anti-acne composition or an antibacterial preparation, in particular for dental or oral care.

11. Process for the preparation of a preparation according to one or more of Claims 8 to 10, **characterised in that** the compound selected from the group of the compounds of the formulae I-3 to I-5, I-7 to I-10 and I-12 to I-28 is mixed with a suitable vehicle.

12. Compound of the formula Ia or Ib, 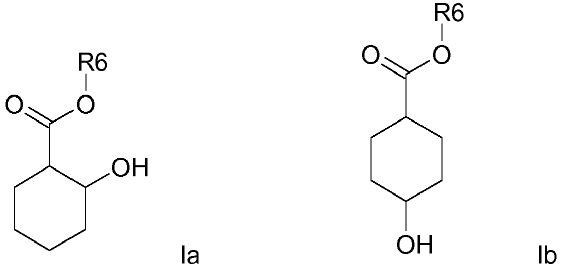 in which R6 stands for a radical selected from
- H,
- (CH₂-CH₂-O)ₙ-CH₂-CH₂-OH, where n= 0 to 20,
- straight-chain or branched alkyl group having 1 to 20 C atoms,
- straight-chain or branched alkenyl or alkynyl group having 2 to 20 C atoms and one or more double or triple bonds,
where the alkyl, alkenyl or alkynyl group may also contain one or more saturated or unsaturated C₃- to C₁₂-cycloalkyl groups,
where compounds of the formula Ia in which R6 stands for H (compound I-11), methyl (compound I-1), ethyl (compound I-2), isopropyl (compound I-4), tert-butyl (compound I-5), n-butyl (compound I-6), n-hexyl (compound I-8), CH₂CH(CH₂CH₃)₂, CH₂CH₂CH(CH₃)₂, C(=CH₂)CH₃ or cyclopentyl are excluded,
and where compounds of the formula Ib in which R6 stands for H (compound I-25), methyl (compound I-15), ethyl (compound I-16), propyl (compound I-17), isopropyl (compound I-18) or tert-butyl (compound I-19) are excluded.

13. Compound according to Claim 12, **characterised in that** the compound is selected from the following compounds:
| | | | |
|---|---|---|---|
| I-3 | 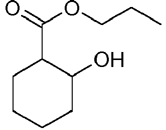 | | |
| I-7 | 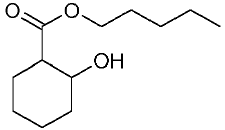 | I-9 | 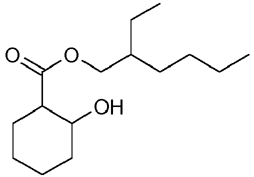 |
| I-10 | 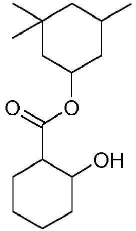 | I-12 | 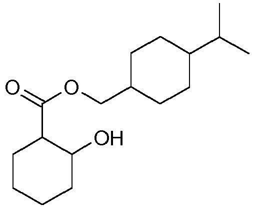 |
| I-13 | 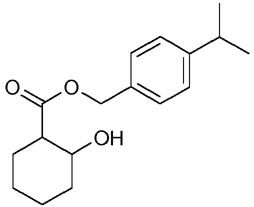 | I-14 | 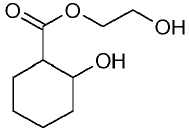 |
| I-20 | 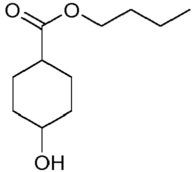 | I-21 | 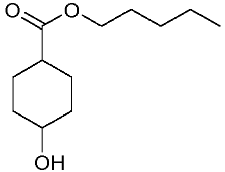 |
| I-22 | 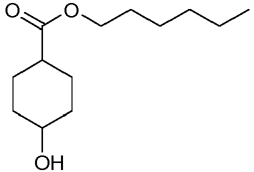 | I-23 | 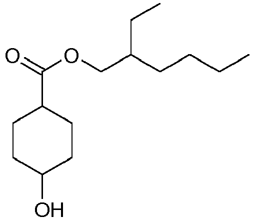 |
| I-24 | 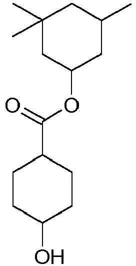 | I-26 | 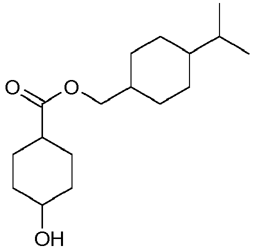 |
| I-27 | 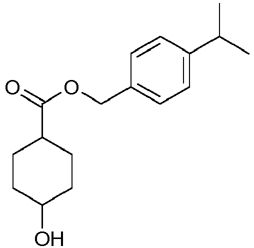 | I-28 | 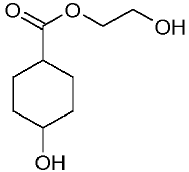 |

14. Process for the preparation of a compound of the formula Ia or Ib, according to Claim 12 or 13, **characterised in that** a compound of the formula la-x or Ib-x 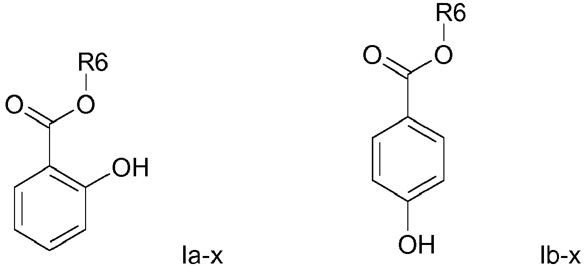 is converted into the corresponding hydrogenated product of the formula Ia or Ib by means of hydrogenation.

15. Compound of the formula I 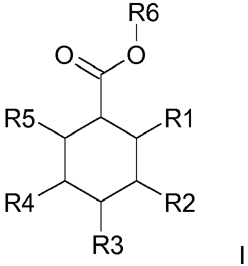 in which R1, R2, R3, R4 and R5 stand, independently of one another, for a radical selected from
- H, OH, OCOCH₃, O-(CH₂-CH₂-O)ₙ-CH₂-CH₂-OH, where n=0 to 20,
- straight-chain or branched alkyl or alkoxy group having 1 to 20 C atoms,
where at least one of the radicals R1, R2, R3, R4 and R5 stands for OH, OCOCH₃ or O-(CH₂-CH₂-O)ₙ-CH₂-CH₂-OH, where n=0 to 20,
and in which R6 stands for a radical selected from
- H,
- (CH₂-CH₂-O)ₙ-CH₂-CH₂-OH, where n= 0 to 20,
- straight-chain or branched alkyl group having 1 to 20 C atoms,
- straight-chain or branched alkenyl or alkynyl group having 2 to 20 C atoms and one or more double or triple bonds,
where the alkyl, alkenyl or alkynyl group may also contain one or more saturated or unsaturated C₃- to C₁₂-Cycloalkyl groups,
for therapeutic use as antimicrobial active compound.

16. Compound according to Claim 15 for use in dental or oral care products.

17. Compound of the formula I 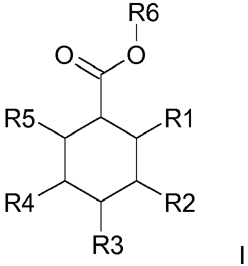 in which R1, R2, R3, R4 and R5 stand, independently of one another, for a radical selected from
- H, OH, OCOCH₃, O-(CH₂-CH₂-O)ₙ-CH₂-CH₂-OH, where n=0 to 20,
- straight-chain or branched alkyl or alkoxy group having 1 to 20 C atoms,
where at least one of the radicals R1, R2, R3, R4 and R5 stands for OH, OCOCH₃ or O-(CH₂-CH₂-O)ₙ-CH₂-CH₂-OH, where n=0 to 20,
and in which R6 stands for a radical selected from
- H,
- (CH₂-CH₂-O)ₙ-CH₂-CH₂-OH, where n= 0 to 20,
- straight-chain or branched alkyl group having 1 to 20 C atoms,
- straight-chain or branched alkenyl or alkynyl group having 2 to 20 C atoms and one or more double or triple bonds,
where the alkyl, alkenyl or alkynyl group may also contain one or more saturated or unsaturated C₃- to C₁₂-Cycloalkyl groups,
for use as antimicrobial anti-acne or antidandruff active compound.

18. Compound according to one or more of Claims 15 to 17, **characterised in that** the radicals R1, R2, R3, R4 and R5 stand, independently of one another, for H or OH, where 1, 2 or 3 of the radicals R1, R2, R3, R4 and R5 stand for OH.

19. Compound according to one or more of Claims 15 to 18, **characterised in that** the radical R6 stands for a radical selected from
- H,
- CH₂-CH₂-OH,
- straight-chain or branched alkyl group having 1 to 10 C atoms, which may also contain a saturated or unsaturated C₆-cycloalkyl group.

20. Compound according to Claim 19, **characterised in that** a compound of the formula I is selected from the compounds of the formulae I-1 to I-28
| | | | |
|---|---|---|---|
| I-1 | 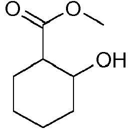 | I-2 | 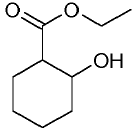 |
| I-3 | 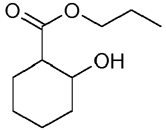 | I-4 | 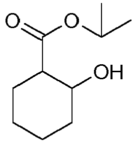 |
| I-5 | 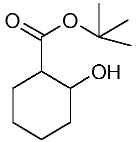 | I-6 | 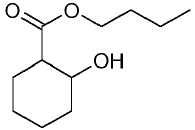 |
| I-7 | 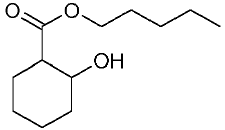 | I-8 | 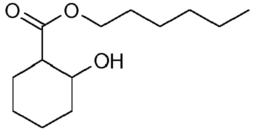 |
| I-9 | 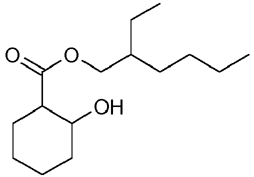 | I-10 | 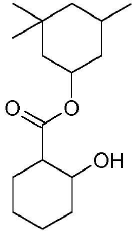 |
| I-11 | 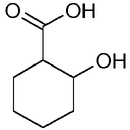 | I-12 | 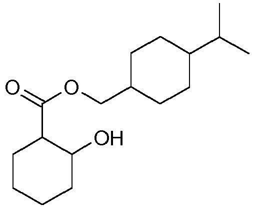 |
| I-13 | 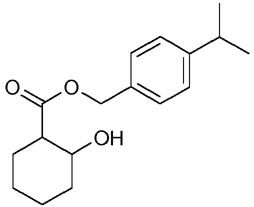 | I-14 | 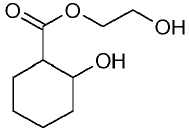 |
| I-15 | 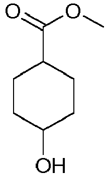 | I-16 | 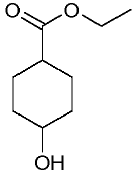 |
| I-17 | 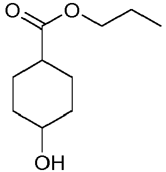 | I-18 | 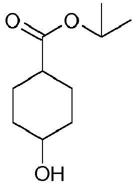 |
| I-19 | 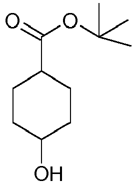 | I-20 | 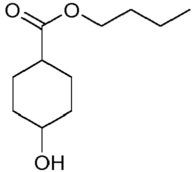 |
| I-21 | 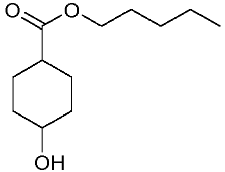 | I-22 | 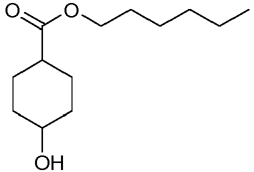 |
| I-23 | 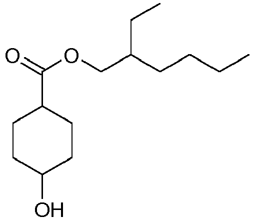 | I-24 | 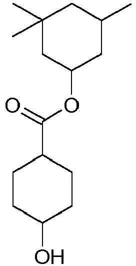 |
| I-25 | 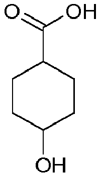 | I-26 | 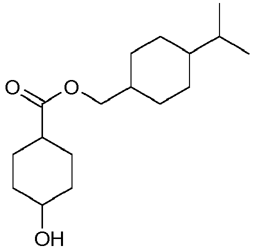 |
| I-27 | 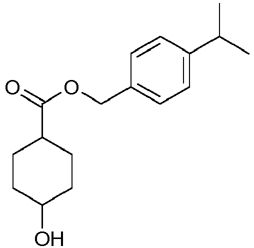 | I-28 | 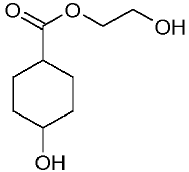 |

## Revendications

1. Utilisation non thérapeutique d'au moins un composé de formule I 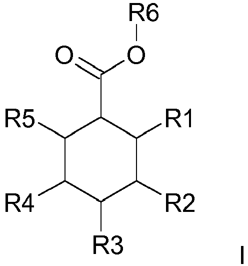 dans lequel R1, R2, R3, R4 et R5 représentent, indépendamment les uns des autres, un radical choisi parmi
- H, OH, OCOCH₃, O-(CH₂-CH₂-O)ₙ-CH₂-CH₂-OH, où n=0 à 20,
- un groupement alkyle ou alcoxy à chaîne linéaire ou ramifiée ayant de 1 à 20 atomes de C,
où au moins l'un des radicaux R1, R2, R3, R4 et R5 représente OH, OCOCH₃ ou O-(CH₂-CH₂-O)ₙ-CH₂-CH₂-OH, où n=0 à 20,
et où R6 représente un radical choisi parmi
- H,
- (CH₂-CH₂-O)ₙ-CH₂-CH₂-OH, où n= 0 à 20,
- un groupement alkyle à chaîne linéaire ou ramifiée ayant de 1 à 20 atomes de C,
- un groupement alcényle ou alcynyle à chaîne linéaire ou ramifiée ayant de 2 à 20 atomes de C et une ou plusieurs doubles ou triples liaisons,
où le groupement alkyle, alcényle ou alcynyle peut également contenir un ou plusieurs groupements C₃- à C₁₂-Cycloalkyle saturés ou insaturés, comme composé actif antimicrobien dans les aliments, les produits ménagers, les matières plastiques, le papier et/ou les peintures.

2. Utilisation non thérapeutique d'au moins un composé de formule I 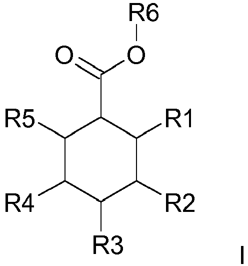 dans lequel R1, R2, R3, R4 et R5 représentent, indépendamment les uns des autres, un radical choisi parmi
- H, OH, OCOCH₃, O-(CH₂-CH₂-O)ₙ-CH₂-CH₂-OH, où n=0 à 20,
- un groupement alkyle ou alcoxy à chaîne linéaire ou ramifiée ayant de 1 à 20 atomes de C,
où au moins l'un des radicaux R1, R2, R3, R4 et R5 représente OH, OCOCH₃ ou O-(CH₂-CH₂-O)ₙ-CH₂-CH₂-OH, où n=0 à 20,
et où R6 représente un radical choisi parmi
- H,
- (CH₂-CH₂-O)ₙ-CH₂-CH₂-OH, où n= 0 à 20,
- un groupement alkyle à chaîne linéaire ou ramifiée ayant de 1 à 20 atomes de C,
- un groupement alcényle ou alcynyle à chaîne linéaire ou ramifiée ayant de 2 à 20 atomes de C et une ou plusieurs doubles ou triples liaisons,
où le groupement alkyle, alcényle ou alcynyle peut également contenir un ou plusieurs groupements C₃- à C₁₂-cycloalkyle saturés ou insaturés, comme composé actif antimicrobien dans des formulations cosmétiques/pharmaceutiques, des produits médicinaux, des aliments, des produits ménagers, des matières plastiques, du papier et/ou des peintures, afin d'améliorer la conservation.

3. Utilisation non thérapeutique d'au moins un composé de formule I 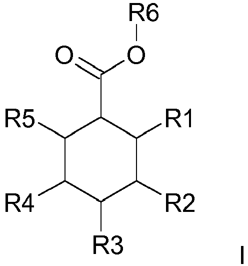 dans lequel R1, R2, R3, R4 et R5 représentent, indépendamment les uns des autres, un radical choisi parmi
- H, OH, OCOCH₃, O-(CH₂-CH₂-O)ₙ-CH₂-CH₂-OH, où n=0 à 20,
- un groupement alkyle ou alcoxy à chaîne linéaire ou ramifiée ayant de 1 à 20 atomes de C,
où au moins l'un des radicaux R1, R2, R3, R4 et R5 représente OH, OCOCH₃ ou O-(CH₂-CH₂-O)ₙ-CH₂-CH₂-OH, où n=0 à 20,
et où R6 représente un radical choisi parmi
- H,
- (CH₂-CH₂-O)ₙ-CH₂-CH₂-OH, où n= 0 à 20,
- un groupement alkyle à chaîne linéaire ou ramifiée ayant de 1 à 20 atomes de C,
- un groupement alcényle ou alcynyle à chaîne linéaire ou ramifiée ayant de 2 à 20 atomes de C et une ou plusieurs doubles ou triples liaisons,
où le groupement alkyle, alcényle ou alcynyle peut également contenir un ou plusieurs groupements C₃- à C₁₂-cycloalkyle saturés ou insaturés,
comme composé actif antitranspirant ou déodorant.

4. Utilisation selon l'une ou plusieurs parmi les revendications 1 à 3, **caractérisée en ce que** les radicaux R1, R2, R3, R4 et R5 représentent, indépendamment les uns des autres, H ou OH, où 1, 2 ou 3 parmi les radicaux R1, R2, R3, R4 et R5 représentent OH.

5. Utilisation selon la revendication 4, **caractérisée en ce que** le composé de formule I est choisi parmi les composés de formules la et Ib, 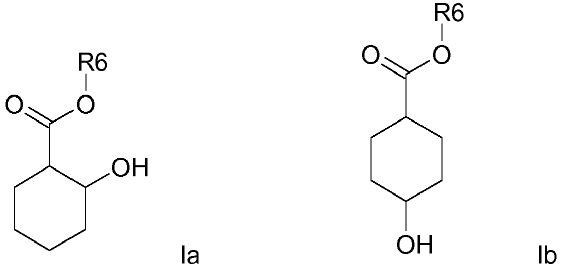 dans lesquels R6 représente un radical choisi parmi
- H,
- (CH₂-CH₂-O)ₙ-CH₂-CH₂-OH, où n= 0 à 20,
- un groupement alkyle à chaîne linéaire ou ramifiée ayant de 1 à 20 atomes de C,
- un groupement alcényle ou alcynyle à chaîne linéaire ou ramifiée ayant de 2 à 20 atomes de C et une ou plusieurs doubles ou triples liaisons,
où le groupement alkyle, alcényle ou alcynyle peut également contenir un ou plusieurs groupements C₃- à C₁₂-cycloalkyle saturés ou insaturés.

6. Utilisation selon l'une ou plusieurs parmi les revendications 1 à 5, **caractérisée en ce que** le radical R6 représente un radical choisi parmi
- H,
- CH₂-CH₂-OH,
- un groupement alkyle à chaîne linéaire ou ramifiée ayant de 1 à 10 atomes de C, pouvant également contenir un groupement C₆-cyclo-alkyle saturé ou insaturé.

7. Utilisation selon la revendication 6, **caractérisée en ce qu'**un composé de formule I est utilisé, choisi parmi les composés de formules I-1 à I-28
| | | | |
|---|---|---|---|
| I-1 | 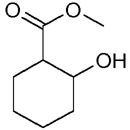 | I-2 | 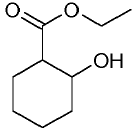 |
| I-3 | 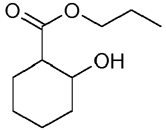 | I-4 | 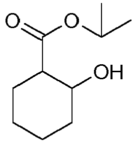 |
| I-5 | 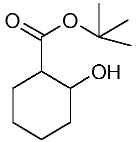 | I-6 | 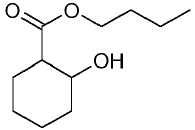 |
| I-7 | 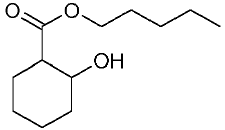 | I-8 | 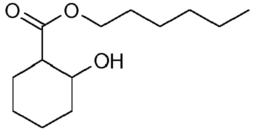 |
| I-9 | 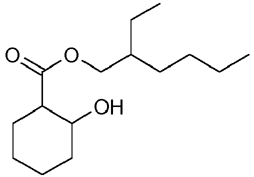 | I-10 | 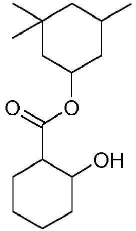 |
| I-11 | 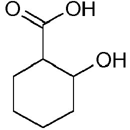 | I-12 | 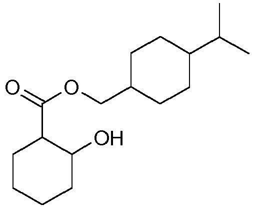 |
| I-13 | 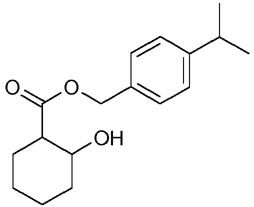 | I-14 | 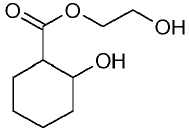 |
| I-15 | 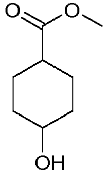 | I-16 | 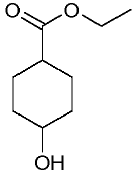 |
| I-17 | 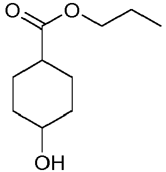 | I-18 | 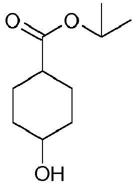 |
| I-19 | 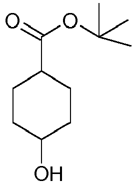 | I-20 | 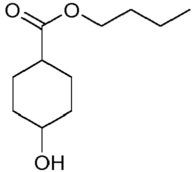 |
| I-21 | 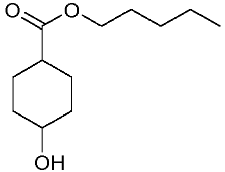 | I-22 | 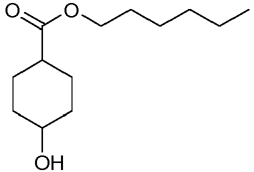 |
| I-23 | 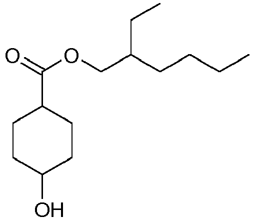 | I-24 | 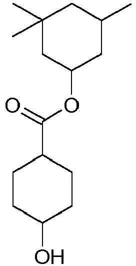 |
| I-25 | 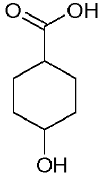 | I-26 | 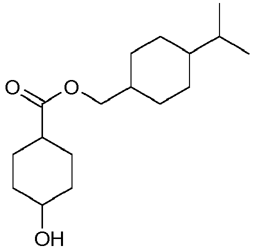 |
| I-27 | 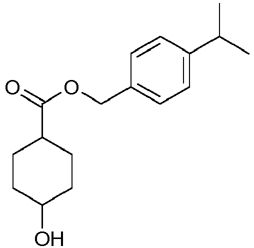 | I-28 | 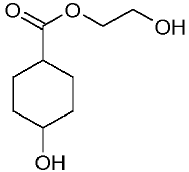 |

8. Préparation cosmétique ou pharmaceutique comprenant au moins un composé choisi dans le groupe constitué par les composés de formules I-3 à I-5, I-7 à I-10 et I-12 à I-28
| | | | |
|---|---|---|---|
| I-3 | 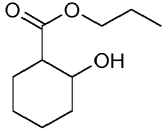 | I-4 | 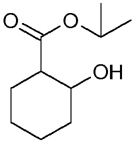 |
| I-5 | 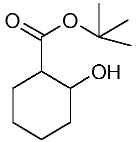 | | |
| I-7 | 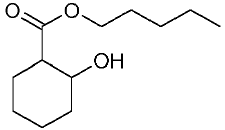 | I-8 | 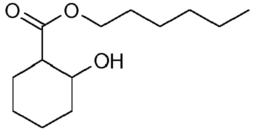 |
| I-9 | 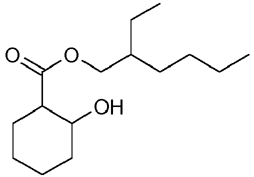 | I-10 | 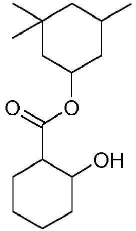 |
| | | I-12 | 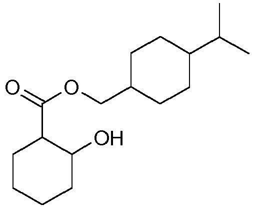 |
| I-13 | 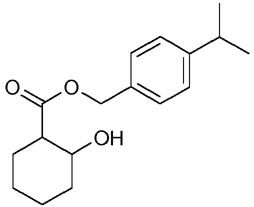 | I-14 | 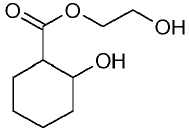 |
| I-15 | 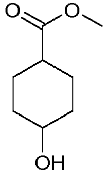 | I-16 | 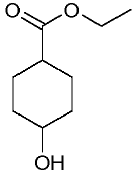 |
| I-17 | 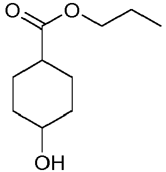 | I-18 | 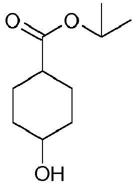 |
| I-19 | 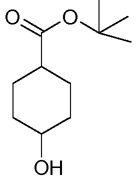 | I-20 | 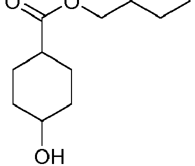 |
| I-21 | 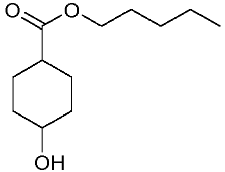 | I-22 | 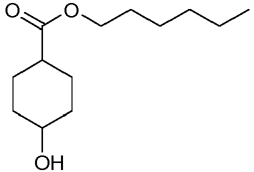 |
| I-23 | 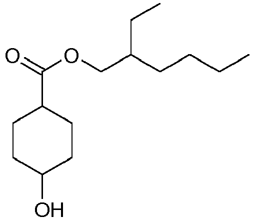 | I-24 | 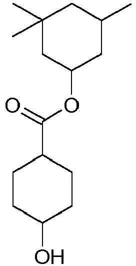 |
| I-25 | 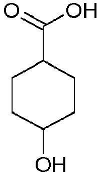 | I-26 | 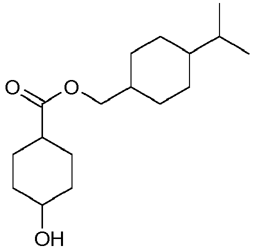 |
| I-27 | 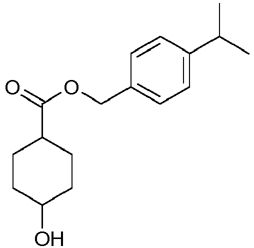 | I-28 | 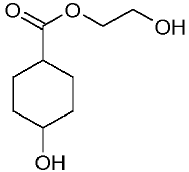 |
et au moins un véhicule convenable.

9. Préparation selon la revendication 8, **caractérisée en ce que** le composé choisi dans le groupe constitué par les composés de formules I-3 à I-5, I-7 à I-10 et I-12 à I-28 est présent selon une quantité allant de 0,01 à 20% en poids, sur la base de la quantité totale de la préparation.

10. Préparation selon la revendication 8 ou 9, **caractérisée en ce que** la préparation est un déodorant, un antitranspirant, une composition antipelliculaire ou anti-acnéique ou une préparation antibactérienne, destinée en particulier à un soin dentaire ou oral.

11. Procédé de préparation d'une préparation selon l'une ou plusieurs parmi les revendications 8 à 10, **caractérisé en ce que** le composé choisi dans le groupe constitué par les composés de formules I-3 à I-5, I-7 à I-10 et I-12 à I-28 est mélangé avec un véhicule convenable.

12. Composé de formule la ou Ib, 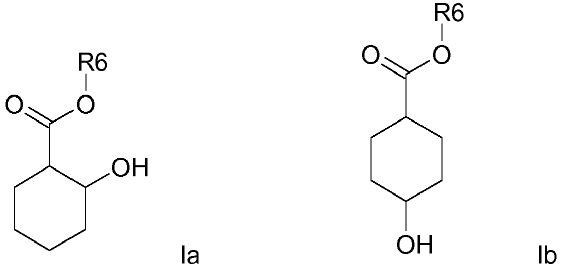 dans lequel R6 représente un radical choisi parmi
- H,
- (CH₂-CH₂-O)ₙ-CH₂-CH₂-OH, où n= 0 à 20,
- un groupement alkyle à chaîne linéaire ou ramifiée ayant de 1 à 20 atomes de C,
- un groupement alcényle ou alcynyle à chaîne linéaire ou ramifiée ayant de 2 à 20 atomes de C et une ou plusieurs doubles ou triples liaisons,
où le groupement alkyle, alcényle ou alcynyle peut également contenir un ou plusieurs groupements C₃- à C₁₂-cycloalkyle saturés ou insaturés, où les composés de formule la dans lesquels R6 représente H (composé I-11), méthyle (composé I-1), éthyle (composé I-2), isopropyle (composé I-4), tertio-butyle (composé I-5), n-butyle (composé I-6), n-hexyle (composé I-8), CH₂CH(CH₂CH₃)₂, CH₂CH₂CH(CH₃)₂, C(=CH₂)CH₃ ou cyclopentyle sont exclus,
et où les composés de formule Ib dans lesquels R6 représente H (composé I-25), méthyle (composé I-15), éthyle (composé I-16), propyle (composé I-17), isopropyle (composé I-18) ou tertio-butyle (composé I-19) sont exclus.

13. Composé selon la revendication 12, **caractérisé en ce que** le composé est choisi parmi les composés suivants :
| | | | |
|---|---|---|---|
| I-3 | 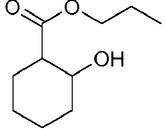 | | |
| I-7 | 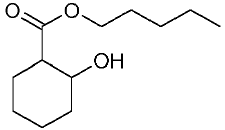 | I-9 | 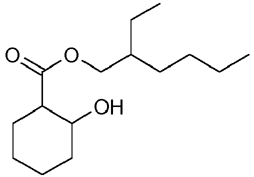 |
| I-10 | 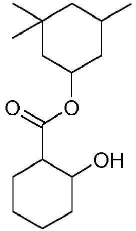 | I-12 | 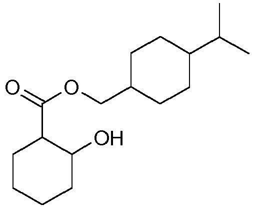 |
| I-13 | 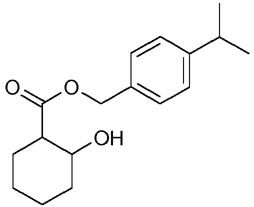 | I-14 | 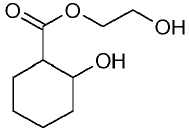 |
| I-20 | 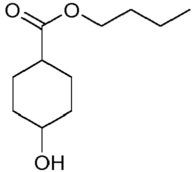 | I-21 | 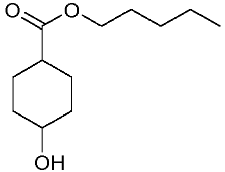 |
| I-22 | 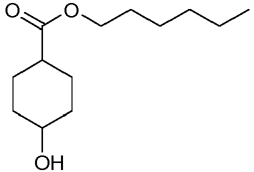 | I-23 | 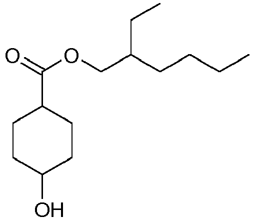 |
| I-24 | 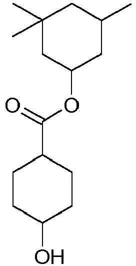 | I-26 | 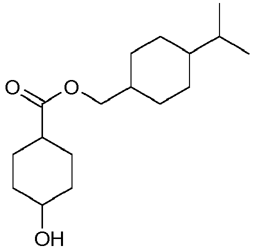 |
| I-27 | 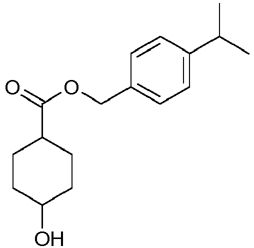 | I-28 | 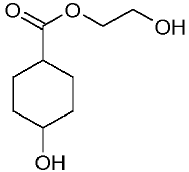 |

14. Procédé de préparation d'un composé de formule la ou Ib, selon la revendication 12 ou 13, **caractérisé en ce qu'**un composé de formule la-x ou Ib-x 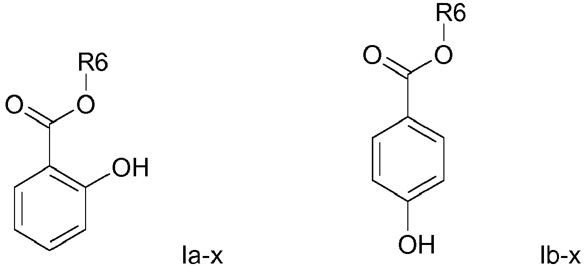 est converti en produit hydrogéné correspondant de formule la ou Ib au moyen d'une hydrogénation.

15. Composé de formule I 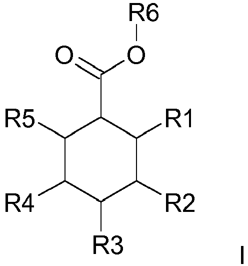 dans lequel R1, R2, R3, R4 et R5 représentent, indépendamment les uns des autres, un radical choisi parmi
- H, OH, OCOCH₃, O-(CH₂-CH₂-O)ₙ-CH₂-CH₂-OH, où n=0 à 20,
- un groupement alkyle ou alcoxy à chaîne linéaire ou ramifiée ayant de 1 à 20 atomes de C,
où au moins l'un des radicaux R1, R2, R3, R4 et R5 représente OH, OCOCH₃ ou O-(CH₂-CH₂-O)ₙ-CH₂-CH₂-OH, où n=0 à 20,
et où R6 représente un radical choisi parmi
- H,
- (CH₂-CH₂-O)ₙ-CH₂-CH₂-OH, où n= 0 à 20,
- un groupement alkyle à chaîne linéaire ou ramifiée ayant de 1 à 20 atomes de C,
- un groupement alcényle ou alcynyle à chaîne linéaire ou ramifiée ayant de 2 à 20 atomes de C et une ou plusieurs doubles ou triples liaisons,
où le groupement alkyle, alcényle ou alcynyle peut également contenir un ou plusieurs groupements C₃- à C₁₂-cycloalkyle saturés ou insaturés,
pour une utilisation thérapeutique comme composé actif antimicrobien.

16. Composé selon la revendication 15, pour une utilisation dans des produits de soin dentaire ou oral.

17. Composé de formule I 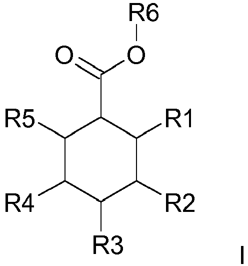 dans lequel R1, R2, R3, R4 et R5 représentent, indépendamment les uns des autres, un radical choisi parmi
- H, OH, OCOCH₃, O-(CH₂-CH₂-O)ₙ-CH₂-CH₂-OH, où n=0 à 20,
- un groupement alkyle ou alcoxy à chaîne linéaire ou ramifiée ayant de 1 à 20 atomes de C,
où au moins l'un des radicaux R1, R2, R3, R4 et R5 représente OH, OCOCH₃ ou O-(CH₂-CH₂-O)ₙ-CH₂-CH₂-OH, où n=0 à 20,
et où R6 représente un radical choisi parmi
- H,
- (CH₂-CH₂-O)ₙ-CH₂-CH₂-OH, où n= 0 à 20,
- un groupement alkyle à chaîne linéaire ou ramifiée ayant de 1 à 20 atomes de C,
- un groupement alcényle ou alcynyle à chaîne linéaire ou ramifiée ayant de 2 à 20 atomes de C et une ou plusieurs doubles ou triples liaisons,
où le groupement alkyle, alcényle ou alcynyle peut également contenir un ou plusieurs groupements C₃- à C₁₂-cycloalkyle saturés ou insaturés,
pour une utilisation comme composé actif antimicrobien anti-acnéique ou antipelliculaire.

18. Composé selon l'une ou plusieurs parmi les revendications 15 à 17, **caractérisé en ce que** les radicaux R1, R2, R3, R4 et R5 représentent, indépendamment les uns des autres, H ou OH, où 1, 2 ou 3 parmi les radicaux R1, R2, R3, R4 et R5 représentent OH.

19. Composé selon l'une ou plusieurs parmi les revendications 15 à 18, **caractérisé en ce que** le radical R6 représente un radical choisi parmi
- H,
- CH₂-CH₂-OH,
- un groupement alkyle à chaîne linéaire ou ramifiée ayant de 1 à 10 atomes de C, qui peut également contenir un groupement C₆-cyclo-alkyle saturé ou insaturé.

20. Composé selon la revendication 19, **caractérisée en ce qu'**un composé de formule I est choisi parmi les composés de formules I-1 à I-28
| | | | |
|---|---|---|---|
| I-1 | 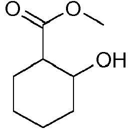 | I-2 | 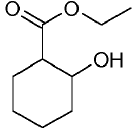 |
| I-3 | 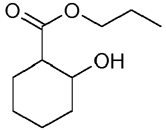 | I-4 | 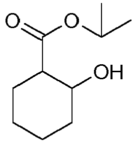 |
| I-5 | 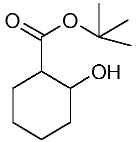 | I-6 | 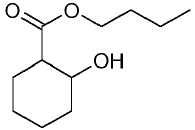 |
| I-7 | 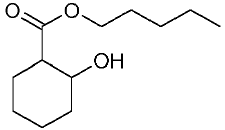 | I-8 | 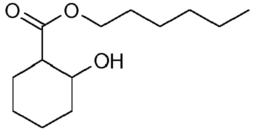 |
| I-9 | 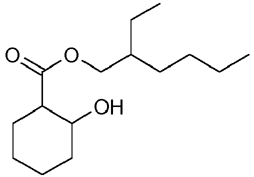 | I-10 | 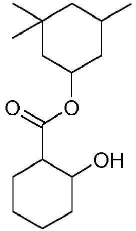 |
| I-11 | 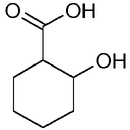 | I-12 | 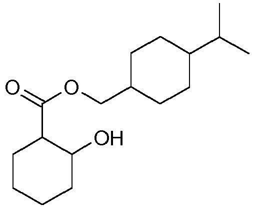 |
| I-13 | 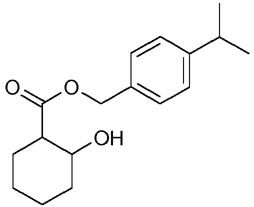 | I-14 | 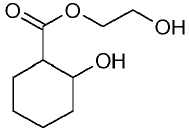 |
| I-15 | 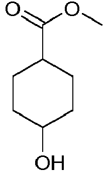 | I-16 | 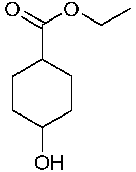 |
| I-17 | 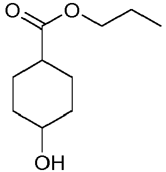 | I-18 | 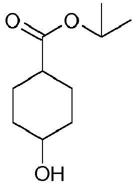 |
| I-19 | 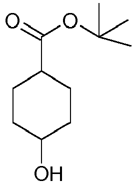 | I-20 | 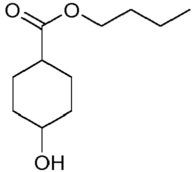 |
| I-21 | 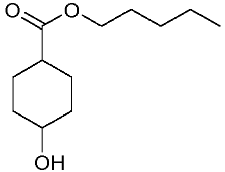 | I-22 | 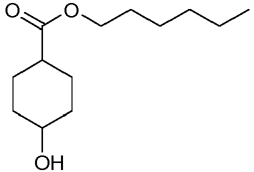 |
| I-23 | 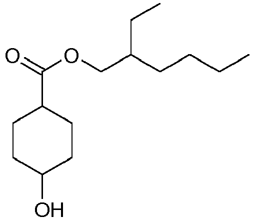 | I-24 | 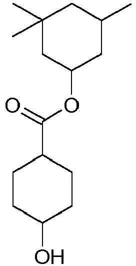 |
| I-25 | 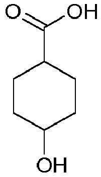 | I-26 | 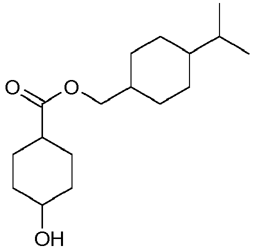 |
| I-27 | 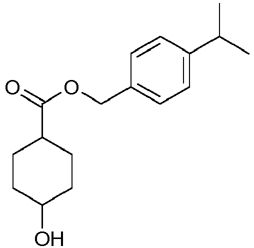 | I-28 | 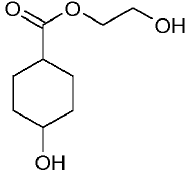 |
